(19) Europäisches Patentamt
European Patent Office
Office européen des brevets

(11) EP 2 075 289 B1

(12) # FASCICULE DE BREVET EUROPEEN

(45) Date de publication et mention de la délivrance du brevet:
**15.08.2012 Bulletin 2012/33**

(21) Numéro de dépôt: **08164735.6**

(22) Date de dépôt: **19.09.2008**

(51) Int Cl.:
***C09B 23/14*** *(2006.01)* ***C09B 49/12*** *(2006.01)*
***A61Q 5/08*** *(2006.01)* ***A61K 8/49*** *(2006.01)*
***A61Q 5/06*** *(2006.01)* ***A61Q 5/10*** *(2006.01)*

(54) **Composé styryl tetrahydroquinolinium thiol/disulfure, procédé d'éclaircissement des matières kératiniques à partir de ce colorant**

Styryltetrahydrochinolinium-Thiol/Disulfidverbindung, Verfahren zum Aufhellen keratinischer Materialien unter Verwendung dieses Farbstoffs

Styryl tetrahydroquinolinium thiol/disulfide compound, process for lightening keratin materials using this dye

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **21.09.2007 FR 0757753**

(43) Date de publication de la demande:
**01.07.2009 Bulletin 2009/27**

(73) Titulaire: **L'Oréal**
**75008 Paris (FR)**

(72) Inventeurs:
- **Greaves, Andrew**
  **77700 Bailly Romainvilliers (FR)**
- **Daubresse, Nicolas**
  **78170 La Celles St Cloud (FR)**
- **Manfre, Franco**
  **94170 Le Perreux sur Marne (FR)**

(74) Mandataire: **Fevrier, Murielle Françoise E.**
**L'Oréal**
**D.I.P.I.**
**25-29 Quai Aulagnier**
**92600 Asnières-sur-Seine Cedex (FR)**

(56) Documents cités:
**EP-A- 1 464 324** **EP-A- 1 647 580**
**EP-A- 2 001 960** **EP-A- 2 004 757**
**EP-A- 2 018 847** **WO-A-96/41173**
**WO-A-03/028685** **WO-A-2004/091556**
**WO-A-2005/004822** **WO-A-2006/134043**
**WO-A-2006/136617** **WO-A-2007/110539**
**WO-A-2007/110542** **WO-A2-2004/091473**
**WO-A2-2005/097051**

- **GEOFFREY J. ASHWELL, ABDUL MOHIB AND JAMES R. MILLER: "Induced rectification from self-assembled monolayers of sterically hindered -bridged chromophores" JOURNAL OF MATERIALS CHEMISTRY, vol. 15, no. 11, 27 janvier 2005 (2005-01-27), pages 1160-1166, XP002419175**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention concerne la coloration de matières kératiniques à l'aide de colorants fluorescents styryl tétrahydroquinolinium thiols/disulfures.

Il est connu de teindre les fibres kératiniques, notamment humaines, par une coloration directe. Le procédé classiquement utilisé en coloration directe consiste à appliquer sur les fibres kératiniques des colorants directs qui sont des molécules colorées et colorantes ayant une affinité pour les fibres, à les laisser diffuser, puis à rincer les fibres.

Les colorants directs qui sont classiquement utilisés sont par exemple des colorants du type nitrés benzéniques, des colorants anthraquinoniques, des nitropyridines, des colorants du type azoïque, xanthénique, acridinique, azinique ou triarylméthane.

La coloration des fibres kératiniques à partir de ces colorants directs classiques ne permet pas d'éclaircir de façon notable les fibres kératiniques.

L'éclaircissement de la couleur de fibres kératiniques foncées vers des nuances plus claires, en modifiant éventuellement la nuance de celles-ci, constitue une demande importante.

Classiquement, pour obtenir une coloration plus claire, on met en oeuvre un procédé de décoloration chimique. Ce procédé consiste à traiter les fibres kératiniques, telles que les cheveux, par un système oxydant fort, généralement constitué par du peroxyde d'hydrogène associé ou non à des persels, le plus souvent en milieu alcalin.

Ce système de décoloration présente l'inconvénient de dégrader les fibres kératiniques, et d'altérer leurs propriétés cosmétiques. Les fibres ont en effet tendance à devenir rêches, plus difficilement démêlables et plus fragiles. Enfin, l'éclaircissement ou la décoloration de fibres kératiniques par des agents oxydants est incompatible avec les traitements de modification de la forme desdites fibres, particulièrement dans les traitements de défrisage.

Une autre technique d'éclaircissement consiste à appliquer sur les cheveux foncés des colorants directs fluorescents. Cette technique décrite notamment dans les documents WO 03/028685, WO 2004/091473 et WO 2005/004822 permet de respecter la qualité de la fibre kératinique lors du traitement. Cependant ces colorants directs fluorescents ne possèdent pas une tenacité satisfaisante vis-à-vis des agents extérieurs.

Pour augmenter la ténacité des colorations directes, il est connu d'utiliser des colorants disulfures notamment colorants à chromophore imidazoliums dans les demandes de brevet WO 2005/097051 ou EP 1647580, et des colorants à chromophores pyridinium/indolinium-styryles dans les demandes de brevet WO 2006/134043 et WO 2006/136617.

Le but de la présente invention est de fournir de nouveaux systèmes de coloration de matières kératiniques en particulier les fibres kératiniques humaines, notamment les cheveux, qui ne présentent pas les inconvénients des procédés de décoloration existants.

Particulièrement, un but de l'invention est de fournir des systèmes de coloration directe permettant d'obtenir des effets éclaircissants notamment sur des fibres kératiniques naturellement ou artificiellement foncées, tenaces face à des shampooings successifs, qui ne dégradent pas les fibres kératiniques et qui n'altèrent pas leurs propriétés cosmétiques.

Un autre but de l'invention est de colorer de façon chromatique et rémanente vis-à-vis des agressions extérieures les matières kératiniques. L'invention vise également à mettre à disposition des composés colorant les fibres kératiniques telles que les cheveux avec une faible sélectivité de coloration entre la racine et la pointe, que ce soit sur fibres naturelles ou permanentées.

Ces buts sont atteints avec la présente invention qui a pour objet un procédé de coloration de matières kératiniques, en particulier les fibres kératiniques, notamment humaines telles que les cheveux, plus particulièrement les cheveux foncés, consistant à appliquer sur les matières kératiniques, une composition tinctoriale, comprenant dans un milieu cosmétique approprié, au moins un colorant fluorescent disulfure ou thiol, choisi parmi les colorants de formules (I) et (II) suivantes :

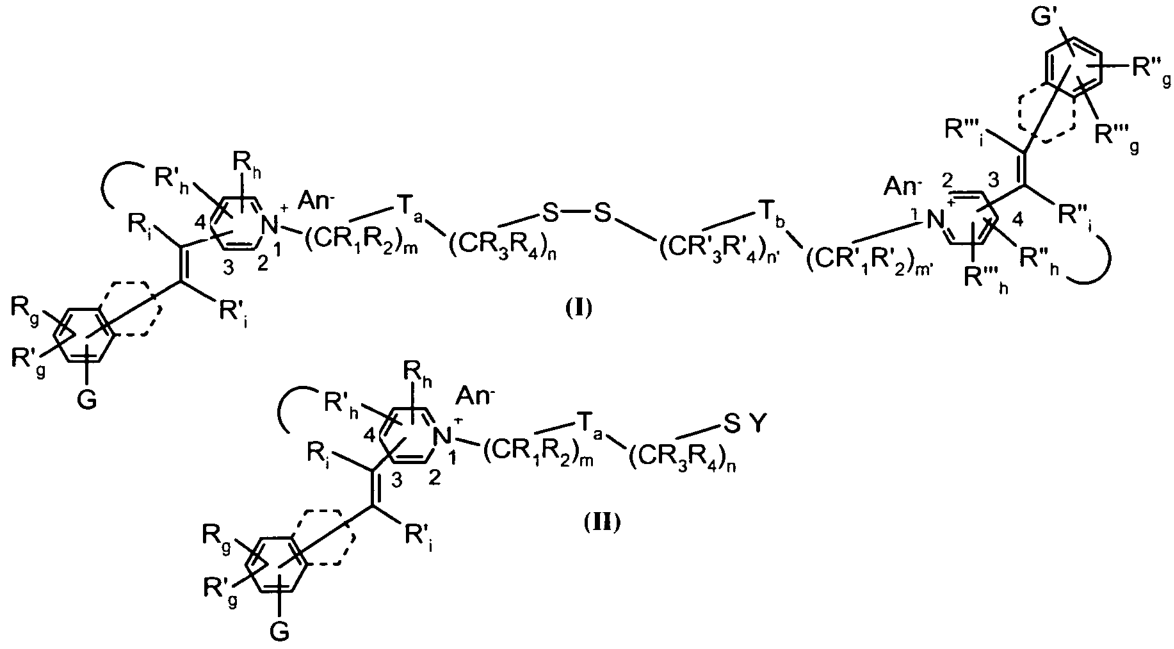

(I)

(II)

leurs sels d'acide organique ou minéral, isomères optiques, isomères géométriques, et les solvates tels que hydrates ; formules **(I)** et **(II)** dans lesquelles :

➢

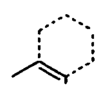

représente un groupe aryle, hétérocycle ou hétéroaryle fusionné au cycle phényle ; ou alors est absent du cycle phényle ; lorsque le cycle est présent il représente notamment un cycle benzo ou indéno, plus particulièrement benzo ;

➢ G et G', identiques ou différents, représentent un groupe $-NR_c,R_d$ ou $(C_1-C_6)$alcoxy éventuellement substitué, notamment non substitué ou alors G ou G' est absent ;

➢ $R_c$, et $R_d$, identiques ou différents, représentent un atome d'hydrogène, un groupe $(C_1-C_6)$alkyle éventuellement substitué, aryl$(C_1-C_4)$alkyle, $(C_1-C_6)$alcoxy$(C_1-C_6)$alkyle, un groupe $(C_1-C_6)$alkyle éventuellement substitué ;
$R_c$ et $R_d$ représentent particulièrement un atome d'hydrogène, un groupe $(C_1-C_3)$alkyle, ou un groupe $(C_1-C_3)$ alkyle substitué par i) un groupe hydroxy, ii) amino, iii) (di)$(C_1-C_3)$alkylamino, ou iv) ammonium quaternaire (R")(R"')(R"") $N^+$- ;

➢ ou alors les deux radicaux $R_c$ et $R_d$ portés par le même atome d'azote forment ensemble un groupe hétérocyclique ou hétéroaryle ; particulièrement l'hétérocycle est monocyclique et comprend entre 5 et 7 chaînons et l'hétéroaryle est bicyclique et comprend de 7 à 11 chaînons; plus particulièrement les groupes sont choisis parmi le pipéridinyle, l'imidazolyle, le pyrrolidinyle et l'indolyle ; l'hétérocycle pouvant être substitué par au moins un groupement hydroxy ;

➢ $R_g$, $R'_g$, $R"_g$, $R"'_g$, $R_h$, et $R"'_h$, identiques ou différents, représentent un atome d'hydrogène, d'halogène, un groupe amino, (di)$(C_1-C_4)$alkylamino, cyano, carboxy, hydroxy, trifluorométhyle, acylamino, alcoxy en $C_1-C_4$, (poly)hydroxyalcoxy en $C_2-C_4$, $(C_1-C_4)$alkylcarbonyloxy $(C_1-C_4)$alcoxycarbonyle, $(C_1-C_4)$alkylcarbonyl-amino, acylamino, carbamoyle , $(C_1-C_4)$alkylsulfonylamino, un radical amino-sulfonyle, ou un radical $(C_1-C_{16})$alkyle éventuellement substitué par un groupe choisi parmi $(C_1-C_{12})$alcoxy, hydroxy, cyano, carboxy, amino, (di)$(C_1-C_4)$alkylamino, ou alors les deux radicaux alkyles portés par l'atome d'azote du groupe amino forment un hétérocycle comprenant de 5 à 7 chaînons et comprenant éventuellement un autre hétéroatome identique ou différent à celui de l'atome d'azote ; particulièrement $R_g$, $R'_g$, $R"_g$, $R"'_g$, $R_h$, et $R"'_h$ représentent un atome d'hydrogène ;
ou alors deux groupes $R_g$ et $R'_g$, $R"_g$ et $R"'_g$, portés par deux atomes de carbone adjacents, forment ensembles un cycle benzo, indéno, un groupe hétérocycloalkyle fusionné ou hétéroaryle fusionné ; le cycle benzo, indéno, hétérocycloalkyle ou hétéroaryle étant éventuellement substitué par un atome d'halogène, $(C_1-C_4)$alkyle, un groupe

amino, $(C_1-C_4)$alkylamino, $(C_1-C_4)$dialkylamino, cyano, carboxy, hydroxy, trifluorométhyle, un radical acylamino, alcoxy en $C_1-C_4$, (poly)hydroxyalcoxy en $C_2-C_4$, alkylcarbonyloxy alcoxycarbonyle, alkylcarbonylamino, un radical acylamino, carbamoyle, alkylsulfonylamino, un radical amino-sulfonyle, ou un radical $(C_1-C_{16})$ alkyle éventuellement substitué par : un groupe choisi parmi $(C_1-C_{12})$ alcoxy, hydroxy, cyano, carboxy, amino, $(C_1-C_4)$ alkylamino, $(C_1-C_4)$ dialkylamino, ou alors les deux radicaux alkyles portés par l'atome d'azote du groupe amino forment un hétérocycle comprenant de 5 à 7 chaînons et comprenant éventuellement un autre hétéroatome identique ou différent à celui de l'atome d'azote ; particulièrement $R_g$ et $R'_g$; $R''_g$ et $R'''_g$ forment ensemble un groupe benzo ;

➢ ou alors lorsque G et/ou G' représentent $-NR_cR_d$ deux groupes $R_c$ et $R'_g$, $R_d$ et Rg et/ou $R_c$ et $R''_g$, $R_d$ et $R'''_g$, forment ensemble un hétéroaryle ou hétérocycle saturé, éventuellement substitué par un ou plusieurs groupes $(C_1-C_6)$alkyle ; notamment l'hétérocycle ou l'hétéroaryle contient un ou deux hétéroatomes choisis parmi l'azote et l'oxygène et l'hétérocycle comprend entre 5 et 7 chaînons et l'hétéroaryle contient entre 7 et 11 chaînons ; particulièrement l'hétérocycle est choisi parmi les groupe morpholinyle, pipérazinyle, pipéridinyle, homopipéridinyle, pyrrolidinyle et l'hétéroaryle est un indolyle ;

➢ $R_i'$ et $R'''_i$, identiques ou différents, représentent un atome d'hydrogène, ou un groupe $(C_1-C_4)$ alkyle ; particulièrement $R'_i$ et $R'''_i$, représentent un atome d'hydrogène ;

➢ $R'_h$ avec $R_i$ et $R''_h$ avec $R''_i$ forment ensemble avec les atomes de carbone qui les portent un groupe cycloalkyle en $C_5-C_7$ éventuellement substitué, fusionné au groupe pyridinium, particulièrement un groupe cyclohexyle ; étant entendu que le radical $R'_h$ ou $R''_h$ et le groupe styryle portant le radical $R_i$ ou $R''_i$ sont positionnés sur des atomes de carbone adjacents des groupes pyridiniums ; plus particulièrement ils sont positionnés respectivement sur les atomes de carbone 3 et 4 des groupes pyridinium ;

➢ $R_1$, $R_2$, $R_3$, $R_4$, $R'_1$, $R'_2$, $R'_3$ et $R'_4$, identiques ou différents, représentent :

- un atome d'hydrogène,
- un groupe $(C_1-C_4)$alkyle,
- $(C_1-C_{12})$alcoxy,
- hydroxy,
- cyano,
- $-C(O)O^-M^+$ avec $M^+$ représentant un métal alcalin ou $M^+$ et $An^-$ sont absents,
- carboxy,
- (di)$(C_1-C_4)$(alkyl)amino, lesdits radicaux alkyle pouvant former avec l'atome d'azote qui les portent, un hétérocycle comprenant de 5 à 7 chaînons, comprenant éventuellement un autre hétéroatome différent ou non de l'azote ;
  notamment $R_1$, $R_2$, $R_3$, $R_4$, $R'_1$, $R'_2$, $R'_3$ et $R'_4$, sont des atomes d'hydrogène ou un groupe
- $C(O)O^-M^+$ ; particulièrement $R_1$, $R_2$, $R_3$, $R_4$, $R'_1$, $R'_2$, $R'_3$ et $R'_4$ représentent un atome d'hydrogène ;

➢ $T_a$, $T_b$, identiques ou différentes représentent :

i) soit une liaison covalente σ,
ii) soit un ou plusieurs radicaux ou leurs combinaisons choisis parmi $-SO_2-$, -O-, -S-, -N(R)-, $-N^+(R)(R°)-$, -C (O)-, avec R et R°, identiques ou différents, représentant un atome d'hydrogène, un radical alkyle en $C_1-C_4$, hydroxyalkyle en $C_1-C_4$ ou un aryl$(C_1-C_4)$alkyle ; notamment $T_a$ est identique à $T_b$ et ils représentent une liaison covalente □ou un groupe choisi parmi -N(R)-, -C(O)-, -C(O)-N(R)-, -N(R)-C(O)-, -C(O)-N(R)-C(O)-, -O-C(O)-, -C(O)-O- et $-N^+(R)(R^o)-$, avec R, R° identiques ou différents représentant un atome d'hydrogène, un groupe $C_1-C_4$ alkyle ; particulièrement $T_a$ et $T_b$ représentent -C(O)-N(R)-, -N(R)-C(O)- ;
iii) soit un radical hétérocycloalkyle ou hétéroaryle, cationique ou non, préférentiellement monocycliques, contenant notamment deux hétéroatomes, particulièrement deux atomes d'azote, et comportant notamment de 5 à 7 chaînons tel que l'imidazolium, pyridinium ou pyrolidinium éventuellement substitué par un groupe $(C_1-C_4)$ alkyle tel que méthyle ;

➢ m, m', n et n', identiques ou différents, représentent un entier compris inclusivement entre 0 et 6 avec m+n, m'+n', identiques ou différents, représentent un entier compris inclusivement entre 1 et 10 ; particulièrement la somme m+n=m'+n'= un entier compris inclusivement entre 2 et 4 ; préférentiellement m+n=m'+n'=2;

➢ $An^-$ représentant un contre-ion anionique ; et

➢ Y représente : i) un atome d'hydrogène ; ii) un métal alcalin ; iii) un métal alcalino-terreux ; iv) un groupe ammonium : $N^+R^\alpha R^\beta R^\gamma R^\delta$ ou un groupe phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe $(C_1-C_4)$alkyle ; ou v) un groupe protecteur de fonction thiol ;

étant entendu que lorsque le composé de formule (I) ou (II) contient d'autres parties cationiques, il se trouve associé à

un ou plusieurs contre-ions anioniques permettant d'atteindre l'électroneutralité de la formule (I) ou (II).

Un autre objet de l'invention est une composition tinctoriale comprenant, dans un milieu cosmétique approprié au moins un colorant fluorescent disulfure (I) ou un colorant fluorescent thiol de formule (II) tels que définis précédemment, et éventuellement un agent réducteur.

L'invention a aussi pour objet de nouveaux colorants fluorescents disulfures de formule (I) et thiols de formule (II).

Le procédé de coloration selon l'invention permet de colorer de façon visible les matières kératiniques foncées, en particulier les fibres kératiniques humaines foncées, notamment les cheveux foncés.

De plus le procédé de l'invention permet d'obtenir une coloration des matières kératiniques en particulier les fibres kératiniques humaines, notamment les cheveux, sans dégrader ladite matière, rémanente vis-à-vis de shampooings, des agressions courantes (soleil, transpiration), et des autres traitements capillaires. Le procédé de l'invention permet également d'obtenir un éclaircissement des matières kératiniques telles que les fibres kératiniques particulièrement les fibres kératiniques foncées et plus particulièrement les cheveux foncés.

Les colorants de l'invention sont par ailleurs stables vis-à-vis des oxydants, et présentent une solubilité dans les milieux de teinture cosmétique satisfaisante. Ces colorants étendent la gamme colorielle aux jaunes et oranges. Les colorants de formule (I) ou (II) colorent après application sur fibres kératiniques de façon chromatique et rémanente vis-à-vis des agressions extérieures les matières kératiniques avec une faible sélectivité de coloration entre la racine et la pointe, et sur différents types de fibres.

Au sens de l'invention, on entend par matière kératinique foncée celle qui présente une luminescence L* chiffrée dans le système C.I.E. L*a*b*, inférieure ou égale à 45 et de préférence inférieure ou égale à 40, sachant par ailleurs que L*=0 équivaut au noir et L*=100 au blanc.

Au sens de l'invention, on entend par cheveux naturellement ou artificiellement foncés, des cheveux dont la hauteur de ton est inférieure ou égale à 6 (blond foncé) et de préférence inférieure ou égale à 4 (châtain).

L'éclaircissement des cheveux est évalué par la variation de «hauteur de ton» avant et après application du composé de formule (I) ou (II). La notion « ton » repose sur la classification des nuances naturelles, un ton séparant chaque nuance de celle qui la suit ou la précède immédiatement. Cette définition et la classification des nuances naturelles est bien connue des professionnels de la coiffure et publiée dans l'ouvrage « Science des traitements capillaires » de Charles ZVIAK 1988, Ed. Masson, p. 215 et 278.

Les hauteurs de ton s'échelonnent de 1 (noir) à 10 (blond très clair), une unité correspondant à un ton ; plus le chiffre est élevé et plus la nuance est claire.

Un cheveu coloré artificiellement est un cheveu dont la couleur a été modifiée par un traitement de coloration par exemple une coloration avec des colorants directs ou des colorants d'oxydation.

Au sens de l'invention, on entend par cheveux décolorés, des cheveux dont la hauteur de ton est supérieure à 4 (châtain) et de préférence supérieure à 6 (blond foncé).

Un moyen pour mesurer l'effet éclaircissant apporté aux cheveux après application des colorants fluorescent de l'invention est d'utiliser le phénomène de réflectance des cheveux.

De préférence, la composition doit, après application sur des cheveux foncés amener aux résultats ci-dessous.

- On s'intéresse aux performances de réflectance des cheveux lorsqu'on les irradie avec de la lumière visible dans la gamme de longueurs d'onde allant de 400 à 700 nanomètres.
- On compare alors les courbes de réflectance en fonction de la longueur d'onde, des cheveux traités avec la composition de l'invention et des cheveux non traités.
- La courbe correspondant aux cheveux traités doit montrer une réflectance dans la gamme des longueurs d'onde allant de 500 à 700 nanomètres supérieure à la courbe correspondant aux cheveux non traités.
- Cela signifie que, dans la gamme de longueur d'onde allant de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%. Ceci n'empêche pas qu'il peut exister dans la gamme de longueur d'onde allant de 540 à 700 nanomètres, au moins une plage où la courbe de réflectance correspondant aux cheveux traités soit superposable, ou inférieure à la courbe de réflectance correspondant aux cheveux non traités.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

Au sens de la présente invention, et à moins qu'une indication différente ne soit donnée :

- les radicaux « aryle » ou « hétéroaryle » ou la partie aryle ou hétéroaryle d'un radical peuvent être substitués par au moins un substituant porté par un atome de carbone, choisi parmi :

- un radical alkyle en $C_1$-$C_{16}$, de préférence en $C_1$-$C_8$, éventuellement substitué par un ou plusieurs radicaux choisis parmi les radicaux hydroxy, alcoxy en $C_1$-$C_2$, (poly)-hydroxyalcoxy en $C_2$-$C_4$, acylamino, amino substitué par deux radicaux alkyle, identiques ou différents, en $C_1$-$C_4$, éventuellement porteurs d'au moins un groupe hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
- un atome d'halogène tel que chlore, fluor ou brome ;
- un groupe hydroxy ;
- un radical alcoxy en $C_1$-$C_2$ ;
- un radical alkylthio en $C_1$-$C_2$ ;
- un radical (poly)-hydroxyalcoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

  i) un groupe hydroxy,
  ii) un groupe amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,

- -NR-COR' dans lequel le radical R est un atome d'hydrogène, un radical alkyle en $C_1$-$C_4$ éventuellement porteur d'au moins un groupe hydroxy et le radical R' est un radical alkyle en $C_1$-$C_2$ ;
- $(R)_2N$-CO- dans lequel les radicaux R, identiques ou non, représentent un atome d'hydrogène, un radical alkyle en $C_1$-$C_4$ éventuellement porteur d'au moins un groupe hydroxy ;
- $R'SO_2$-NR- dans lequel le radical R représente un atome d'hydrogène, un radical alkyle en $C_1$-$C_4$ éventuellement porteur d'au moins un groupe hydroxy et le radical R' représente un radical alkyle en $C_1$-$C_4$, un radical phényle ;
- $(R)_2N$-$SO_2$- dans lequel les radicaux R, identiques ou non, représentent un atome d'hydrogène, un radical alkyle en $C_1$-$C_4$ éventuellement porteur d'au moins un groupe hydroxy,
- un radical carboxylique sous forme acide ou salifiée (de préférence avec un métal alcalin ou un ammonium, substitué ou non) ;
- un groupe cyano ;
- un groupe polyhalogénoalkyle comprenant de 1 à 6 atomes de carbones et de 1 à 6 atome d'halogène, identiques ou différents, le groupe polyhalogénoalkyle est par exemple le trifluorométhyle ;

- la partie cyclique ou hétérocyclique d'un radical non aromatique peut être substituée par au moins un substituant choisi parmi les groupes :

  - hydroxy,
  - alcoxy en $C_1$-$C_4$,
  - alkyle en $C_1$-$C_4$,
  - (poly)hydroxyalcoxy en $C_2$-$C_4$,
  - un radical alkylthio en $C_1$-$C_2$ ;
  - RCO-NR'- dans lequel le radical R' est un atome d'hydrogène, un radical alkyle en $C_1$-$C_4$ éventuellement porteur d'au moins un groupe hydroxy et le radical R est un radical alkyle en $C_1$-$C_2$, amino substitué par deux groupes alkyle identiques ou différents en $C_1$-$C_4$ éventuellement porteurs d'au moins un groupe hydroxy ;
  - RCO-O- dans lequel le radical R est un radical alkyle en $C_1$-$C_4$, amino substitué par un ou deux groupes alkyle identiques ou différents en $C_1$-$C_4$ éventuellement porteurs d'au moins un groupe hydroxy, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote ;
  - RO-CO- dans lequel le radical R est un radical alkyle en $C_1$-$C_4$ éventuellement porteurs d'au moins un groupe hydroxy ;

- un radical cyclique, hétérocyclique, ou une partie non aromatique d'un radical aryle

ou hétéroaryle, peut également être substitué par un ou plusieurs groupes oxo ou thioxo ;

- un radical « aryle » représente un groupe mono ou polycyclique, condensé ou non, comprenant de 6 à 22 atomes de carbones, et dont au moins un cycle est aromatique ; préférentiellement le radical aryle est un phényle, biphényle, naphtyle, indényle, anthracényle, ou tétrahydronaphtyle ;
- un radical « diarylalkyle » représente un groupe comportant sur le même atome de carbone d'un groupe alkyle deux groupe aryle, identiques ou différents tel que diphénylméthyle ou 1,1-diphényléthyle ;
- un « radical hétéroaryle » représente un groupe mono ou polycyclique, condensé ou non, éventuellement cationique, comprenant de 5 à 22 chaînons, de 1 à 6 hétéroatomes choisis parmi l'atome d'azote, d'oxygène, de soufre et de sélénium, et dont au moins un cycle est aromatique ; préférentiellement un radical hétéroaryle est choisis parmi acridinyle, benzimidazolyle, benzobistriazolyle, benzopyrazolyle, benzopyridazinyle, benzoquinolyle, benzothiazolyle, benzotriazolyle, benzoxazolyle, pyridinyle, tétrazolyle, dihydrothiazolyle, imidazopyridinyle, imidazolyle, indolyle, isoquinolyle, naphthoimidazolyle, naphthooxazolyle, naphthopyrazolyle, oxadiazolyle, oxazolyle, oxazolopyridyle, phénazinyle, phénooxazolyle, pyrazinyle, pyrazolyle, pyrilyle, pyrazoyltriazyle, pyridyle, pyridinoimidazolyle, pyrrolyle, quinolyle, tétrazolyle, thiadiazolyle, thiazolyle, thiazolopyridinyle, thiazoylimidazolyle, thiopyrylyle, triazolyle, xanthylyle et son sel d'ammonium ;
- un radical « dihétéroarylalkyle » représente un groupe comportant sur le même atome de carbone d'un groupe alkyle deux groupe hétéroaryle, identiques ou différents tel que difurylméthyle, 1,1-difuryléthyle, dipyrrolylméthyle, dithiénylméthyle ;
- un « radical cyclique » est un radical cycloalkyle non aromatique, mono ou polycyclique, condensé ou non, contenant de 5 à 22 atomes de carbone, pouvant comporter de 1 à plusieurs insaturations ; particulièrement le radical cyclique est un cyclohexyle ;
- un radical « cyclique stériquement encombré» est un radical cyclique, aromatique ou non, substitué ou non, encombré par effet ou contrainte stérique, comprenant de 6 à 14 chaînons, pouvant être pontés, à titre de radicaux stériquement encombrés on peut citer le bicyclo[1.1.0]butane, les mésytyles tels que le 1,3,5-triméthylpnényle, le 1,3,5-triterbutylphényle, le 1,3,5-isobutylphényle, le 1,3,5-trimétylsillylphényle et l'adamantyle ;
- un « radical hétérocyclique ou hétérocycle» est un radical non aromatique mono ou polycyclique, condensé ou non, contenant de 5 à 22 chaînons, comportant de 1 à 6 hétéroatomes choisis parmi l'atome d'azote, d'oxygène, de soufre et de sélénium ;
- un « radical alkyle » est un radical hydrocarboné en $C_1$-$C_{16}$, linéaire ou ramifié, de préférence en $C_1$-$C_8$ ;
- l'expression « éventuellement substitué » attribué au radical alkyle sous entend que ledit radical alkyle peut être substitué par un ou plusieurs radicaux choisis parmi les radicaux i) hydroxy, ii) alcoxy en $C_1$-$C_4$, iii) acylamino, iv) amino éventuellement substitué par un ou deux radicaux alkyle, identiques ou différents, en $C_1$-$C_4$, lesdits radicaux alkyles pouvant former avec l'atome d'azote qui les portent un hétérocyle comprenant de 5 à 7 chaînons, comprenant éventuellement un autre hétéroatome différent ou non de l'azote ; v) ou un groupe ammonium quaternaire $-N^+$ R'R"R"', $M^-$ pour lequel R', R", R"', identiques ou différents représentent un atome d'hydrogène,

ou un groupe alkyle en $C_1$-$C_4$, ou alors $-N^+$ R'R"R"' forme un hétéroaryle tel que imidazolium éventuellement substitué par un groupe $C_1$-$C_4$ alkyle, et M représente le contre-ion de l'acide organique, minéral ou de l'halogénure correspondant,

- un « radical alcoxy » est un radical alkyle-oxy ou alkyl-O- pour lequel le radical alkyle est un radical hydrocarboné, linéaire ou ramifié, en $C_1$-$C_{16}$ préférentiellement en $C_1$-$C_8$;
- un « radical alkylthio » est un radical alkyl-S- pour lequel le radical alkyle est un radical hydrocarboné, linéaire ou ramifié, en $C_1$-$C_{16}$ préférentiellement en $C_1$-$C_8$ ; lorsque le groupe alkylthio est éventuellement substitué, cela sous entend que le groupe alkyle est éventuellement substitué tel que défini précédemment ;
- les bornes délimitant l'étendue d'une plage de valeurs sont comprises dans cette plage de valeurs ;
- un « sel d'acide organique ou minéral » est plus particulièrement choisi parmi un sel dérivé i) d'acide chlorhydrique HCl, ii) d'acide bromhydrique HBr, iii) d'acide sulfurique $H_2SO_4$, iv) d'acides alkylsulfoniques : Alk-$S(O)_2OH$ tels que d'acide méthylsulfonique et d'acide éthylsulfonique ; v) d'acides arylsulfoniques : Ar-$S(O)_2OH$ tel que d'acide benzène sulfonique et d'acide toluène sulfonique ; vi) d'acide citrique ; vii) d'acide succinique ; viii) d'acide tartrique ; ix) d'acide lactique, x) d'acides alkoxysulfiniques : Alk-O-S(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3COOH$ ; xiv) d'acide triflique $CF_3SO_3H$ et xv) d'acide tétrafluoroborique $HBF_4$;
- un « contre-ion anionique » est un anion ou un groupe anionique associé à la charge cationique du colorant ; plus particulièrement le contre-ion anionique est choisi parmi i) les halogénures tels que le chlorure, le bromure ; ii) les nitrates ; iii) les sulfonates parmi lesquels les $C_1$-$C_6$ alkylsulfonates : Alk-$S(O)_2O^-$ tels que le méthylsulfonate ou mésylate et l'éthylsulfonate ; iv) les arylsulfonates : Ar-$S(O)_2O^-$ tel que le benzènesulfonate et le toluènesulfonate

ou tosylate ; v) le citrate ; vi) le succinate ; vii) le tartrate ; viii) le lactate ; ix) les alkylsulfates : Alk-O-S(O)O$^-$ tels que le méthysulfate et l'éthylsulfate ; x) les arylsulfates : Ar-O-S(O)O$^-$ tels que le benzènesulfate et le toluènesulfate ; xi) les alkoxysulfates : Alk-O-S(O)$_2$O$^-$ tel que le méthoxy sulfate et l'éthoxysulfate ; xii) les aryloxysulfates : Ar-O-S(O)$_2$O$^-$, xiii) le phosphate ; xiv) l'acétate ; xv) le triflate ; et xvi) les borates tels que le tétrafluoroborate ;

- les « solvates » représentent les hydrates ainsi que l'association avec des alcools linéaires ou ramifiés en $C_1$-$C_4$ linéaire ou ramifié tels que l'éthanol, l'isopropanol, le n-propanol.

Les colorants fluorescents disulfures de formule (I) ou thiols de formule (II) sont des composés capables d'absorber dans le rayonnement UV ou visible à une longueur d'onde $\lambda_{abs}$ comprise entre 250 et 800 nm et capables de réémettre dans le domaine du visible à une longueur d'onde d'émission $\lambda_{ém}$ comprise entre 400 et 800 nm.

De préférence les composés fluorescents de formules (I) ou (II) de l'invention sont des colorants capables d'absorber dans le visible $\lambda_{abs}$ comprise entre 400 et 800 nm et de réémettre dans le visible $\lambda_{ém}$ comprise entre 400 et 800 nm. Plus préférentiellement les colorants de formules (I) ou (II) sont des colorants capables d'absorber à une $\lambda_{abs}$ comprise entre 420 nm et 550 nm et de réémettre dans le visible à une $\lambda_{ém}$ comprise entre 470 et 600 nm.

Les composés fluorescents de l'invention de formule (II) contiennent une fonction SY qui peut se trouver sous la forme covalente -S-Y ou ionique -S- Y$^+$ selon la nature de Y et du pH du milieu.

Un mode particulier concerne les colorants fluorescents thiols de formule (II) à fonction SY où Y représente un atome d'hydrogène, ou un métal alcalin. Avantageusement Y représente un atome d'hydrogène.

Conformément à un autre mode de réalisation particulier de l'invention, dans la formule (II) précitée, Y est un groupe protecteur connu par l'homme du métier comme par exemple ceux décrits dans les ouvrages « Protective Groups in Organic Synthesis », T. W. Greene, John Willey & Sons ed., NY, 1981, pp.193-217; « Protecting Groups », P. Kocienski, Thieme, 3ème ed., 2005, chap. 5. Etant entendu que Y en tant que groupement protecteur ne peut constituer avec l'atome d'azote qui le porte un colorant disulfure i.e. ne peut constituer un composé de formule (I). Le groupement protecteur Y ne peut représenter dans la formule (II) un groupement directement relié à l'atome de soufre via un autre atome de soufre non oxydé.

Particulièrement lorsque Y représente un groupe protecteur de la fonction thiol, Y est choisi parmi les radicaux suivants :

■ ($C_1$-$C_4$)alkylcarbonyle ;
■ ($C_1$-$C_4$)alkylthiocarbonyle ;
■ ($C_1$-$C_4$)alcoxycarbonyle ;
■ ($C_1$-$C_4$)alcoxythiocarbonyle ;
■ ($C_1$-$C_4$)alkylthio-thiocarbonyle ;
■ (di)($C_1$-$C_4$)(alkyl)aminocarbonyle ;
■ (di)($C_1$-$C_4$)(alkyl)aminothiocarbonyle;
■ arylcarbonyle comme phénylcarbonyle ;
■ aryloxycarbonyle ;
■ aryl($C_1$-$C_4$)alcoxycarbonyle ;
■ (di)($C_1$-$C_4$)(alkyl)aminocarbonyle comme diméthylaminocarbonyle ;
■ ($C_1$-$C_4$)(alkyl)arylaminocarbonyle ;
■ carboxy ;
■ $SO_3^-$; M$^+$ avec M$^+$ représentant un métal alcalin tel que le sodium ou le potassium, ou alors An$^-$ de la formule (II) et M$^+$ sont absents ;
■ aryle éventuellement substitué tel que le phényle, dibenzosubéryle, ou 1,3,5-cycloheptatriényle,
■ hétéroaryle éventuellement substitué ; dont notamment l'hétéroaryle cationiques ou non, comprenant de 1 à 4 hétéroatomes suivants :

i) monocycliques à 5, 6 ou 7 chaînons tels que furanyle ou furyle, pyrrolyle ou pyrryle, thiophényle ou thiényle, pyrazolyle, oxazolyle, oxazolium, isoxazolyle, isoxazolium, thiazolyle, thiazolium, isothiazolyle, isothiazolium, 1,2,4-triazolyle, 1,2,4-triazolium, 1,2,3-triazolyle, 1,2,3-triazolium, 1,2,4-oxazolyle, 1,2,4-oxazolium, 1,2,4-thiadiazolyle, 1,2,4-thiadiazolium, pyrylium, thiopyridyle, pyridinium, pyrimidinyle, pyrimidinium, pyrazinyle, pyrazinium, pyridazinyle, pyridazinium, triazinyle, triazinium, tétrazinyle, tétrazinium, azépine, azépinium, oxazépinyle, oxazépinium, thiépinyle, thiépinium, imidazolyle, imidazolium ;
ii) bicycliques à 8 à 11 chaînons tels que indolyle, indolinium, benzoimidazolyle, benzoimidazolium, benzoxazolyle, benzoxazolium, dihydrobenzoxazolinyle, benzothiazolyle, benzothiazolium, pyridoimidazolyle, pyridoimidazolium, thiénocycloheptadiényle, ces groupes mono ou bicycliques étant éventuellement substitués par un ou plusieurs groupes tels que ($C_1$-$C_4$)alkyle comme méthyle, ou polyhalogéno($C_1$-$C_4$)alkyle comme trifluorométhyle ;

iii) ou tricyclique ABC suivant :

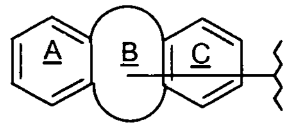

dans lequel les deux cycles A, C comportent éventuellement un hétéroatome, et le cycle B est un cycle à 5, 6 ou 7 chaînons particulièrement à 6 chaînons et contient au moins un hétéroatome comme pypéridyle, pyranyle ;

■ hétérocycloalkyle éventuellement substitué, éventuellement cationique, le groupe hétérocycloalkyle représente notamment un groupe monocyclique saturé ou partiellement saturé à 5, 6 ou 7 chaînons comprenant de 1 à 4 hétéroatomes choisis parmi l'oxygène, le soufre et l'azote, tel que di/tétrahydrofuranyle, di/tétrahydrothiophényle, di/tétrahydropyrrolyle, di/tétrahydropyranyle, di/tétra/hexahydrothiopyranyle, dihydropyridyle, pipérazinyle, pipéridinyle, tétraméthylpipéridinyle, morpholinyle, di/tétra/hexahydroazépinyle, di/tétrahydropyrimidinyle ces groupes étant éventuellement substitués par un ou plusieurs groupes comme $(C_1\text{-}C_4)$alkyle, oxo ou thioxo ; ou l'hétérocycle représente le groupe suivant :

14

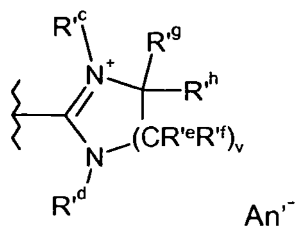

dans lequel $R'^c$, $R'^d$, $R'^e$, $R'^f$, $R'^g$ et $R'^h$, identiques ou différents représentent un atome d'hydrogène ou un groupe $(C_1\text{-}C_4)$ alkyle, ou alors deux groupe $R'^g$ avec $R'^h$, et/ou $R'^e$ avec $R'^f$ forment un groupe oxo ou thioxo, ou alors $R'^g$ avec $R'^e$ forment ensemble un cycloalkyle ; et v représente un entier compris inclusivement entre 1 et 3 ; préférentiellement $R'^c$ à $R'^h$ représentent un atome d'hydrogène ; et $An'^-$ représente un contre-ion anionique;

■ isothiouronium ;
■ $-C(NR'^cR'^d)=N^+R'^eR'^f$; $An^-$ avec $R'^c$, $R'^d$, $R'^e$ et $R'^f$, identiques ou différents représentent un atome d'hydrogène ou un groupe $(C_1\text{-}C_4)$alkyle ; préférentiellement $R'^c$ à $R'^f$ représentent un atome d'hydrogène ; et $An'^-$ représente un contre-ion anionique;
■ isothiourée ;
■ $-C(NR'^cR'^d)=NR'^e$; $An^-$ avec $R'^c$, $R'^d$, $R'^e$ et $An^-$ sont tels que définis précédemment ;
■ (di)aryl$(C_1\text{-}C_4)$alkyle éventuellement substitué tel que le 9-anthracénylméthyle, phénylméthyle ou diphénylméthyle éventuellement substitué par un plusieurs groupes notamment choisis parmi $(C_1\text{-}C_4)$ alkyle, $(C_1\text{-}C_4)$ alcoxy comme le méthoxy, hydroxy, alkylcarbonyle, (di)$(C_1\text{-}C_4)$(alkyl)amino comme le diméthylamino ;
■ (di)hétéroaryl$(C_1\text{-}C_4)$akyle éventuellement substitué, le groupe hétéroaryle est notamment, cationique ou non, monocyclique, comprenant 5 ou 6 chaînons et de 1 à 4 hétéroatomes choisis parmi l'azote, l'oxygène et le soufre, tels que les groupes pyrrolyle, furanyle, thiophényle, pyridyle, pyridyle N-oxyde tels que le 4-pyridyle ou 2-pyridyl-N-oxyde, pyrylium, pyridinium, triazinyle, éventuellement substitué par un ou plusieurs groupe tel que alkyle particulièrement méthyle, avantageusement le (di)hétéroaryl$(C_1\text{-}C_4)$akyle est (di)hétéroarylméthyle ou (di) hétéroaryléthyle ;
■ $-CR^1R^2R^3$ avec $R^1$, $R^2$ et $R^3$ identiques ou différents, représentant un atome d'halogène ou un groupe choisi parmi :

i) $(C_1\text{-}C_4)$alkyle ;
ii) $(C_1\text{-}C_4)$alcoxy ;
iii) aryle éventuellement substitué tel que phényle éventuellement substitué par un ou plusieurs groupes comme $(C_1\text{-}C_4)$alkyle, $(C_1\text{-}C_4)$alcoxy, hydroxy ;
iv) hétéroaryle éventuellement substitué tel que thiophényle, furanyle, pyrrolyle, pyranyle, pyridyle, éventuellement substitué par un groupe $(C_1\text{-}C_4)$alkyle ;

v) $P(Z^1)R'^1R'^2R'^3$ avec $R'^1$ et $R'^2$ identiques ou différents représentent un groupe hydroxy, $(C_1\text{-}C_4)$alcoxy ou alkyle, $R'^3$ représente un groupe hydroxy ou $(C_1\text{-}C_4)$alcoxy, et $Z^1$ représente un atome d'oxygène ou de soufre ;

- cyclique stériquement encombré ; et
- alcoxyalkyle éventuellement substitué tels que le méthoxyméthyle (MOM), éthoxyéthyle (EOM) et l'isobutoxyméthyle.

Selon un mode de réalisation particulier les colorants fluorescents thiols protégés de formule (II) comportent un groupe Y i) hétéroaryle monocyclique à 5 ou 6 chaînons aromatiques, cationiques comprenant de 1 à 4 hétéroatomes choisis parmi l'oxygène, le soufre et l'azote, tels que oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyle, pyrazinium, pyridazinium, triazinium, tétrazinium, oxazépinium, thiépinyle, thiépinium, imidazolium ; ii) hétéroaryle bicyclique à 8 à 11 chaînons cationique tels que indolinium, benzoimidazolium, benzoxazolium, benzothiazolium, ces groupes hétéroaryle mono ou bicycliques étant éventuellement substitués par un ou plusieurs groupes tels que alkyle comme méthyle, ou polyhalogéno$(C_1\text{-}C_4)$alkyle comme trifluorométhyle; iii) ou hétérocyclique suivant :

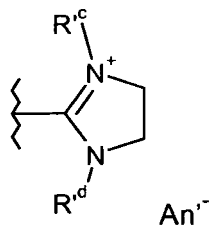

dans lequel $R'^c$ et $R'^d$, identiques ou différents, représentent un atome d'hydrogène ou un groupe $(C_1\text{-}C_4)$alkyle ; préférentiellement $R'^c$ à $R'^d$ représentent un groupe $(C_1\text{-}C_4)$alkyle tel que méthyle ; et $An'^-$ représente un contre-ion anionique.

Particulièrement Y représente un groupe choisi parmi oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium et imidazolium, benzoimidazolium, benzoxazolium, benzothiazolium, ces groupes étant éventuellement substitués par un ou plusieurs groupes $(C_1\text{-}C_4)$ alkyle notamment méthyle.

En particulier Y représente un métal alcalin ou un groupe protecteur tel que:

- ➢ $(C_1\text{-}C_4)$alkylcarbonyle comme méthylcarbonyle ou éthylcarbonyle ;
- ➢ arylcarbonyle comme phénylcarbonyle ;
- ➢ $(C_1\text{-}C_4)$alkoxycarbonyle ;
- ➢ aryloxycarbonyle ;
- ➢ aryl$(C_1\text{-}C_4)$alkoxycarbonyle ;
- ➢ (di) $(C_1\text{-}C_4)$ (alkyl)aminocarbonyle comme diméthylaminocarbonyle;
- ➢ $(C_1\text{-}C_4)$(alkyl)arylaminocarbonyle ;
- ➢ aryle éventuellement substitué tel que le phényle ;
- ➢ hétéroaryle monocyclique à 5 ou 6 chaînons tels que imidazolyle ou pyridyle ;
- ➢ hétéroaryle monocyclique cationique à 5, 6 chaînons tels que pyrylium, pyridinium, pyrimidinium, pyrazinium, pyridazinium, triazinium, imidazolium ; ces groupes étant éventuellement substitués par un ou plusieurs, identiques ou différents, groupes $(C_1\text{-}C_4)$alkyle tel que méthyle ;
- ➢ hétéroaryle bicyclique cationique à 8 à 11 chaînons tels qu benzoimidazolium, ou le benzoxazolium ; ces groupes étant éventuellement substitué par un ou plusieurs, identiques ou différents, groupes $(C_1\text{-}C_4)$alkyle tel que méthyle ;
- ➢ hétérocycle cationique de formule suivante :

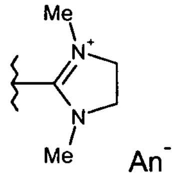

- ➢ isothiouronium $-C(NH_2)=N^+H_2$; $An^-$;
- ➢ isothiourée $-C(NH_2)=NH$ ;
- ➢ $SO_3^-$, $M^+$ avec $M^+$ représentant un métal alcalin tel que le sodium ou le potassium, ou alors $An^-$ de la formule (II) et $M^+$ sont absents.

Conformément à un mode de réalisation particulier de l'invention les colorants fluorescents disulfures de formule **(I)** présentent un axe de symétrie C2 entre les deux atomes de soufre du radical central disulfure.

Selon un mode particulier de l'invention, les colorants de l'invention appartiennent à une des deux formules (**Ia**), (**IIa**) qui possèdent le groupe phényle portant le groupe amino $R_cR_dN$- préférentiellement en para, soit en position 1-4, du groupe styryle :

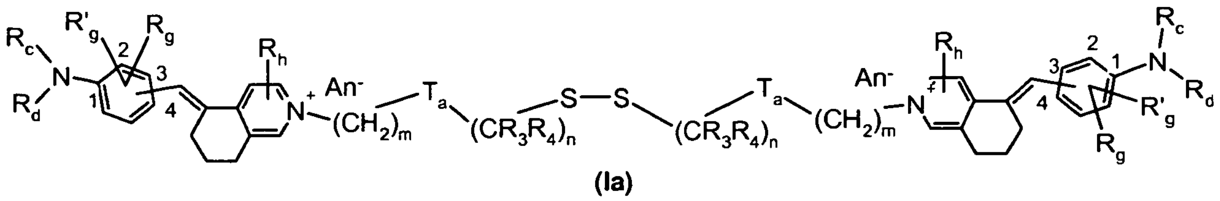

**(Ia)**

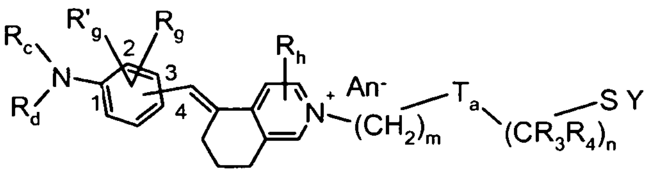

**(IIa)**

avec pour les formules (**Ia**), (**IIa**) :

➢ $R_3$ et $R_4$, identiques ou différents, représentent :

- un atome d'hydrogène,
- un groupe $(C_1\text{-}C_4)$alkyle,
- $-C(O)O^-M^+$ avec $M^+$ représentant un métal alcalin ou $M^+$ et $An^-$ sont absents,
- Carboxy ;

➢ $R_g$, $R'_g$, et $R_h$, identiques ou différents, représentent un atome d'hydrogène, d'halogène, un groupe (di)$(C_1\text{-}C_4)$(alkyl)amino, hydroxy, acylamino, $(C_1\text{-}C_4)$alcoxy, $(C_1\text{-}C_4)$alkylcarbonylamino ou un radical $(C_1\text{-}C_6)$alkyle ; particulièrement $R_g$, $R'_g$, et $R_h$, représentent un atome d'hydrogène ;

➢ deux groupes $R_c$ et $R'_g$ et/ou $R_d$ et $R_g$, forment ensemble un hétérocycle saturé ou un hétéroaryle, éventuellement substitué par un ou plusieurs groupe $(C_1\text{-}C_6)$alkyle, notamment un hétérocycle et l'hétéroaryle contenant un ou deux hétéroatomes choisis parmi l'azote et l'oxygène, l'hétérocycle contenant entre 5 et 7 chaînons et l'hétéroaryle contenant entre 7 et 11 chaînons; particulièrement l'hétérocycle est choisi parmi les groupe morpholinyle, pypérazinyle, pypéridinyle et pyrrolidinyle, et l'hétéroaryle est un indolyle ;

➢ $R_c$ et $R_d$, identiques ou différents, représentent un atome d'hydrogène, un groupe $(C_1\text{-}C_6)$alkyle éventuellement substitué, aryl$(C_1\text{-}C_4)$alkyle, un groupe $(C_1\text{-}C_6)$alkyle éventuellement substitué ; $R_c$ et $R_d$ représentent particulièrement un groupe $(C_1\text{-}C_4)$alkyle, ou un groupe $(C_1\text{-}C_4)$ alkyle substitué par i) un groupe hydroxy, ii) amino, iii) (di)$(C_1\text{-}C_3)$alkylamino, ou iv) ammonium quaternaire (R")(R"')(R"")$N^+$-, plus particulièrement substitué par le groupe alkyle est substitué par un groupe hydroxy ;

➢ ou alors les deux radicaux $R_c$ et $R_d$ portés par le même atome d'azote forment ensemble un groupe hétérocyclique ou hétéroaryle ; particulièrement l'hétérocycle est monocyclique et comprend entre 5 et 7 chaînons et est éventuellement substitué par particulièrement un ou deux groupes, identiques ou différents, hydroxy et/ou $(C_1\text{-}C_4)$alkyle ; plus particulièrement les groupes sont choisis parmi le pipéridino, le pyrrolidino ; ledit groupe hétérocyclique étant substitué, en particulier par 1 ou 2 substituants hydroxy ;

➢ $T_a$, représente une liaison covalente σ, ou un groupe choisi parmi : -N(R)-, -C(O)-N(R)-, -N(R)-C(O)- ; particulièrement -C(O)-N(R)-, -N(R)-C(O)- ou une liaison covalente ;

➢ m et n, identiques ou différents, représentent un entier compris inclusivement entre 1 et 6 avec la somme m+n représente un entier compris inclusivement entre 2 et 6 ; préférentiellement m = 1 et/ou n = 1, 2, ou 3 ;

➢ $An^-$ représentant un contre-ion anionique ; et

➢ Y, est tel que définis précédemment ;

étant entendu que lorsque les composés de formules **(Ia)** ou **(IIa)** contiennent d'autres parties cationiques, ils se trouvent associés à un ou plusieurs contre-ions anioniques permettant d'atteindre l'électroneutralité de la formules **(Ia)** ou **(IIa)**.

A titre d'exemple on peut citer les colorants fluorescents appartenant aux formules (I) et (II) suivantes:

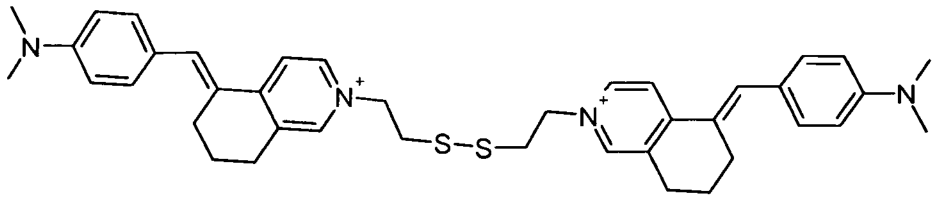

2 $An^-$

**1**

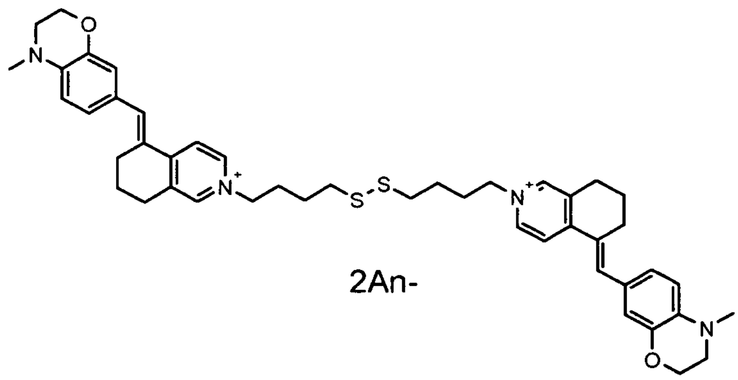

2An-

**2**

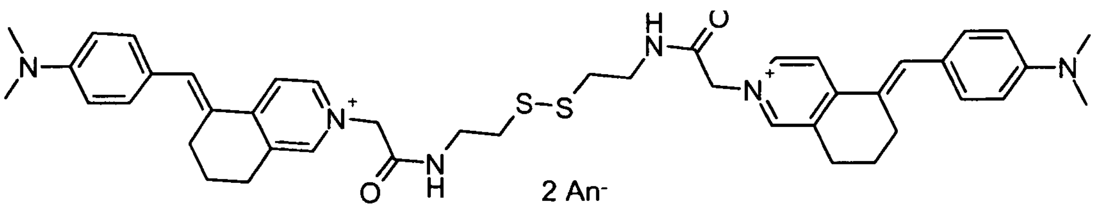

2 $An^-$

**3**

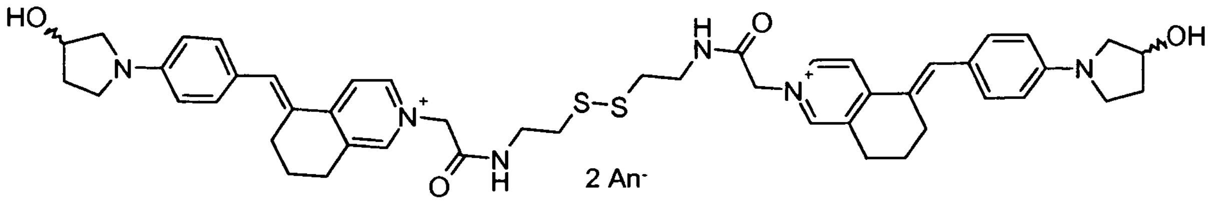

2 $An^-$

**4**

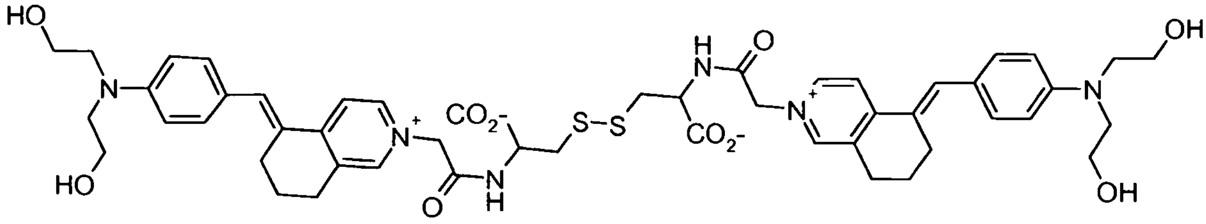

5

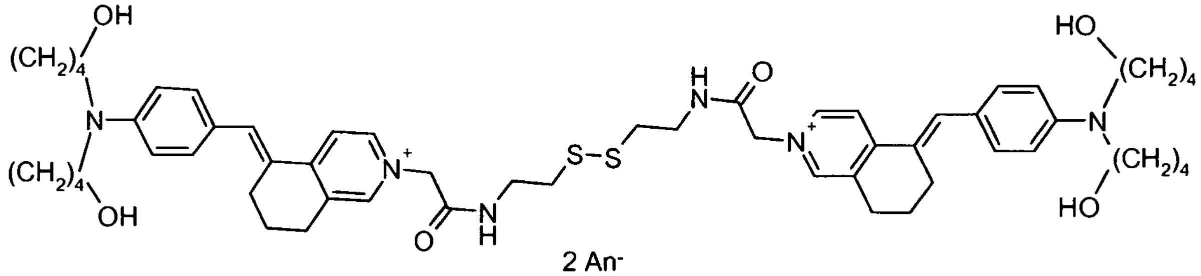

2 $An^-$

6

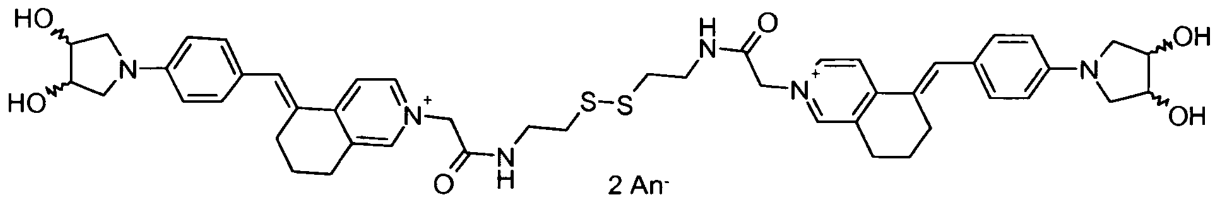

2 $An^-$

7

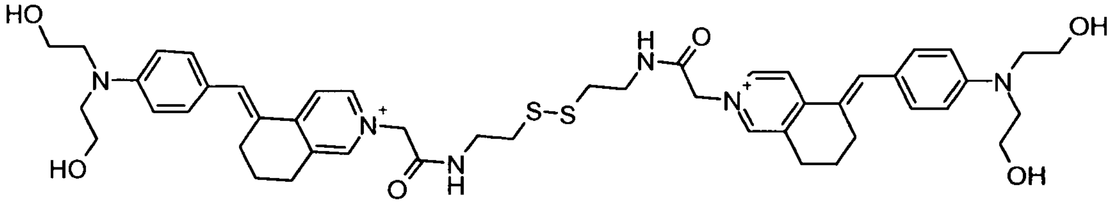

2 $An^-$

8

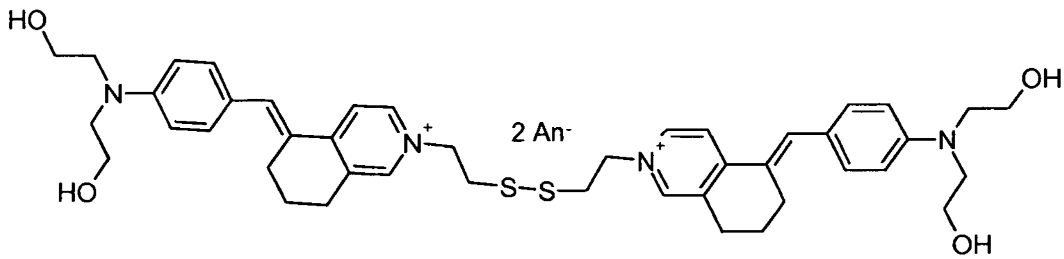

9

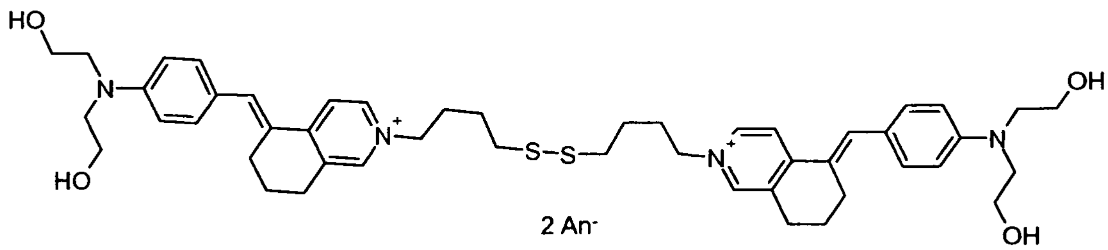

**10**

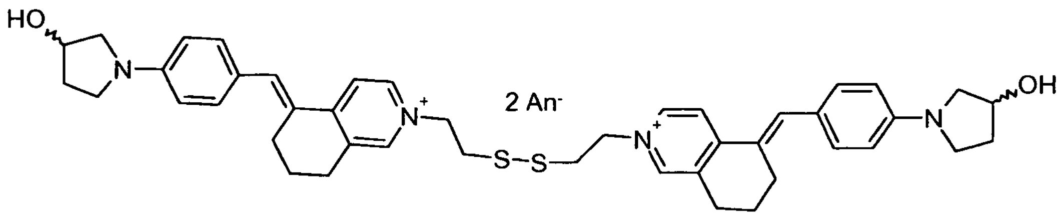

**11**

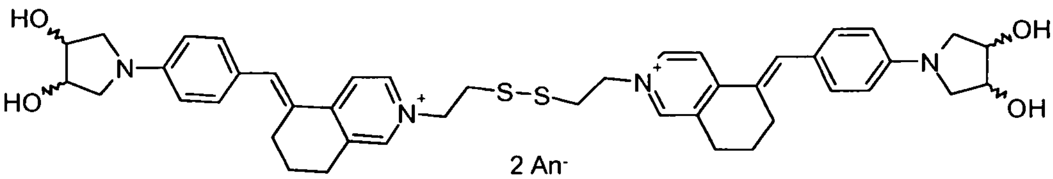

**12**

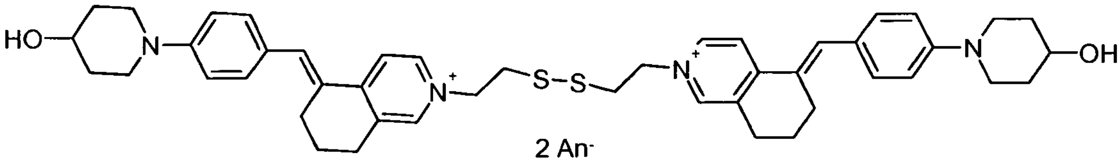

**13**

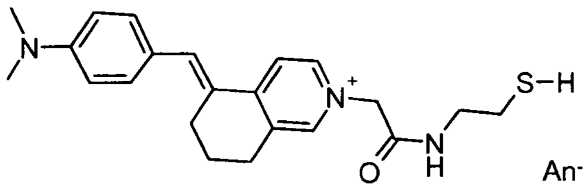

**14**

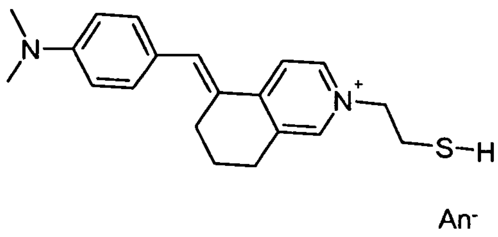

**15**

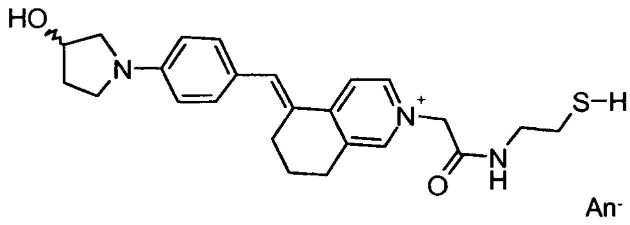

**16**

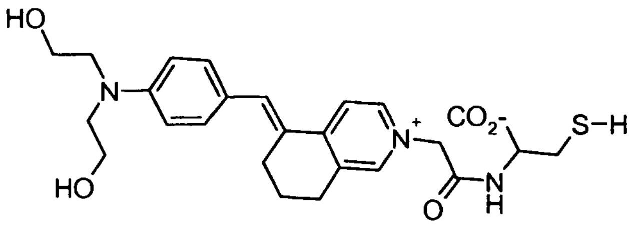

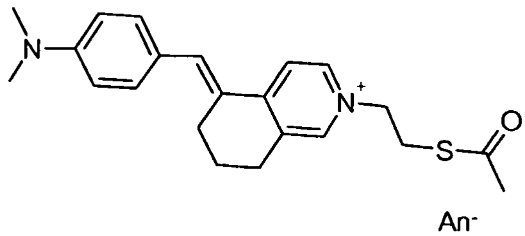

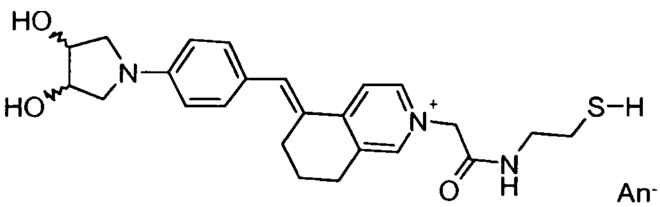

**19**

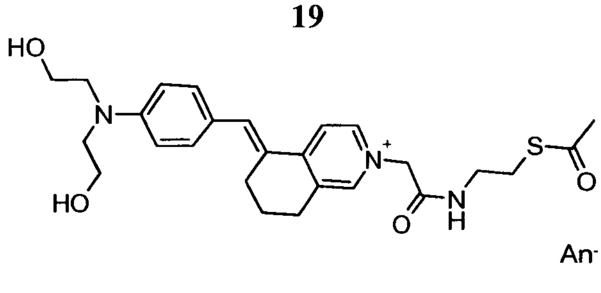

**20**

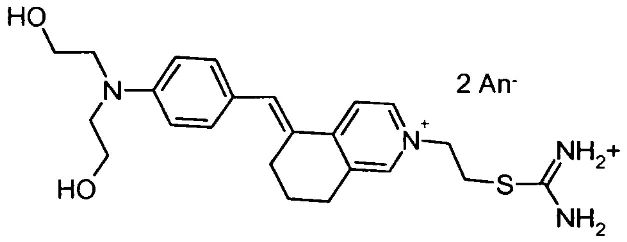

**21**

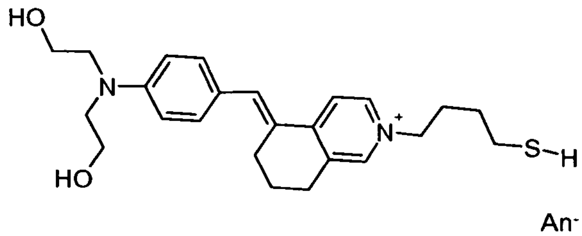

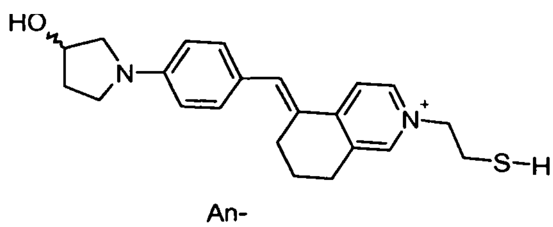

**23**

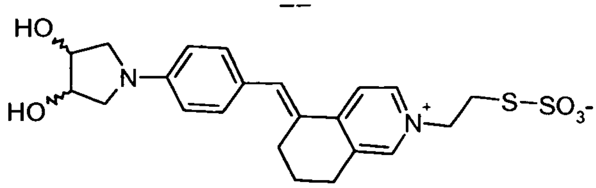

**24**

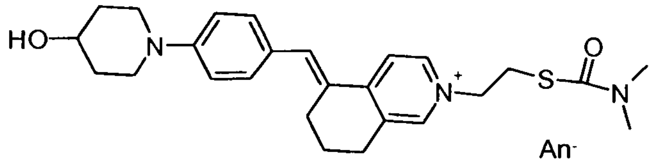

**25**

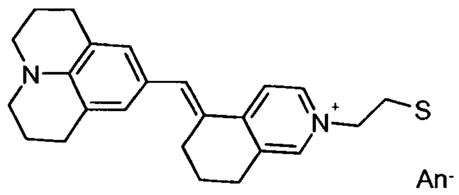

**26**

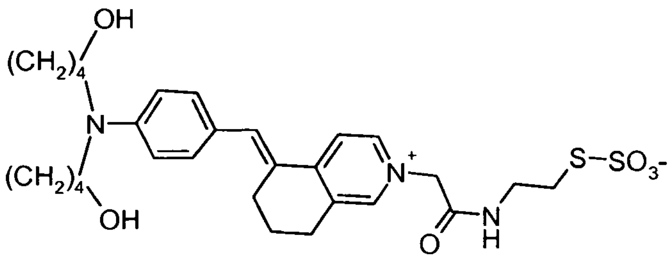

**27**

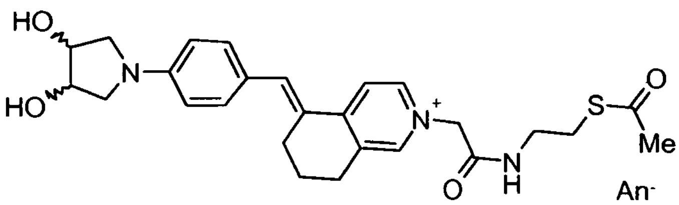

**28**

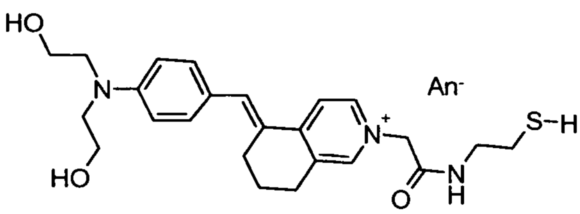

**29**

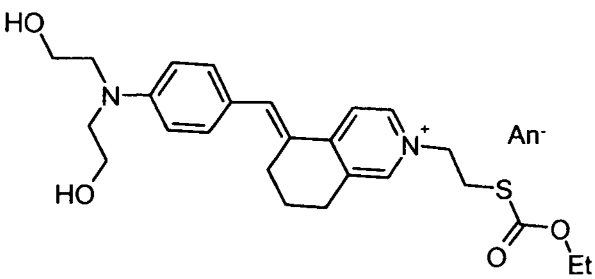

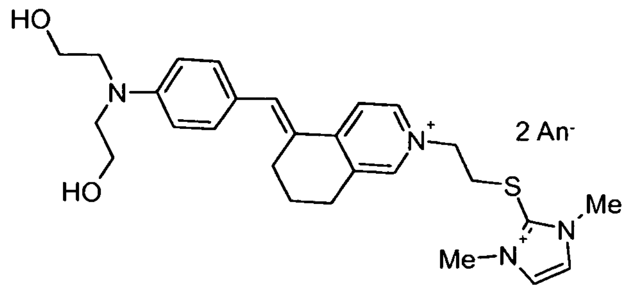

**31**

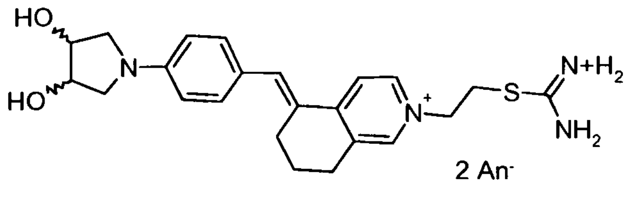

**32**

avec An⁻, identiques ou différents, représentant un contre-ion anionique.

Les colorants thiols protégés de formule **(II')** peuvent être synthétisés en deux étapes. La première étape consistant à préparer le colorant thiol non protégé **(II")** selon les méthodes connues de l'homme de l'art comme par exemple « Thiols and organic Sulfides », « Thiocyanates and Isothiocyanates, organic », Ullmann's Encyclopedia, Wiley-VCH, Weinheim, 2005. Et la deuxième étape consistant à protéger la fonction thiol selon les méthodes classiques connues par l'homme du métier pour conduire aux colorants thiols protégés de formule (**I**I'). A titre d'exemple pour protéger la fonction thiol -SH du colorant thiol on peut utiliser les méthodes des ouvrages « Protective Groups in Organic Synthesis », T. W. Greene, John Willey & Sons ed., NY, 1981, pp.193-217 ; « Protecting Groups », P. Kocienski, Thieme, 3ème ed., 2005, chap. 5.

Nous pouvons illustrer cette méthode par la méthode consistant i) à générer des colorants fluorescents thiols de formule **(II")** par réduction d'un colorant fluorescent à deux chromophores, hétérocyclique, portant une fonction disulfure -S-S- tels que **(I')** et ii) à protéger selon les méthodes classiques ladite fonction thiol de **(II")** avec le réactif **7** Y'R pour accéder aux colorants fluorescents thiols protégés de formule **(II')**. Le composé thiol **(II")** peut également être métallé avec un métal alcalin ou alcalino terreux Met* pour conduire au colorant fluorescent thiolate de formule **(II"')**.

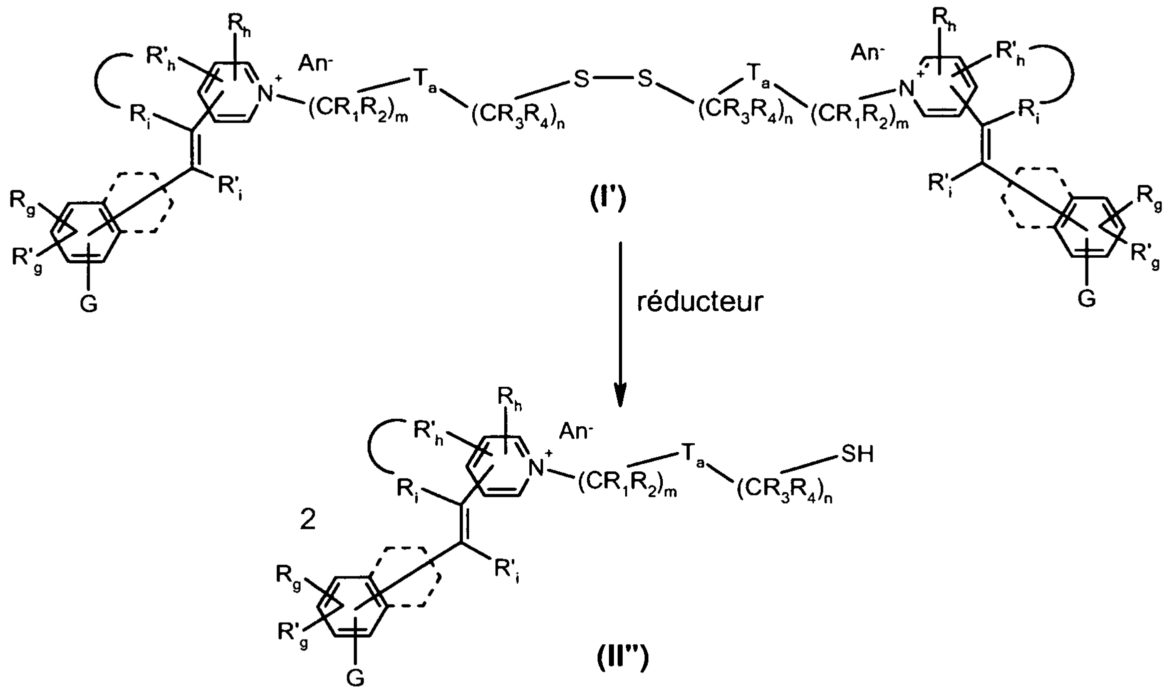


(I')

(II'')

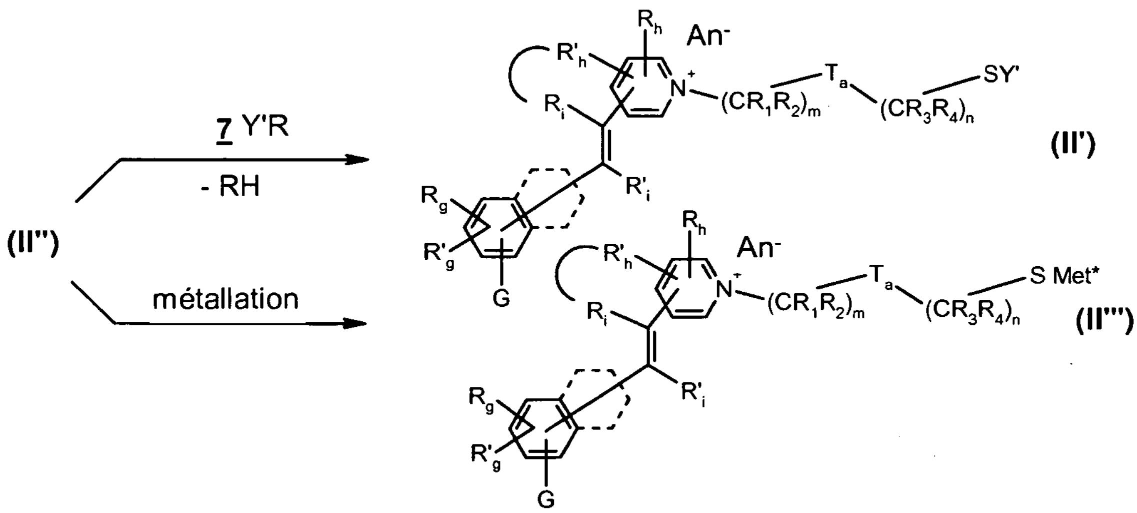


(II'')

(II')

(II''')

avec Y' représentant un groupe protecteur de fonction thiol ; Met* représentant un métal alcalin ou un métal alcalino terreux, particulièrement le sodium ou le potassium, étant entendu que lorsque le métal est un métal alcalino-terreux 2 chromophores à fonction thiolate-S- peuvent être associés à 1 Métal$^{2+}$.

avec $R_1$, $R_2$, $R_3$, $R_4$, $R_g$, $R'_g$, $R_h$, $R'_h$, $R_i$, $R'_i$, m, n, G et $An^-$ sont tels que définis précédemment ; Y' représente un groupe protecteur de fonction thiol ; et R représente un groupe partant nucléofuge, comme par exemple mésylate, tosylate, triflate ou halogénure.

Selon une autre possibilité, on peut faire réagir un composé thiol protégé **(b)** par un groupe protecteur Y' tel que défini précédemment préparé selon une des procédures décrites dans les ouvrages cités précédemment, ledit composé thiol protégé comprenant au moins une fonction nucléophile avec une quantité suffisante, préférentiellement équimolaire,

d'un "chromophore fluorescent réactif" ou d'un composé comprenant un tel "chromophore fluorescent réactif" **(a)**. En d'autres termes **(a)** comprend une fonction électrophile pour former un groupement de liaison ou une liaison covalente $\Sigma$ comme cela peut être schématisé ci-dessous dans la préparation de colorants fluorescents de formule **(II')** :

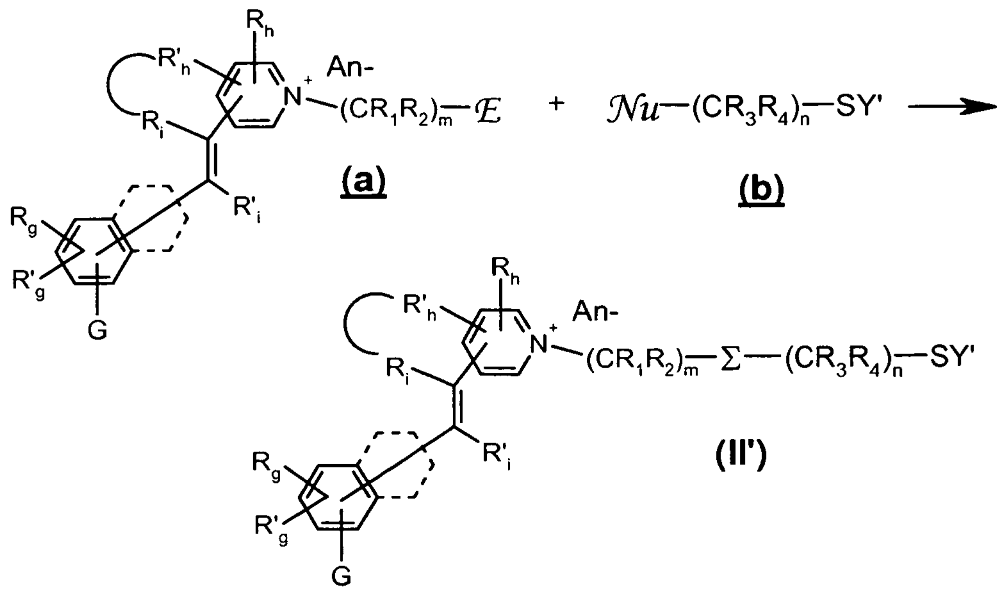

(a)

(b)

(II')

avec $R_1$, $R_2$, $R_3$, $R_4$, $R_g$, $R'_g$, $R_h$, $R'_h$, $R_i$, $R'_i$, m, n, G, Y' et $An^-$ sont tels que définis précédemment ; *Nu* représentant un groupe nucléophile ; *E* représentant un groupe électrophile ; et Σ le groupement de liaison générée après attaque du nucléophile sur l'électrophile étant entendu que ∑ est un sous-ensemble de la définition de Ta telle que décrite dans les formules (I) et (II).

A titre d'exemple, les liaisons covalentes ∑ pouvant être générées sont répertoriées dans le tableau ci-dessous à partir de condensation d'électrophiles avec des nucléophiles :

| Electrophiles E | Nucléophiles Nu | Liaisons covalentes ∑ |
|---|---|---|
| Esters activés* | Amines | Carboxamides |
| Azotures d'acyles** | Amines | Carboxamides |
| Haloqénures d'acyles | Amines | Carboxamides |
| Haloqénures d'acyles | Alcools | Esters |
| Cyanures d'acyles | Alcools | Esters |
| Cyanures d'acyles | Amines | Carboxamides |
| Halogénures d'alkyles | Amines | Alkylamines |
| Halogénures d'alkyles | Acides carboxyliques | Esters |
| Halogénures d'alkyles | Thiols | Thioesters |
| Halogénures d'alkyles | Alcools | Ethers |
| Acides sulfoniques et leurs sels | Thiols | Thioéthers |
| Acides sulfoniques et leurs sels | Acides carboxyliques | Esters |
| Acides sulfoniques et leurs sels | Alcools | Ethers |
| Anhydrides | Alcools | Esters |
| Anhydrides | Amines | Carboxamides |
| Halogénures d'aryles | Thiols | Thioéthers |
| Halogénures d'aryles | Amines | Arylamines |
| Aziridines | Thiols | Thioéthers |
| Acides carboxyliques | Amines | Carboxamides |

(suite)

| Electrophiles E | Nucléophiles Nu | Liaisons covalentes Σ |
|---|---|---|
| Acides carboxyliques | Alcools | Esters |
| Carbodiimides | Acides carboxyliques | N-acylurées |
| Diazoalcanes | Acides carboxyliques | Esters |
| Epoxides | Thiols | Thioéthers |
| Haloacétamides | Thiols | Thioéthers |
| Esters imidiques | Amines | Amidines |
| Isocyanates | Amines | Urées |
| Isocyanates | Alcools | Uréthanes |
| Isothiocyanates | Amines | Thiourées |
| Maléimides | Thiols | Thioéthers |
| Esters sulfoniques | Amines | Alkylamines |
| Esters sulfoniques | Thiols | Thioéthers |
| Esters sulfoniques | Acides carboxyliques | Esters |
| Esters sulfoniques | Alcools | Ethers |
| Halogénures de sulfonyle | Amines | Sulfonamides |
| **les esters activées de formule générale -CO-Part avec Part représentant un groupe partant tel que oxysuccinimidyle, oxybenzotriazolyle, aryloxy éventuellement substitué ;*<br>*** les azotures d'acyles peuvent* se *réarranger pour donner les isocyanates.* | | |

Une variante à ce procédé est d'utiliser un chromophore fluorescent possédant une fonction acrylate électrophile (-OCO-C=C-) sur laquelle on effectue une réaction d'addition qui génèrera une liaison Σ.

On pourra également utiliser un réactif thiol (α) : Y'-SH comprenant un groupe Y' tel que défini précédemment dont la fonction nucléophile SH peut réagir sur l'atome de carbone en alpha de l'atome d'halogène porté par un chromophore fluorescent pour conduire au colorant fluorescent thiol protégé de formule **(II')**:

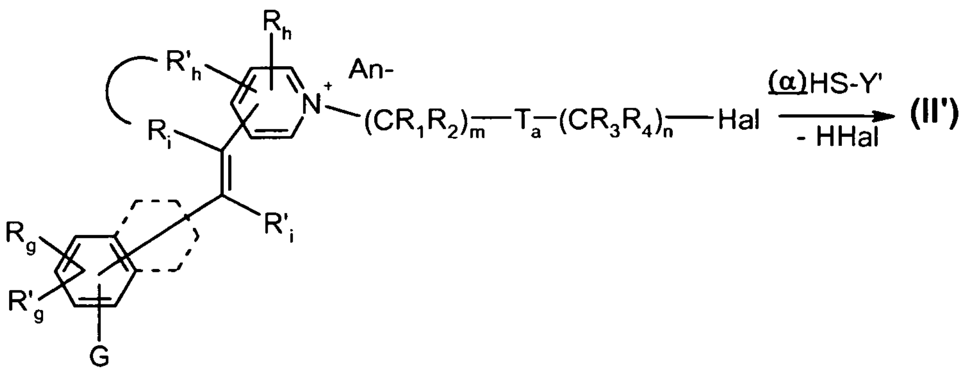


avec $R_1$, $R_2$, $R_3$, $R_4$, $R_g$, $R'_g$, $R_h$, $R'_h$, $T_a$, $R_i$, $R'_i$, m, n, G, Y', **(II')** et $An^-$ sont tels que définis précédemment, et Hal représentant un atome d'halogène nucléofuge tel que le brome, l'iode ou le chlore.

Plus particulièrement on pourra substituer un groupe partant nucléofuge par un dérivé d'une thiourée (S=C(NRR)NRR) ou de la thiourée pour générer les isothiouroniums. Par exemple si le dérivé thiourée est une thioimidazolinium (β), le schéma réactionnel est le suivant :

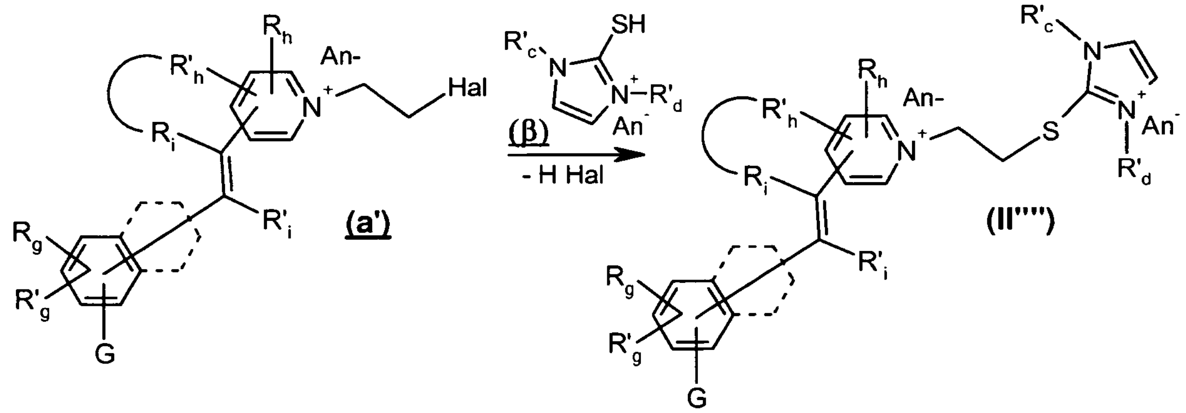


avec R'$_c$, R'$_d$, R$_g$, R'$_g$, R$_h$, R'$_h$, R$_i$, R'$_i$, m, n, G, Hal, An$^-$ sont tels que définis précédemment.

Une autre variante peut permettre d'accéder au composé **(II""sat)** à partir d'un dérivé de thiourée cyclique de type imidazoline **(b')**, suivi de l'alkylation de ladite imidazoline à l'aide de R'$_d$-Gp avec Gp groupe partant tel que chlorure, bromure, tosylate, mésylate :

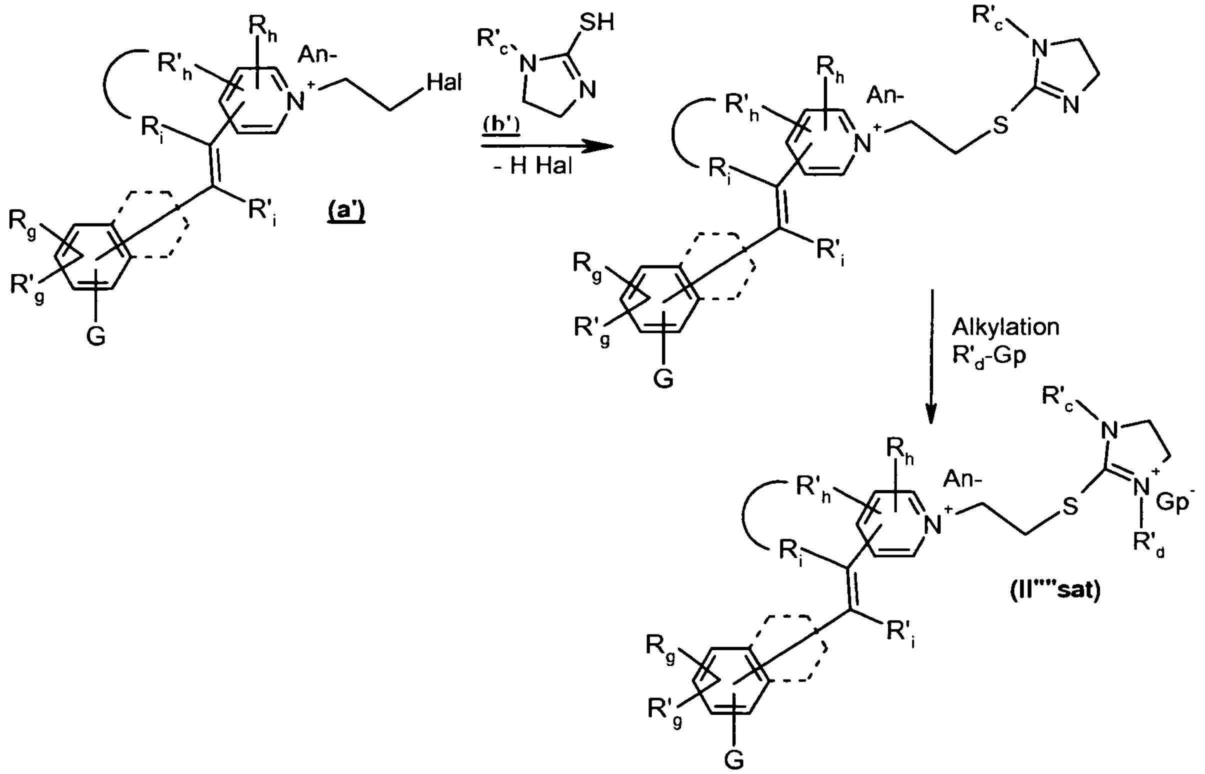


avec R'$_c$, R'$_d$, R$_g$, R'$_g$, R$_h$, R'$_h$, R$_i$, R'$_i$, m, n, G, Hal, An$^-$ sont tels que définis précédemment.

Une variante est d'utiliser à la place de l'halogénure comprenant le chromophore fluorescent <u>(a')</u> un chromophore comprenant un autre type de nucléofuge tel que le tosylate, mésylate.

Conformément à une autre possibilité, certains colorants fluorescents thiols protégés **(II')** peuvent être obtenus en faisant réagir un composé thiol protégé, avec un composé portant deux fonctions acide carboxylique activées selon les méthodes classiques (par exemple réaction avec un carbodiimide ou avec le chlorure de thionyle). Le produit résultant **<u>(d)</u>** est ensuite mis à réagir avec un chromophore fluorescent porteur d'une fonction nucléophile **<u>(c)</u>**, par exemple de type amine primaire ou secondaire, ou de type alcool aliphatique.

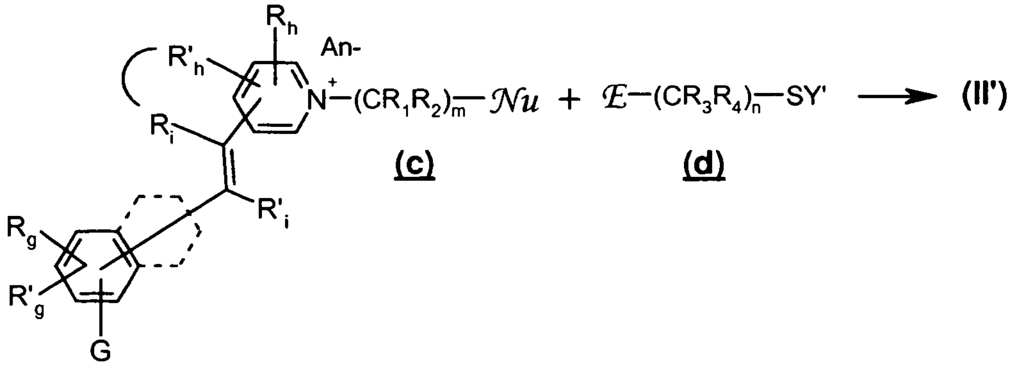


avec $R_1$, $R_2$, $R_3$, $R_4$, $R_g$, $R'_g$, $R_h$, $R'_h$, $R_i$, $R'_i$, G, $T_a$, Y', m, n, AN-, *E*, *Nu* et (II') tels que définis précédemment.

Une autre variante est d'utiliser un dérivé de thiolactone tel que représenté par le schéma ci-dessous :

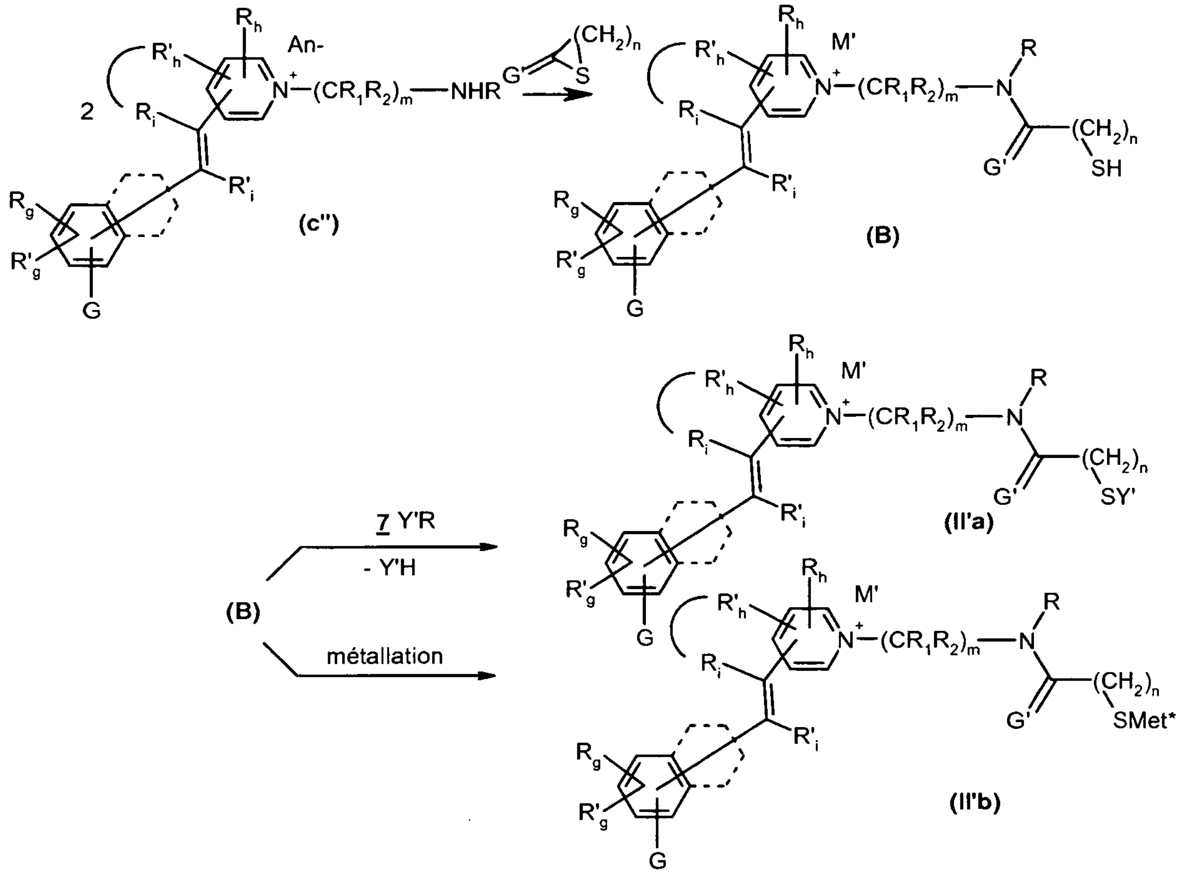


Avec $R_1$, $R_2$, $R_g$, $R'_g$, $R_h$, $R'_h$, $R_i$, $R'_i$, G, $T_a$, Y', Met*, n, m, $An^-$, tels que définis précédemment, G' représentant un atome d'oxygène, de soufre ou un groupe NR' avec R' représentant un atome d'hydrogène ou un radical alkyle, et R représentant un atome d'hydrogène, un radical alkyle en $C_1$-$C_4$, hydroxyalkyle en $C_1$-$C_4$ ou un aryl($C_1$-$C_4$)alkyle. Le dérivé thiolactone est préférentiellement choisi avec n=3 et G' représente un atome d'oxygène.

Une variante de synthèse est de combiner à la première voie la voie précédente ie à partir de deux équivalents du réactif nucléophile <u>**(c)**</u> avec un réactif diéléctrophile disulfure <u>**(i)**</u> il est possible de générer après condensation le produit dichromophorique disulfure **(I")**, ce dernier pouvant subir une réduction pour former le colorant thiol fluorescent hétérocyclique qui a son tour peut soit être protégé pour former le colorant fluorescent thiol protégé **(II')** ou soit être métallé par un métal alcalin pour conduire au colorant fluorescent thiol hétérocyclique métallé **(II"')** :

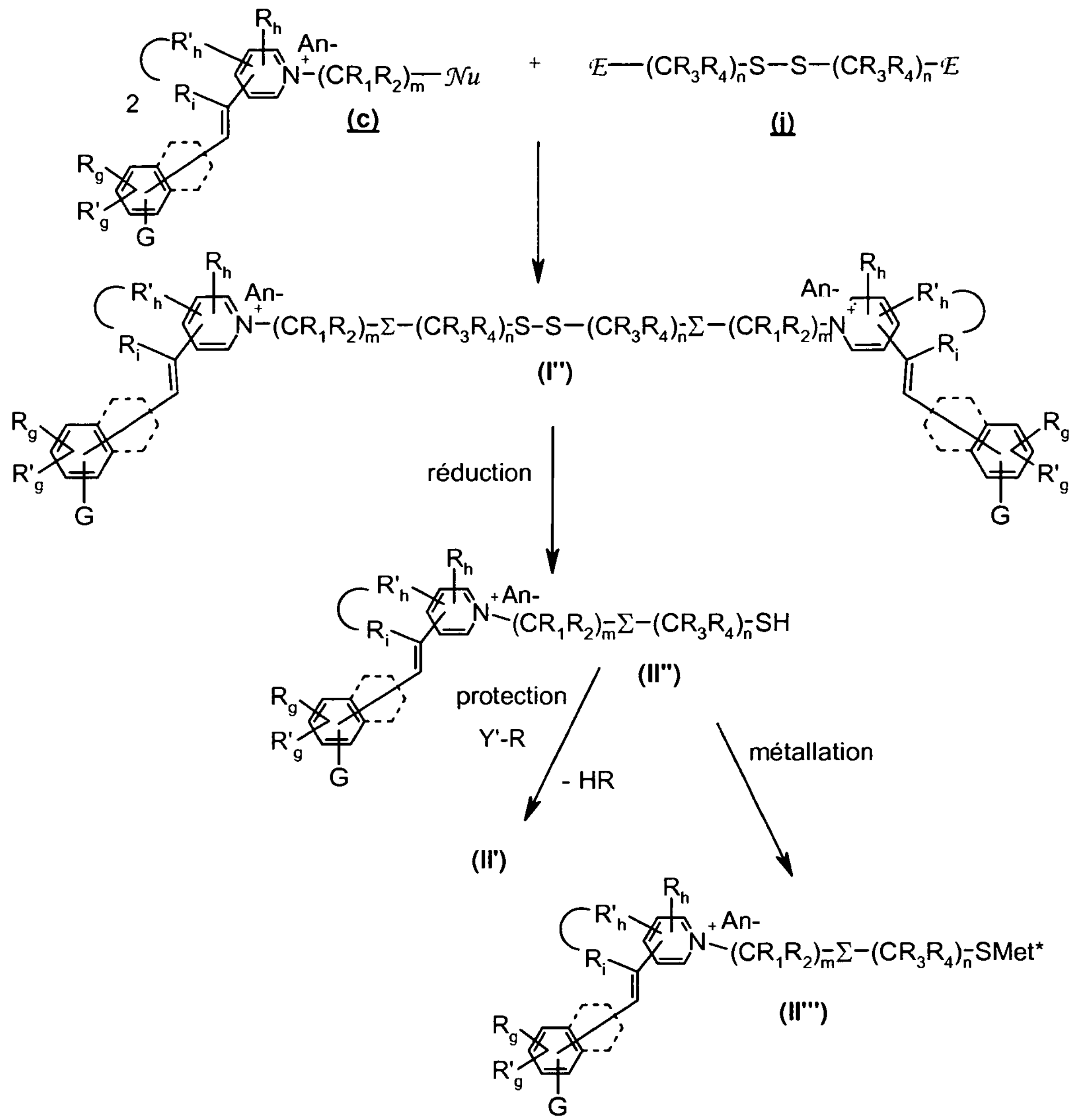


Conformément à une autre possibilité, les colorants fluorescents thiols protégés de formule **(II')** peuvent être obtenus par réaction d'un composé comprenant un groupe thiol protégé par un groupe Y', et un groupe hydroxy activé préalablement en groupe partant nucléofuge **(d'),** comme par exemple mésylate, tosylate, triflate ou halogénure avec un chromophore styrylpyridine **(c').**

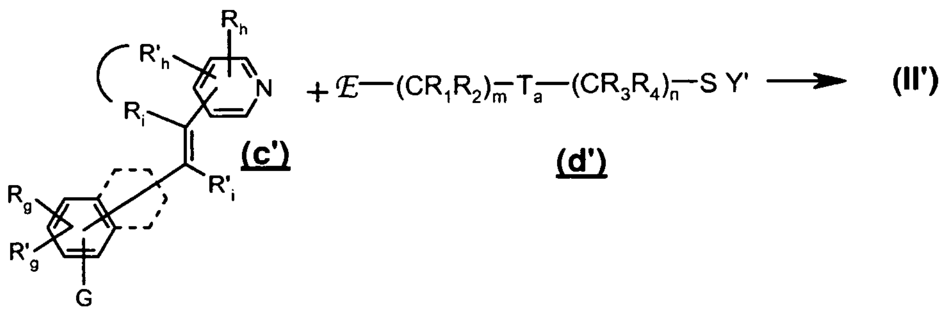

Avec $R_1$, $R_2$, $R_3$, $R_4$, $R_g$, $R'_g$, $R_h$, $R'_h$, $R_i$, $R'_i$, G, $T_a$, Y', m, n, **(II')** et *E* sont tels que définis précédemment.

A titre d'exemple un composé contenant un groupe thiol protégé, contient un groupe partant nucléofuge R, comme par exemple mésylate, tosylate, ou triflate qui peut subir l'attaque nucléophile de l'amine portée par le chromophore fluorescent styrylique :

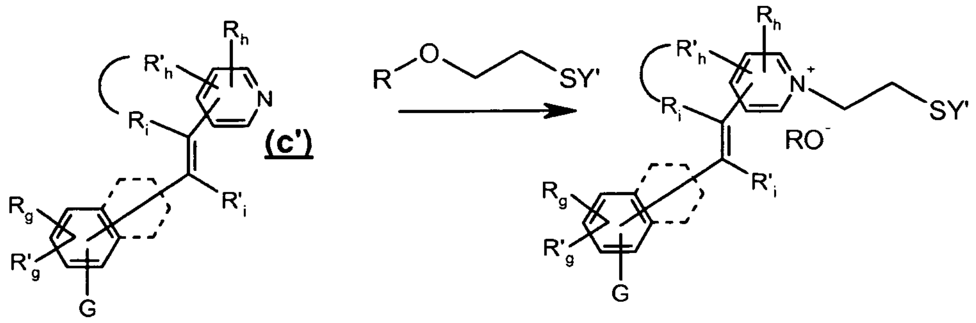

(c')

Une autre alternative provient de l'utilisation d'halogénures comme groupe partant nucléofuge sur un composé thiol pouvant être substitué par une fonction amine primaire par exemple portée par un chromophore fluorescent styrylique :

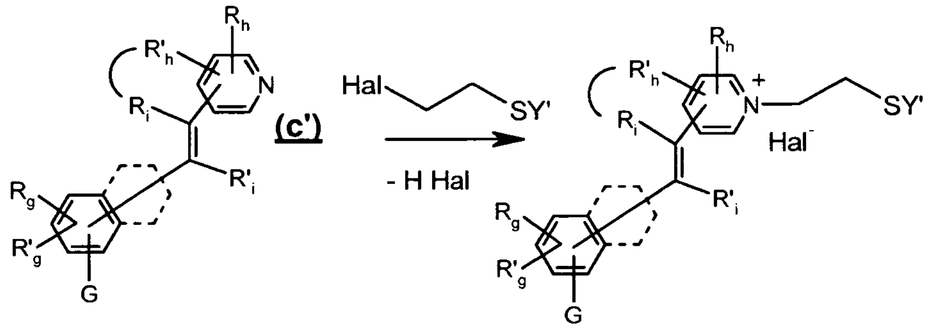


(c')

Conformément à une autre possibilité, les colorants fluorescents thiols de formule **(II)** selon l'invention peuvent être obtenus par réaction d'un composé comprenant un groupe thiol Y tel que défini précédemment et un groupe électrophile **(f)** avec un composé pyridinium comprenant un groupe nucléophile. A titre d'exemple, on pourra condenser un aldéhyde ou un thioaldéhyde lorsque G' représente un atome d'oxygène ou un soufre avec un « méthylène activé » tel que l'alkylpyridinium **(e)** pour générer une liaison éthylène >C=C<. Cette réaction est communément appelée condensation de « Knoevenagel ». Par « méthylènes activés » on sous entend ceux qui comporte préférentiellement en position 2 ou 4 du groupe pyridinium un groupe méthylène $R_i$-$CH_2$-:

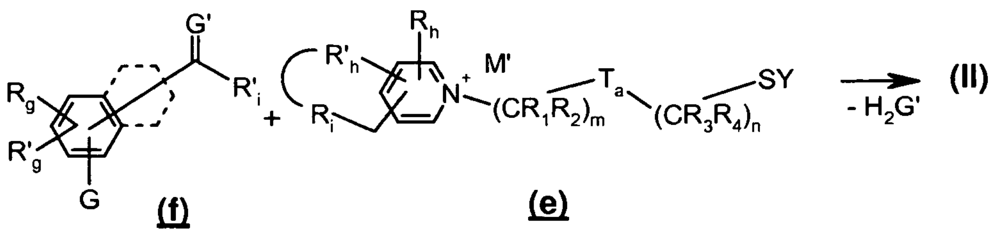


(f) (e) (II)

avec $R_1$, $R_2$, $R_3$, $R_4$, $R_a$, $R_b$, $R_g$, $R'_g$, $R_h$, $R'_h$, $R_i$, $R'_i$, $T_a$, m, n, Y et $An^-$ tels que définis précédemment et G représentant un atome d'oxygène ou de soufre.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S

Y' par la protection du thiol -SH en utilisant les groupes protecteurs classiques. Les colorants fluorescents thiols sont métallés en utilisant également les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Les réactifs de départs sont commerciaux ou accessibles par les méthodes classiques connues par l'homme du métier. A titre d'exemple il est possible de synthétiser le réactif **(I')** à partir de 2 équivalents de dérivé pyridinique **1** et un équivalent de réactif disulfure comprenant deux groupes partants Gp, pour conduire au sel de dipyridinium disulfure **3** qui peut se condenser à son tour avec deux équivalents de composé aryle à groupe aldéhyde/thioaldéhyde **4** pour conduire à **5.**

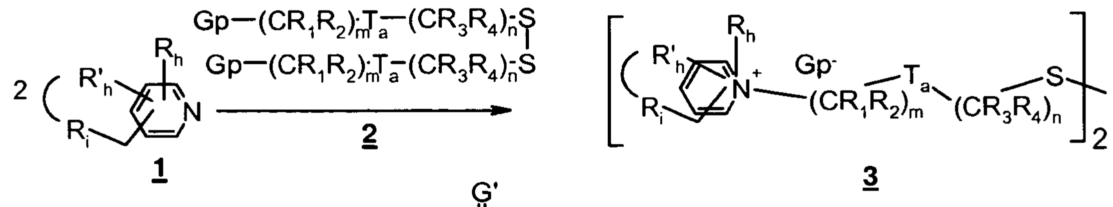
1
2
3

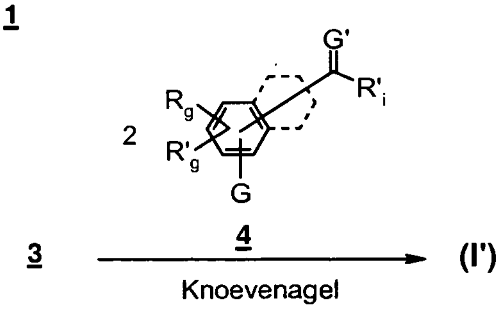

3
4
(I')

avec Gp représentant un groupe partant nucléofuge, comme par exemple mésylate, tosylate, triflate ou halogénure. Les contre-ions Gp- des composés **(I')**, ci-dessus peuvent être remplacés par des contre-ions An$^-$ d'autres natures à partir de méthodes connues par l'homme du métier notamment par résine échangeuse d'ion.

Les colorants disulfures dissymétriques de formule **(I)** peuvent être synthétisés en une étape en faisant réagir un colorant fluorescent thiol non protégé avec un colorant fluorescent thiol protégé par Y' pour former le colorant disulfure de formule **(I).**

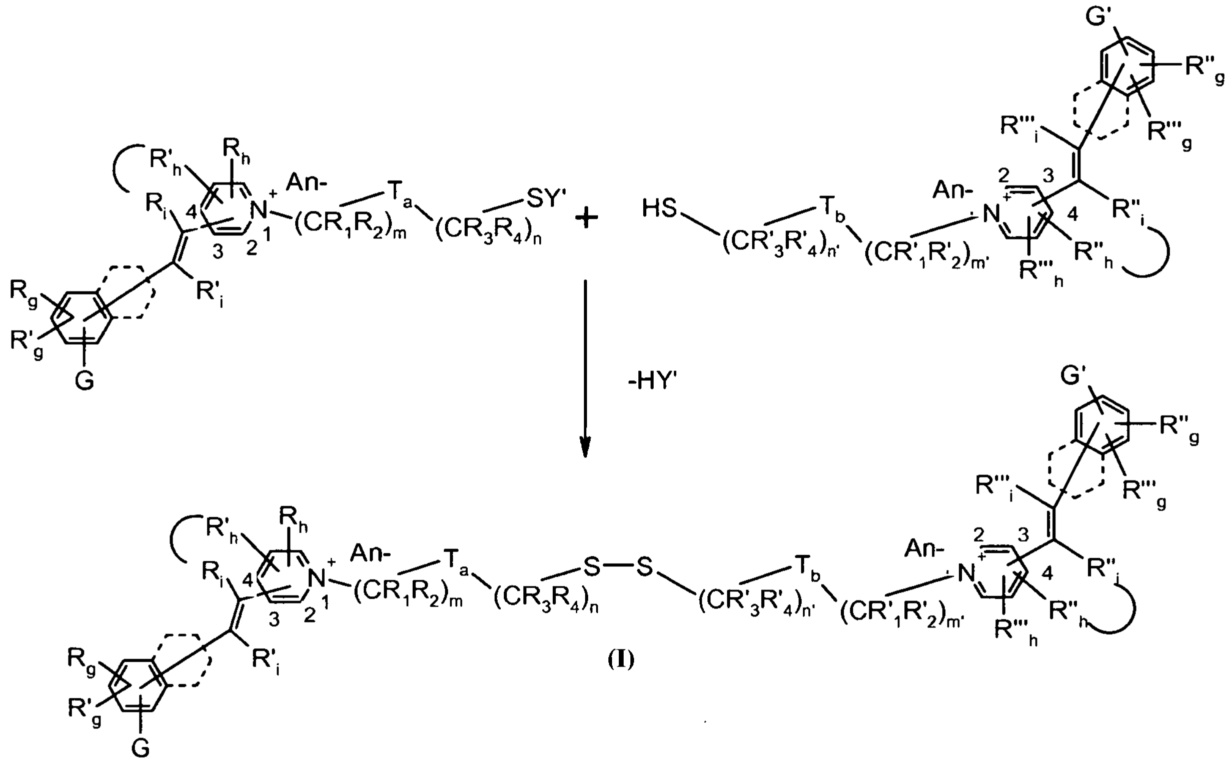


(I)

avec $R_g$, $R'_g$, $R''_g$, $R'''_g$, $R_h$, $R'_h$, $R''_h$, $R'''_h$, $R_i$, $R'_i$, $R'_i$, $R''_i$, m, m', n, n', $T_a$, $T_b$, G, G', Hét et $An^-$ sont tels que définis précédemment ; Y' représente un groupe protecteur de fonction thiol.

Les colorants disulfures fluorescents symétriques de formule **(I')** peuvent être synthétisés par oxydation des colorants hémicyanines thiols **(II).**

L'oxydation peut se faire avec un agent oxydant peut éventuellement être associé. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que les perborates et les persulfates, ainsi que les enzymes parmi lesquelles on peut citer les peroxydases, les oxydo-réductases à 2 électrons tels que les uricases et les oxygénases à 4 électrons comme les laccases. L'utilisation du peroxyde d'hydrogène est particulièrement préférée.

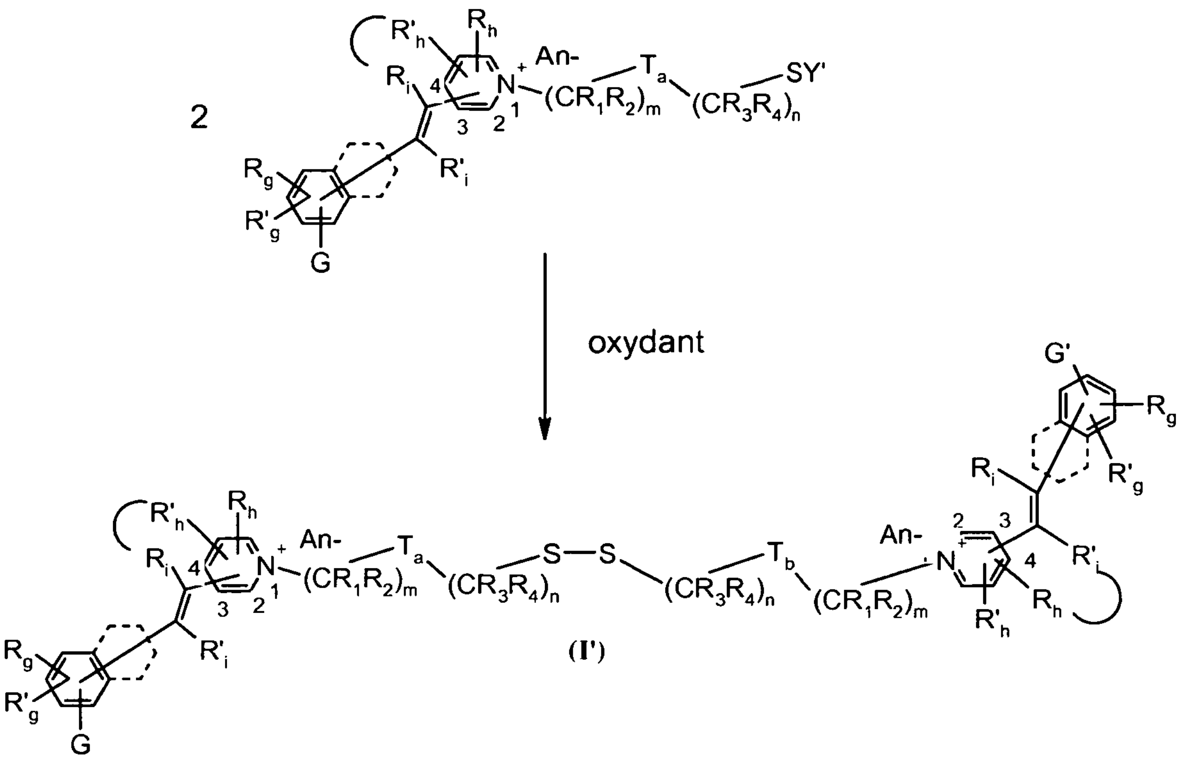


(I')

avec $R_g$, $R'_g$, $R''_g$, $R'''_g$, $R_h$, $R'_h$, $R''_h$, $R'''_h$, $R_i$, $R'_i$, $R''_i$, $R'''_i$, m, m', n, n', $T_a$, $T_b$, G, G' et $An^-$ sont tels que définis précédemment ; Y' représente un atome d'hydrogène ou un métal alcalin, éventuellement un groupement protecteur de thiol dans le cas où ce groupement peut être déprotégé par un agent oxydant ou un agent alcalin (tel que par exemple les groupement acyles).

Selon une autre possibilité, on peut faire réagir un composé disulfure **(b1),** ledit composé disulfure comprenant deux fonctions nucléophiles avec une quantité suffisante, préférentiellement deux équivalents, d'un chromophore **(a1)** hémicyanine styryle, et qui comprend une fonction électrophile pour former un groupement de liaison covalente Σ; voir ci-dessous la préparation de colorants de formule **(I')**

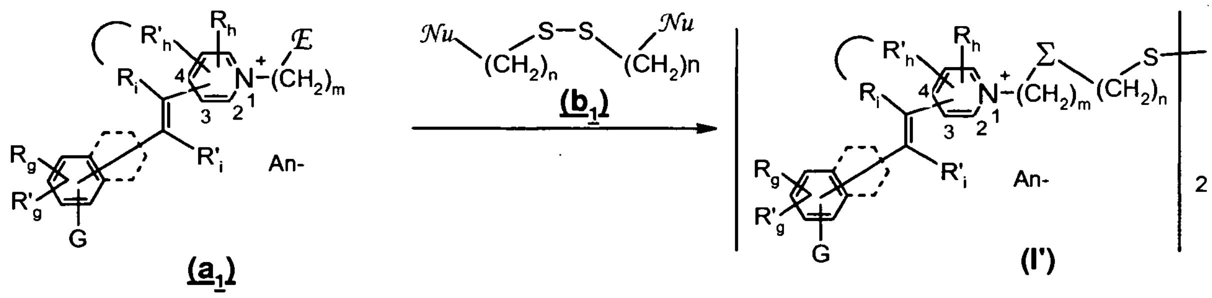

**(a1)** **(b1)** **(I')**

avec $R_g$, $R'_g$, $R_h$, $R'_h$, $R'_i$, $R''_i$, m, n, G, $An^{--}$, Σ, *E* et *Nu* tels que définis précédemment, étant toujours entendu que Σ représente un sous ensemble de Ta défini dans les formules**(I)** et

Une variante à ce procédé est d'utiliser un chromophore hémicyanine styryle possédant une fonction acrylate électrophile (-OCO-C=C-) sur laquelle on effectue une réaction d'addition qui génèrera une liaison covalente dans un groupe de liaison Σ.

Conformément à une autre possibilité, les colorants disulfures de formule **(I')** peuvent être obtenus par réaction d'un composé **(b2)** comprenant un groupement disulfure et deux groupements groupement partant *Gp* nucléofuges, comme par exemple mésylate, tosylate, triflate ou halogénure avec un chromophore **(a2)** hémicyanine styryle porteur d'un groupement X-*Z*.

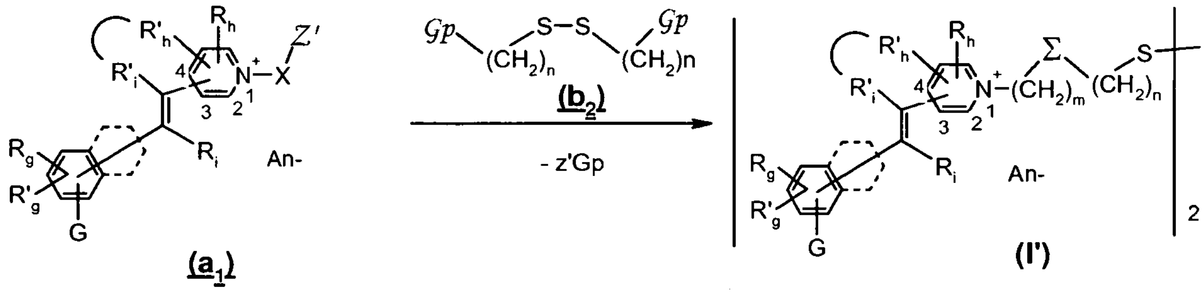


$(a_1)$ (I')

Avec Rg, R'g, $R_h$, $R'_h$, $R'_i$, $R''_i$, m, n, G, $An^-$, $\Sigma$, *Z'* représentant un atome d'hydrogène ou un métal alcalin, X-Z' représentant une chaîne hydrocarbonée porteuse d'un groupement nucléophile susceptible de substituer le groupement Gp, comme par exemple une fonction amino ou hydroxy, étant toujours entendu que $\Sigma$ représente un sous ensemble de Ta défini dans les formules **(I)** et **(II)**

Les réactifs de départs sont commerciaux ou accessibles par les méthodes classiques connues par l'homme du métier.

On pourra se référer à l'ouvrage Advanced Organic Chemistry, « Reactions, Mechanisms and Structures », J. March, 4ème Ed, John Willey & Sons, 1992 ou T. W. Greene « Protective Groups in Organic Synthesis », pour avoir plus de détails sur les conditions opératoires mises en oeuvre pour les procédés mentionnés ci-dessus.

Les colorants fluorescents thiols formés peuvent être transformés en colorants fluorescents thiols protégés -S Y' par la protection du thiol -SH en utilisant les groupes protecteurs classiques. Les colorants fluorescents thiols sont métallés en utilisant également les méthodes classiques connues par l'homme du métier telles que celles décrites dans Advanced Organic Chemistry, « Reactions, Mechanisms and Structures », J. March, 4ème Ed, John Willey & Sons, NY, 1992.

Les colorants thiols protégés peuvent êtres déprotégés par voies classiques telles que celles décrites dans les ouvrages « Protective Groups in Organic Synthesis », T. W. Greene, John Willey & Sons ed., NY, 1981 ; « Protecting Groups », P. Kocienski, Thieme, 3ème ed., 2005.

Un autre objet de l'invention concerne une composition tinctoriale pour colorer les matières kératiniques qui contient au moins un colorant fluorescent disulfure de formule (I) ou thiol de formule (II). Outre la présence d'au moins un colorant fluorescent de formule (I) ou (II), la composition de l'invention peut également contenir un agent réducteur.

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catéchol-borane.

La composition tinctoriale utile dans l'invention contient en général une quantité de colorant fluorescent de formule (I) ou (II) comprise entre 0,001 et 50% par rapport au poids total de la composition. De préférence, cette quantité est comprise entre 0,005 et 20% en poids et encore plus préférentiellement entre 0,01 et 5% en poids par rapport au poids total de la composition.

La composition tinctoriale peut en outre contenir des colorants directs additionnels. Ces colorants directs sont par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

La composition tinctoriale peut contenir une ou plusieurs base d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition.

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

Le ou les coupleurs sont chacun généralement présents en quantité comprise entre 0,001 et 10% en poids du poids total de la composition tinctoriale, de préférence entre 0,005 et 6%.

La ou les bases d'oxydation présentes dans la composition tinctoriale sont en général présentes chacune en quantité comprise entre 0,001 à 10% en poids du poids total de la composition tinctoriale, de préférence entre 0,005 et 6% en poids.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates et les sels d'addition avec une base telles que les hydroxydes de métal alcalin comme la soude, la potasse, l'ammoniaque, les amines ou les alcanolamines.

Le milieu approprié pour la teinture, appelé aussi support de teinture, est un milieu cosmétique généralement constitué par de l'eau ou par un mélange d'eau et d'au moins un solvant organique. A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 99% en poids environ par rapport au poids total de la composition tinctoriale, et encore plus préférentiellement entre 5 et 95% en poids environ.

Selon une variante, la composition de l'invention contient un agent réducteur capable de réduire les liaisons disulfures de la kératine et/ou disulfure des colorants fluorescent de formule (I). Cet agent réducteur est tel que défini précédemment.

La composition tinctoriale peut également renfermer divers adjuvants utilisés classiquement dans les compositions pour la teinture des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

Les adjuvants ci dessus sont en général présents en quantité comprise pour chacun d'eux entre 0,01 et 20% en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale est généralement compris entre 3 et 14 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (y) suivante :

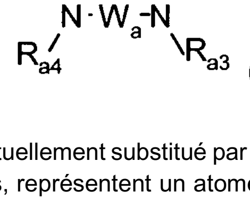
(γ)

dans laquelle $W_a$ est un reste propylène éventuellement substitué par un groupe hydroxy ou un radical alkyle en $C_1$-$C_4$ ; $R_{a1}$, $R_{a2}$, $R_{a3}$ et $R_{a4}$, identiques ou différents, représentent un atome d'hydrogène, un radical alkyle en $C_1$-$C_4$ ou hydroxyalkyle en $C_1$-$C_4$.

La composition tinctoriale peut se présenter sous des formes diverses, telles que sous forme de liquide, de crème, de gel, ou sous toute autre forme appropriée pour réaliser une teinture des fibres kératiniques, et notamment des cheveux.

Un autre objet de l'invention est un procédé de coloration de matière kératinique consistant à appliquer sur les dites matières une composition comprenant au moins un colorant de formule (I) ou (II). Selon un mode de réalisation particulier dans le procédé de l'invention un agent réducteur peut également être appliqué en pré-traitement avant l'application de la composition contenant au moins un colorant fluorescent de formule (I) ou (II).

Cet agent réducteur peut être choisi parmi les thiols par exemple la cystéine, l'homocystéine, l'acide thiolactique, les sels de ces thiols, les phosphines, le bisulfite, les sulfites, l'acide thioglycolique, ainsi que ses esters, notamment le monothioglycolate de glycérol, et le thioglycérol. Cet agent réducteur peut aussi être choisi parmi les borohydrures et leurs dérivés, comme par exemple les sels du borohydrure, du cyanoborohydrure, du triacétoxyborohydrure, du triméthoxyborohydrure : sels de sodium, lithium, potassium, calcium, ammoniums quaternaires (tétraméthylammonium, tétraéthylammonium, tétra n-butylammonium, benzyltriéthylammonium); le catécholborane.

Ce prétraitement peut être de courte durée, notamment de 0,1 seconde à 30 minutes, de préférence de 1 minute à 15 minutes avec un agent réducteur tel que cité précédemment.

Selon un autre procédé, la composition comprenant au moins un colorant fluorescent de formule (I) ou (II) et contient également au moins un agent réducteur tel que défini précédemment. Cette composition est alors appliquée aux cheveux.

Lorsque le colorant fluorescent thiol de formule (II) comprend un groupe Y protecteur de la fonction thiol, le procédé de l'invention peut être précédé d'une étape de déprotection visant à restituer *in-situ* la fonction SH.

A titre d'exemple il est possible de déprotéger la fonction S-Y avec Y groupe protecteur en ajustant le pH comme suit :

| Y : groupe protecteur | déprotection |
|---|---|
| | |
| alkylcarbonyle, | pH>9 |
| arylcarbonyle, | pH>9 |
| alkoxycarbonyle, | pH>9 |
| aryloxycarbonyle, | pH>9 |
| arylalkoxycarbonyle | pH>9 |
| (di)(alkyl)aminocarbonyle, | pH>9 |
| (alkyl)arylaminocarbonyle | pH>9 |
| aryle éventuellement substitué tel que le phényle, | pH>9 |
| hétéroaryle monocyclique à 5, 6 ou 7 chaînons tel que l'oxazolium ; | pH>9 |
| hétéroaryle bicyclique à 8 à 11 chaînons tels que benzoimidazolium, ou benzoxazolium | pH>9 |

L'étape de déprotection peut également être réalisée au cours d'une étape de prétraitement des cheveux comme par exemple, le prétraitement réducteur des cheveux.

Selon une variante, l'agent réducteur est ajouté à la composition tinctoriale contenant au moins un colorant fluorescent de formule (I) ou (II) au moment de l'emploi.

Selon un autre procédé, la composition comprenant au moins un colorant fluorescent de formule (I) ou (II) contient également au moins un agent réducteur tel que défini précédemment. Cette composition est alors appliquée aux cheveux.

Selon une autre variante, l'agent réducteur est appliqué en post-traitement, après l'application de la composition contenant au moins un colorant fluorescent de formule (I) ou (II). La durée du post traitement avec l'agent réducteur peut être courte, par exemple de 0,1 seconde à 30 minutes de préférence de 1 minute à 15 minutes, avec un agent réducteur tel que décrit précédemment. Selon un mode de réalisation particulier l'agent réducteur est un agent de type thiol ou borohydrure tel que décrit précédemment.

Un mode de réalisation particulier de l'invention concerne un procédé dans lequel le colorant fluorescent de formule (I) ou (II) peut être appliqué directement aux cheveux sans réducteurs, exempt de pré ou post-traitement réducteurs.

Un traitement avec un agent oxydant peut éventuellement être associé. Selon un mode de réalisation particulier de l'invention le procédé selon l'invention comprend une étape supplémentaire par application d'un agent oxydant sur les fibres kératiniques. On pourra utiliser n'importe quel type d'agent oxydant classique dans le domaine. Ainsi, il peut être choisi parmi le peroxyde d'hydrogène, le peroxyde d'urée, les bromates de métaux alcalins, les persels tels que

les perborates et les persulfates, ainsi que les enzymes parmi lesquelles on peut citer les peroxydases, les oxydo-réductases à 2 électrons tels que les uricases et les oxygénases à 4 électrons comme les laccases. L'utilisation du peroxyde d'hydrogène est particulièrement préférée.

Cet agent oxydant peut être appliqué sur les fibres avant ou après l'application de la composition contenant au moins un colorant fluorescent de formule (I) ou (II).

L'application de la composition tinctoriale selon l'invention est généralement effectuée à température ambiante. Elle peut cependant être réalisée à des températures variant de 20 à 180°C.

L'invention a aussi pour objet un dispositif à plusieurs compartiments ou "kit" de teinture dans lequel un premier compartiment renferme une composition tinctoriale comprenant au moins un colorant fluorescent de formule (I) ou (II) et un deuxième compartiment renferme un agent réducteur capable de réduire les fonctions disulfures des matières kératiniques et/ou du colorant fluorescent disulfure de formule (I).

L'un de ces compartiments peut en outre contenir un ou plusieurs autres colorants de type colorant direct ou colorant d'oxydation.

Elle concerne aussi un dispositif à plusieurs compartiments dans lequel un premier compartiment contient une composition tinctoriale comprenant au moins un colorant fluorescent de formule (I) ou (II) ; un deuxième compartiment contient un agent réducteur capable de réduire la liaison disulfure des matières kératiniques et/ou du colorant fluorescent disulfure de formule (I) ; un troisième compartiment contient un agent oxydant.

Alternativement, le dispositif de teinture contient un premier compartiment renfermant une composition tinctoriale qui comprend au moins un colorant fluorescent thiol protégé de formule (II) et un deuxième compartiment renfermant un agent capable de déprotéger le thiol protégé pour libérer le thiol.

Chacun des dispositifs mentionnés ci-dessus peut être équipé d'un moyen permettant de délivrer sur les cheveux le mélange souhaité, par exemple tel que les dispositifs décrits dans le brevet FR2 586 913.

Les exemples qui suivent servent à illustrer l'invention sans toutefois présenter un caractère limitatif. Les colorants fluorescents thiols des exemples ci-après ont été entièrement caractérisés par les méthodes spectroscopiques et spectrométriques classiques.

## EXEMPLES DE SYNTHESE

**<u>Exemple 1 :</u> Synthèse du di méthane sulfonate de 2,2'-(disulfanediyldiéthane-2,1-diyl)-bis{5-[4-(diméthylamino) benzylidène]-5,6,7,8-tetrahydroisoquinolinium} [1]**

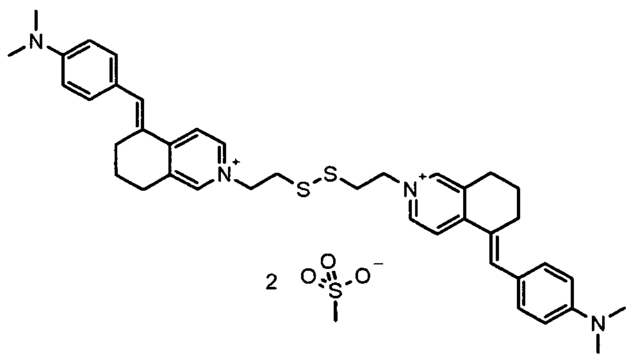

**[1]**

**Schéma de synthèse**

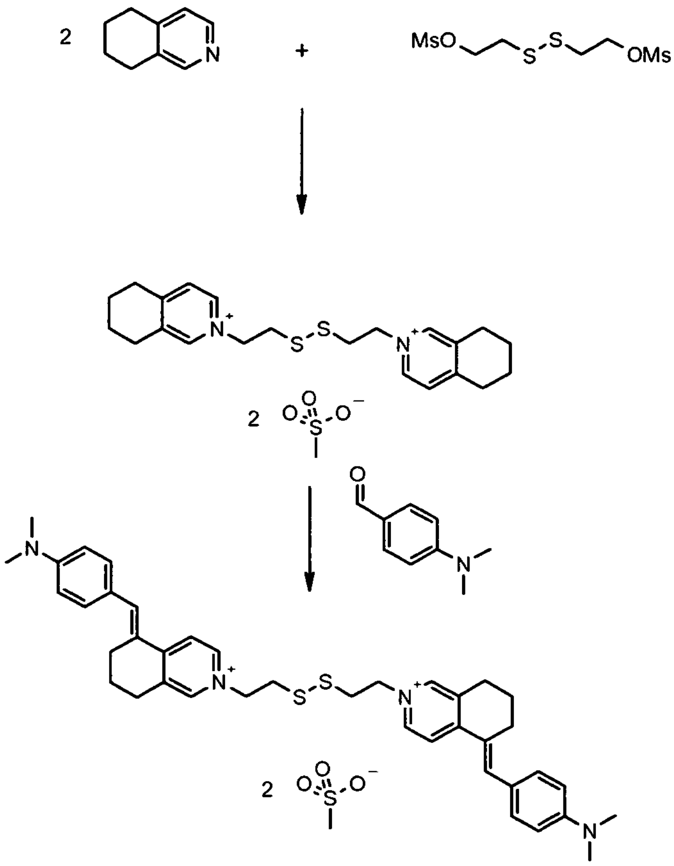

**[1]**

**Etape 1 : synthèse du di méthane sulfonate de 2,2'-(disulfanediyldiethane-2,1-diyl)bis(5,6,7,8-tetrahydroisoquinolinium)**

3.2 g de 5,6,7,8-tetrahydroisoquinoline et 0.7 g de carbonate de potassium sont mélangés dans 3 mL d'acétonitrile et portés à 80°C. Une solution de 3.4 g de dimethanesulfonate de disulfanediyldiéthane-2,1-diyle dans 3 mL d'acétonitrile est ajoutée en 10 min. L'agitation est maintenue pendant 8 h à 80°C puis le milieu réactionnel est ramené à température ambiante, filtré et concentré sous vide. 6 mL de dichlorométhane sont ajoutés et la solution est versée goutte à goutte sur 500 mL d'éther éthylique. L'huile décantée est retraitée deux fois par dilution dans 6 mL de méthanol et addition dans 500 mL d'éther éthylique, une dernière fois par dilution dans 6 mL de dichlorométhane et addition dans 500 mL d'éther éthylique. Après séchage sous vide, 5.2g de solide marron sont recueillis. Les analyses sont conformes à la structure attendue.

**Etape 2 : di méthane sulfonate de 2,2'-(disulfanediyldiéthane-2,1-diyl)bis{5-[4-(diméthylamino)benzylidène]-5,6,7,8-tetrahydroisoquinolinium} [1]**

2.1 g de di méthane sulfonate de 2,2'-(disulfanediyldiethane-2,1-diyl)bis(5,6,7,8-tetrahydroisoquinolinium) et 1.1 ge de diméthylaminobenzaldéhyde sont mélangés dans 6 mL de méthanol. 130 μL de pipéridine sont ajoutés et le mélange est agité pendant 8 h à 21°C. 65 μL de pipéridine sont ajoutés et l'agitation maintenue 48h. 130μL de pipéridine sont encore ajoutés et le mélange maintenu agité pendant 24h. Le mélange obtenu est versé goutte à goutte sur 500 mL de méthyltertiobutyl éther sous vive agitation. Une huile visqueuse décante. Elle est séparée du surnageant, diluée dans 20 mL de dichlorométhane et versée goutte à goutte sur 500 mL de méthyltertiobutyléther. Le précipité rouge orangé formé est filtré, rincé par 50 mL de méthyltertiobutyléther, repris dans 20 mL de dichlorométhane et reversé sur 500 mL de méthyltertiobutyl éther, filtré, rincé et séché. 2.2 g de solide rouge sont obtenus. Les analyses montrent qu'il est conforme.

Exemple 2 : Synthèse du di méthane sulfonate 2,2'-(disulfanediyldibutane-4,1-diyl)bis{5-[(4-méthyl-3,4-dihydro-2H-1,4-benzoxazin-7-yl)méthylène]-5,6,7,8-tetrahydroisoquinolinium} [2]

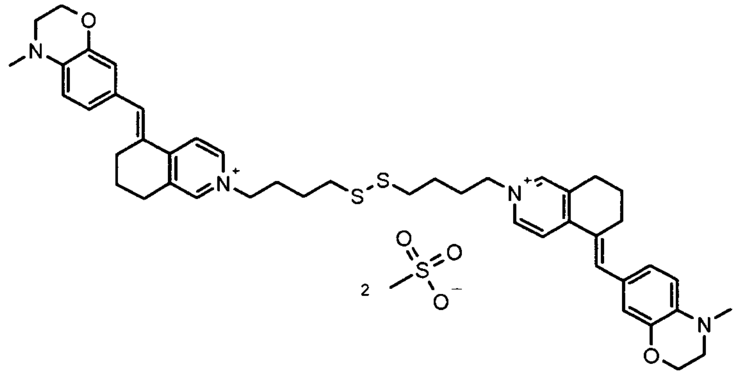

[2]

**Schéma de synthèse**

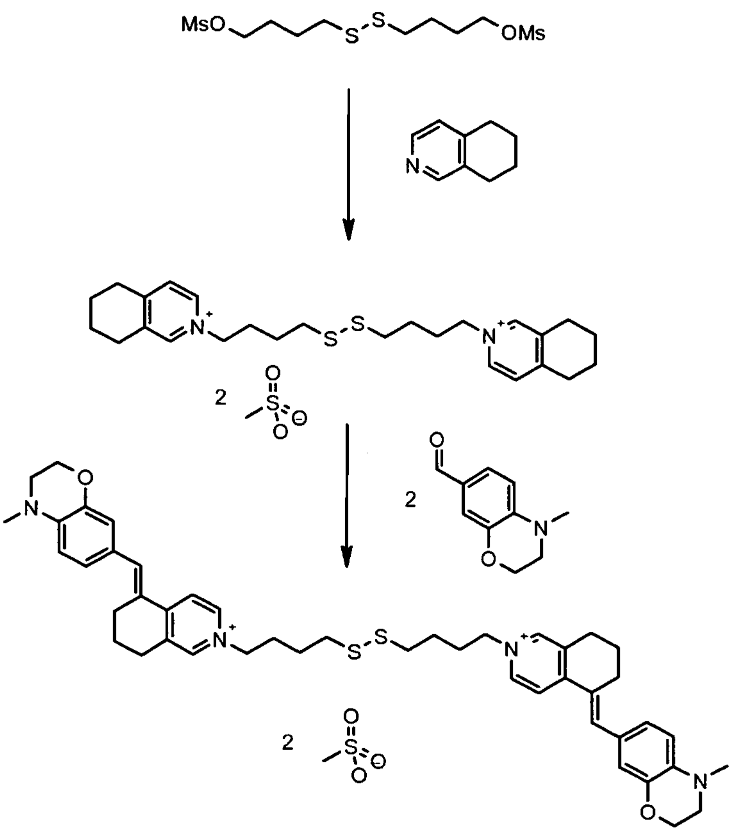

[2]

**Etape 1 : synthèse du di méthane sulfonate de 2,2'-(disulfanediyldibutane-4,1-diyl)bis(5,6,7,8-tetrahydroisoquinolinium)**

2.1 g de 5,6,7,8-tetrahydroisoquinoline et 0.5 g de carbonate de potassium sont mélangés dans 2 mL d'acétonitrile et portés à 100°C. Une solution de 2.7 g de diméthanesulfonate de disulfanediyldibutane-4,1-diyle dans 2 mL d'acétonitrile est ajoutée en 5 min. L'agitation est maintenue pendant 3 h à 100°C puis le milieu réactionnel est ramené à température ambiante et concentré sous vide. 10 mL de dichlorométhane sont ajoutés et la solution est filtrée puis versée goutte à goutte sur 100 mL d'éther éthylique. L'huile décantée est retraitée par dilution dans 10 mL de dichlorométhane et addition dans 500 mL d'éther éthylique, Après séchage sous vide, 3.3 g d(huile marron sont recueillis. Les analyses sont conformes à la structure attendue.

**Etape 2 : di méthane sulfonate de 2,2'-(disulfanediyldibutane-4,1-diyl)bis{5-[(4-methyl-3,4-dihydro-2H-1,4-benzoxazin-7-yl)methylene]-5,6,7,8-tetrahydroisoquinolinium} [2]**

2.8 g de di méthane sulfonate de 2,2'-(disulfanediyldibutane-4,1-diyl)bis(5,6,7,8-tetrahydroisoquinolinium) et 2 g de 4-méthyl-3,4-dihydro-2H-1,4-benzoxazine-7-carbaldéhyde sont mélangés dans 28 mL d'isopropanol. 0.7g de pipéridine sont ajoutés et le mélange est agité pendant 10 h à 80°C. le mélange est refroidi et concentré sous vide, repris dans 20 mL de dichlorométhane et versé goutte à goutte sur 200 mL d'éther éthylique. Un solide et une huile se forment. Ils sont séparés (filtration du surnageant avec le solide), l'huile est reprise par 20 mL de dichlorométhane et le mélange est versé sur 200 mL d'éther éthylique. Une nouvelle fraction solide est recueillie. Les fractions solides obtenues sont reprises chacune dans 20 mL de dichlorométhane et concentrées sous vide poussé. 1.7 g de solide rouge est recueilli. Les analyses montrent que le produit est conforme à la structure attendue.

**<u>Exemple 3</u> : synthèse du bis mésylate de 2,2'-(disulfanediyldiéthane-2,1-diyl)bis[5-({4-[bis(2-hydroxyéthyl)amino]-phényl}méthylidène)-5,6,7,8-tétrahydroisoquinolinium] [3]**

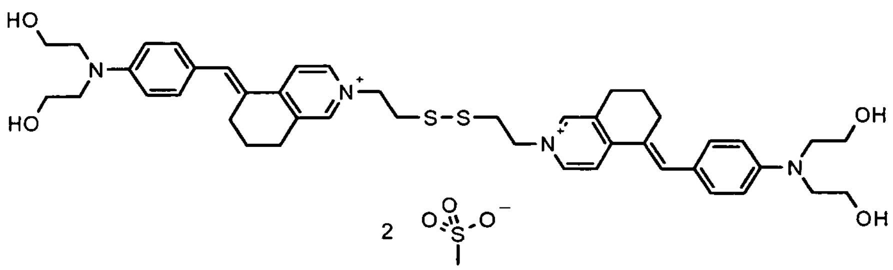

**[3]**

**Schéma de synthèse**

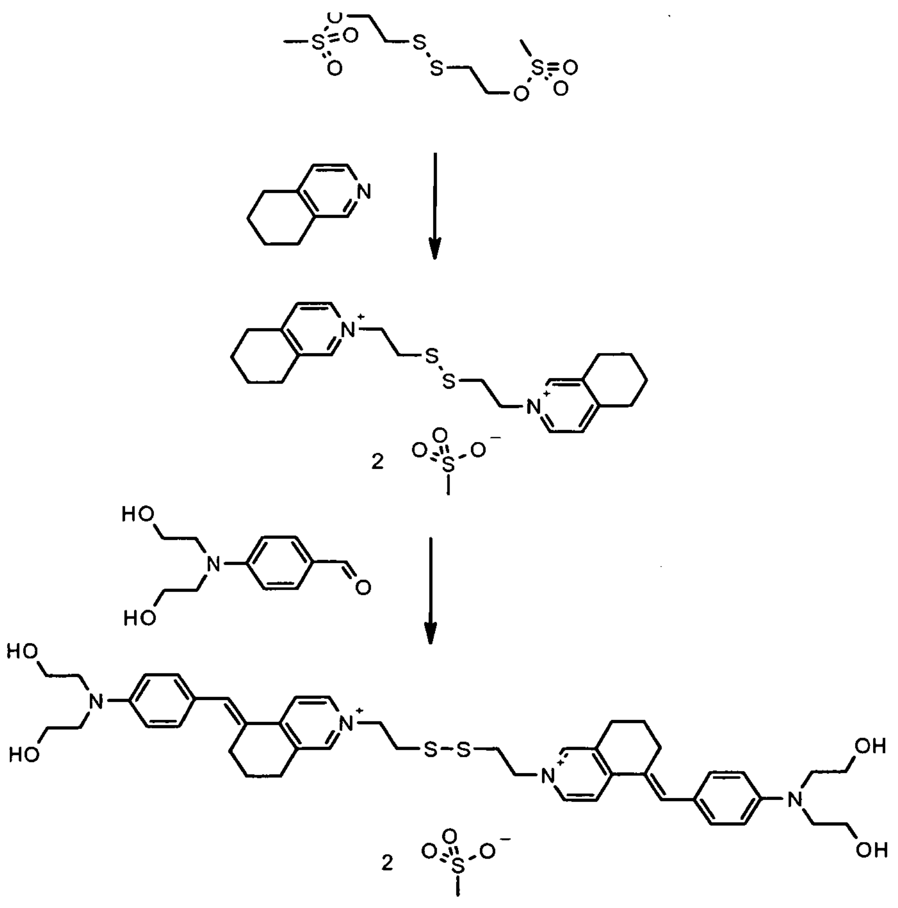

**Etape 1 : synthèse du bis mésylate de 2,2'-(disulfanediyldiethane-2,1-diyl)di-5,6,7,8-tetrahydroisoquinolinium**

50.12 g de sulfonate de disulfanediyldiéthane-2,1-diyl diméthane sont solubilisés dans 90 mL de *N*-méthylpyrrolidinone (NMP). Le mélange est chauffé à 90°C et 46.49 g de tétraisoquinoline sont ajoutés, suivi par 50 mL en plus de NMP et 10 mL d'acétonitrile. Après 4 h à 90 °Cle mélange réactionnel est versé dans 1.7 L d'acétate d'éthyle (AcOEt) et refroidi à -10 °C. Le surnageant est retiré, et la phase organique est lavée avec 100 m d'isopropanol iPrOH et 300 m de diéthyléther, puis pluisiuers fois à l'acétate d'éthyle jusqu'à ce que le tétrahydroisoquinoline ne soit plus détecté par chromatographie sur couche mince (CCM). Après séchage, 77.5 g d'huile sont collectées. Les analyse sont en accord avec la structure attendu dans l'étape 1.

**Etape 2 : synthèse de bis mésylate 2,2'-(disulfanediyldiéthane-2,1-diyl)bis[5-({4-[bis(2-hydroxyethyl)amino] phényl}-méthylidène)-5,6,7,8-tétrahydroisoquinolinium]**

5.12 mL de piperidine sont ajoutés à une suspension de 45.6 g de 4-[bis-(2-hydroxy-éthyl)-amino]-benzaldéhyde et 50g de bis mésylate de 2,2'-(disulfanediyldiethane-2,1-diyl)di-5,6,7,8-tétrahydroisoquinolinium dans 200 m de méthanol. Après 28 h d'agitation à température ambiante, le mélange réactionnel est versé dans 1 L d'acétate d'éthyle. L'huile résultante colante est lavée plusieurs fois à l'acétone puis séchée. Les analyses sont en accord avec la structure du composé **[3]**.

LC/MS gradient $ACONH_4$ 20mM->$CH_3CN$ 10min ESI+/- m/z=385 ; $\lambda_{max}$ = 468 nm.

NMR $^1H$ (ppm) : 1.91 (mH, , 4H), 2.69 (s, 6.7H - mésylate), 2.92 (t, 4H), 2.98 (t, 4H), 3.36 (t, 4H), 3.63 (t, 8H), 3.76 (t, 8H), 4.74 (t, 4H), 6.84 (d, 4H), 7.51 (d, 4H), 7.65 (s, 2H), 8.25 (d, 2H), 8.44 (d, 2H), 8.51 (d, 2H).

## EXEMPLE DE COLORATION

**<u>Exemple 1 :</u> Procédé de coloration - composés [1] et [2]**

Préparation d'une composition A

| | |
|---|---|
| Composé [1] ou [2] | $5x10^{-4}$ mol% |
| Alcool Benzylique | 4 g |
| Polyéthylèneglycol 60E | 6 g |
| Hydroxyethylcellulose | 0,7 g |
| Alkylpolyglucoside en solution aqueuse à 65% MA | 4.5 g |
| Eau déminéralisée | qsp 100g |

Préparation d'une composition B

| | |
|---|---|
| Acide thioglycolique | 1 M |
| Hydroxyde de Sodium | qsp pH 8,5 |
| Eau déminéralisée | qsp 100g |

Au moment de l'emploi, on mélange les compositions A (9 mL) et B (1 mL), puis on applique les formules sur mèches de cheveux blancs naturels à 90 % blancs (BN), blancs permanentés (BP) ou châtains de hauteur de ton 4 (HT4). Le temps de pose est 20 minutes à température ambiante TA.

Après rinçage à l'eau courante on applique un fixateur (Dulcia Vital II®) dilué par 10 avec de l'eau pendant 5 minutes à TA. Après rinçage à l'eau courante et shampooing les mèches sont séchés à l'air on observe un éclaircissement des cheveux foncés ainsi traités : la mèche HT4 est devenue visuellement plus claire que des mèches témoins non traitées. Les mèches de cheveux blancs sont colorées avec des nuances intenses.

*Observations visuelles*

Lors du rinçage et des shampooings des colorations obtenues avec les composés [1] et [2] il n'y a pas de dégorgement visible, la mousse des shampooings et les eaux de rinçage ne sont pratiquement pas colorées.
La couleur observée est conservée sur les BN et BP colorés et l'effet d'éclaircissement reste visible sur les cheveux HT4 shampooinés.

*Résultats de Réflectance pour évaluer l'éclaircissement:*

Les performances d'éclaircissement des compositions conformes à l'invention ont été exprimées en fonction de la réflectance des cheveux. Ces réflectances sont comparées à la réflectance d'une mèche de cheveux non traitées de hauteur de ton HT4.

La réflectance est mesurée au moyen d'un appareil spectrophotocolorimètre KONIKA-MINOLTA ®, CM 3600d et après irradiation des cheveux avec une lumière visible dans la gamme de longueurs d'onde allant de 400 à 700 nanomètres.

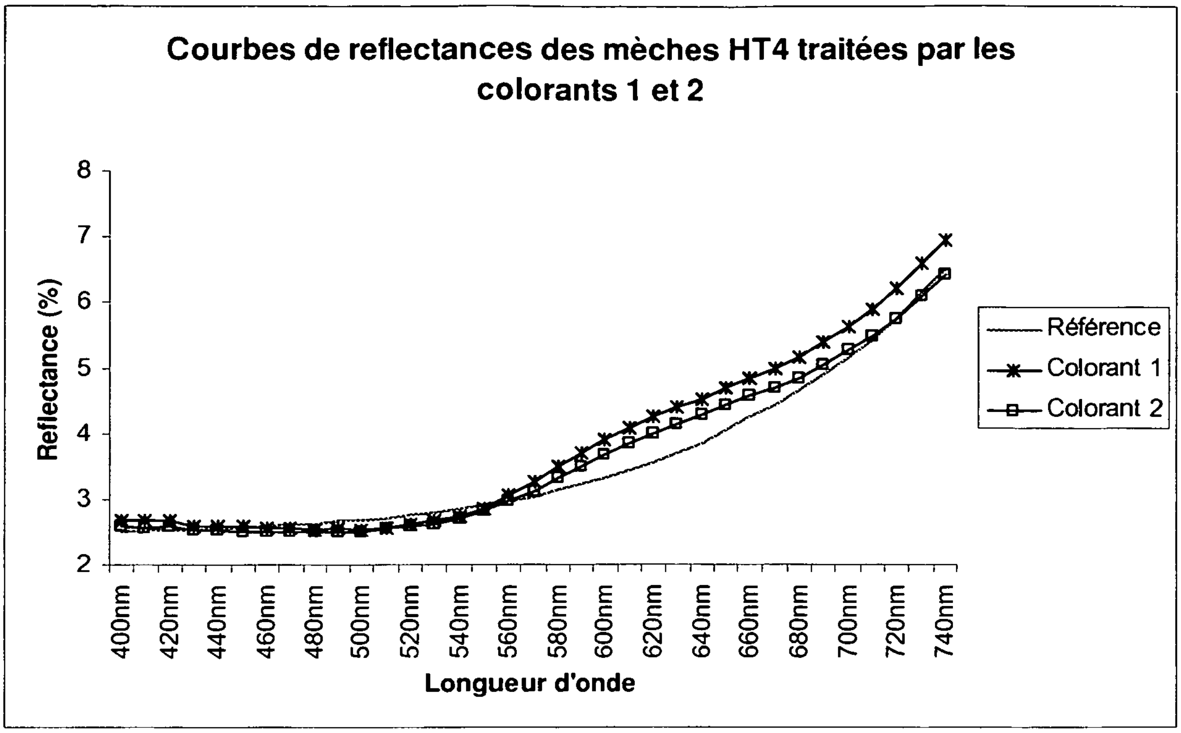


On constate tout d'abord que la réflectance d'une mèche de cheveux traitée avec une composition selon l'invention est supérieure à celle des cheveux non traités. Tout particulièrement la reflectance des mèches traitées avec les colorants [1] et [2] est nettement supérieure à celle de la mèche de référence dans la plage de longueur d'ondes supérieure à 560nm. Les mèches traitées par ces deux composés apparaissent donc plus claires.

*Résultats dans le système L*a*b* pour évaluer la coloration des HT4, BN, BP :*

La couleur des mèches été évaluée dans le système L*a*b* au moyen d'un spectrocolorimètre CM 3600D MINOLTA ®, (Illuminant D65).
Dans ce système L* a* b*, L* représente la luminosité, a* indique l'axe de couleur vert/rouge et b* l'axe de couleur bleu/jaune. Plus la valeur de L est élevée, plus la couleur est claire ou peu intense. Inversement, plus la valeur de L est faible, plus la couleur est foncée ou très intense. Plus la valeur de a* est élevée plus la nuance est rouge et plus la valeur de b* est élevée plus la nuance est jaune.

La variation de la coloration entre les mèches de cheveux HT4, colorées et lavées est mesurée par (ΔE) selon l'équation suivante :

$$\Delta E = \sqrt{(L^* - L_o^*)^2 + (a^* - a_o^*)^2 + (b^* - b_o^*)^2}$$

Dans cette équation, L*, a* et b* représentent les valeurs mesurées avant coloration et $L_0$*, $a_0$* et $b_0$* représentent les valeurs mesurées avant coloration (ou shampooing).

Plus la valeur de ΔE est importante, plus la différence de couleur entre les mèches HT4 et les mèches colorées est importante.

<u>Sur les cheveux foncés HT4 :.</u>

| Composés | L*(D65) | a*(D65) | b*(D65) | Δ E |
|---|---|---|---|---|
| Reférence HT4 | 20 | 2,69 | 3,02 | ------ |
| Composé 1 | 20,53 | 5,72 | 3,84 | 3,19 |
| Composé 2 | 20,12 | 4,92 | 3,7 | 2,34 |

Les valeurs rapportées dans le tableau ci-dessus montrent l'effet de coloration avec éclaircissement obtenu sur les cheveux foncés (HT4) par l'emploi des composés 1 et 2.

<u>Sur les cheveux BN et BP :.</u>

Les cheveux blancs naturels et blancs permanentés sont colorées orange vifs.

| | L*(D65) | a*(D65) | b*(D65) |
|---|---|---|---|
| Blancs naturels | 60,5 | 1,5 | 16,1 |
| Composé 1 | 39,1 | 37,9 | 33,3 |
| Composé 2 | 35,5 | 36,9 | 29,7 |
| Blancs permanentés | 60,9 | 1,8 | 17,0 |
| Composé 1 | 38,9 | 39,8 | 33,5 |
| Composé 2 | 36,0 | 38,5 | 30,5 |

*<u>Rémanence vis-à-vis des shampooings successifs :</u>*

Les mèches traitées sont divisées en deux, une moitié est soumise à 5 shampooings successifs selon un cycle qui comprend le mouillage des mèches à l'eau, le lavage avec un shampooing classique, un rinçage à l'eau suivi d'un séchage.

*Observations visuelles*

Lors des shampooings, il n'y a pas de dégorgement visible, la mousse des shampooings et les eaux de rinçage ne sont pas colorées.

La couleur observée et l'effet d'éclaircissement restent visibles sur les cheveux de hauteur de ton 4 ainsi traités.

*Rémanence vis-à-vis la lumière :*

Une étude de ténacité lumière a été réalisée par exposition au Xénotest sur les mèches de cheveux blancs naturels et blancs permanentés durant 3 heures. Les conditions d'exposition sont 90W/m2, 60% relative humidité et avec une température de la chambre de 35°C.

Apres 3 heures d'exposition à la lumière les cheveux blancs naturels BN et blancs permanentés BP colorées avec les colorants 1 et 2 de l'invention sont quasiment inchangés.

## Revendications

1. Colorant fluorescent de formule **(I)** ou **(II) :**

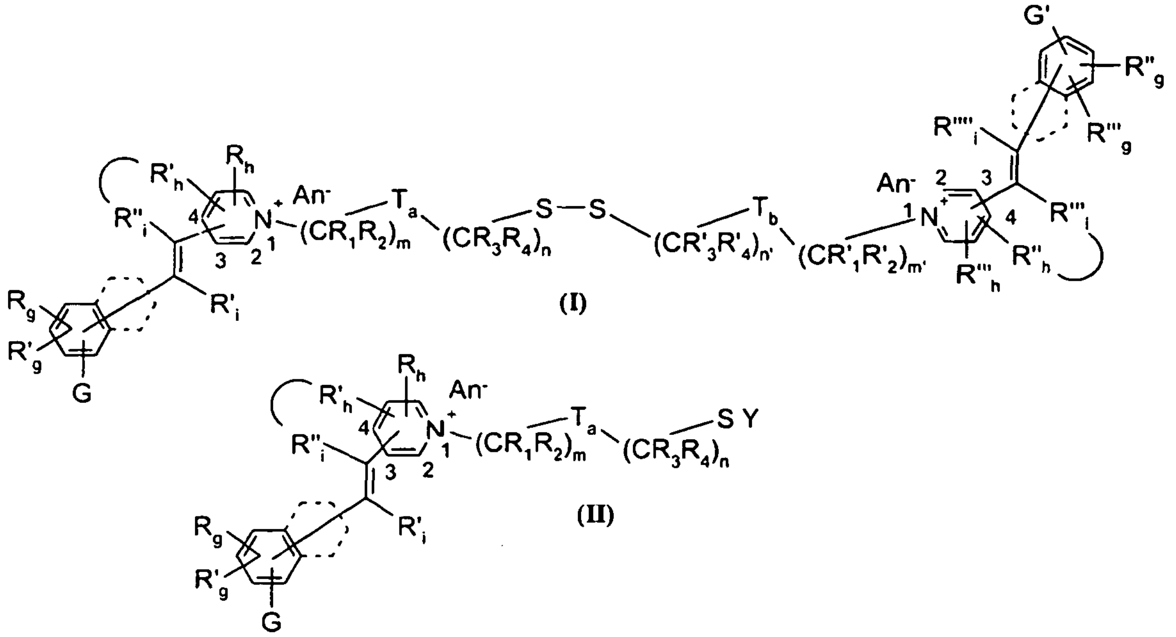

(I)

(II)

ses sels d'acide organique ou minéral, isomères optiques, isomères géométriques, et les solvates tels que hydrates ; formules **(I)** et **(II)** dans lesquelles :

➢

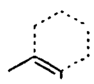

représente un groupe aryle, hétérocycle ou hétéroaryle fusionné au cycle phényle ; ou alors est absent du cycle phényle ;
➢ G et G', identiques ou différents, représentent un groupe $-NR_cR_d$ ou $(C_1-C_6)$alcoxy éventuellement substitué ;
➢ $R_c$ et $R_d$, identiques ou différents, représentent un atome d'hydrogène, un groupe $(C_1-C_6)$alkyle éventuellement substitué, aryl$(C_1-C_4)$alkyle, $(C_1-C_6)$alcoxy$(C_1-C_6)$alkyle, un groupe $(C_1-C_6)$alkyle éventuellement substitué ;
➢ou alors les deux radicaux $R_c$, et $R_d$ portés par le même atome d'azote forment ensemble un groupe hétérocyclique ou hétéroaryle ;
➢ $R_g$, $R'_g$, $R''_g$, $R'''_g$, $R_h$, et $R'''_h$, identiques ou différents, représentent un atome d'hydrogène, d'halogène, un groupe amino, (di)$(C_1-C_4)$alkylamino, cyano, carboxy, hydroxy, trifluorométhyle, acylamino, alcoxy en $C_1-C_4$, (poly)hydroxyalcoxy en $C_2-C_4$, $(C_1-C_4)$alkylcarbonyloxy $(C_1-C_4)$alcoxycarbonyle, $(C_1-C_4)$alkylcarbonyl-amino, acylamino, carbamoyle , $(C_1-C_4)$alkylsulfonylamino, un radical amino-sulfonyle, ou un radical $(C_1-C_{16})$alkyle éventuellement substitué par un groupe choisi parmi $(C_1-C_{12})$alcoxy, hydroxy, cyano, carboxy, amino, (di)$(C_1-C_4)$alkylamino, ou alors les deux radicaux alkyles portés par l'atome d'azote du groupe amino forment un hétérocycle comprenant de 5 à 7 chaînons et comprenant éventuellement un autre hétéroatome identique ou différent à celui de l'atome d'azote ; particulièrement $R_g$, $R'_g$, $R''_g$. $R''_g$, $R_h$, et $R'''_h$ représentent un atome d'hydrogène ;
ou alors deux groupes $R_g$ et $R'_g$, $R''_g$ et $R'''_g$, portés par deux atomes de carbone adjacents, forment ensembles un cycle benzo, indéno, un groupe hétérocycloalkyle fusionné ou hétéroaryle fusionné ; le cycle benzo, indéno, hétérocycloalkyle ou hétéroaryle étant éventuellement substitué par un atome d'halogène, un groupe $(C_1-C_4)$ alkyle, amino, $(C_1-C_4)$ alkylamino, $(C_1-C_4)$ dialkylamino, cyano, carboxy, hydroxy, trifluorométhyle, un radical acylamino, alcoxy en $C_1-C_4$, (poly)hydroxyalcoxy en $C_2-C_4$, alkylcarbonyloxy alcoxycarbonyle, alkylcarbonylamino, un radical acylamino, carbamoyle , alkylsulfonylamino, un radical amino-sulfonyle, ou un radical $(C_1-C_{16})$ alkyle éventuellement substitué par : un groupe choisi parmi $(C_1-C_{12})$ alcoxy, hydroxy, cyano, carboxy, amino, $(C_1-C_4)$ alkylamino, $(C_1-C_4)$ dialkylamino, ou alors les deux radicaux alkyles portés par l'atome d'azote du groupe amino forment un hétérocycle comprenant de 5 à 7 chaînons et comprenant éventuellement un autre

hétéroatome identique ou différent à celui de l'atome d'azote ;
➢ ou alors lorsque G et/ou G' représentent $-NR_cR_d$ deux groupes $R_c$ et $R'_g$, $R_d$ et $R_g$ et/ou $R_c$ et $R''_g$, $R_d$ et $R'''_g$, forment ensemble un hétéroaryle ou hétérocycle saturé, éventuellement substitué par un ou plusieurs groupes $(C_1-C_6)$alkyle ;
➢ $R_i'$ et $R''''_i$, identiques ou différents, représentent un atome d'hydrogène, ou un groupe $(C_1-C_4)$ alkyle ;
➢ $R'_h$ avec $R''_i$ et $R''_h$ avec $R'''_i$ forment ensemble avec les atomes de carbone qui les portent un groupe cycloalkyle en $C_5-C_7$ éventuellement substitué, fusionné au groupe pyridinium, étant entendu que le radical $R'_h$ ou $R''_h$ et le groupe styryle portant le radical $R''_i$ ou $R'''_i$ sont positionnés sur des atomes de carbone adjacents des groupes pyridiniums ;
➢ $R_1$, $R_2$, $R_3$. $R_4$, $R'_1$, $R'_2$, $R'_3$ et $R'_4$, identiques ou différents, représentent :

- un atome d'hydrogène,
- un groupe $(C_1-C_4)$alkyle,
- $(C_1-C_{12})$alcoxy,
- hydroxy,
- cyano,
- $-C(O)O^-M^+$ avec $M^+$ représentant un métal alcalin ou $M^+$ et $An^-$ sont absents,
- carboxy,
- (di)$(C_1-C_4)$(alkyl)amino, lesdits radicaux alkyle pouvant former avec l'atome d'azote qui les portent, un hétérocycle comprenant de 5 à 7 chaînons, comprenant éventuellement un autre hétéroatome différent ou non de l'azote ;

➢ $T_a$, $T_b$, identiques ou différentes représentent :

i) soit une liaison covalente σ,
ii) soit un ou plusieurs radicaux ou leurs combinaisons choisis parmi $-SO_2-$, -O-, -S-, -N(R)-, $-N^+(R)(R°)-$, -C(O)-, avec R et R°, identiques ou différents, représentant un atome d'hydrogène, un radical alkyle en $C_1-C_4$, hydroxyalkyle en $C_1-C_4$ ou un aryl$(C_1-C_4)$alkyle ;
iii) soit un radical hétérocycloalkyle ou hétéroaryle, cationique ou non, préférentiellement monocycliques ;

➢ m, m', n et n', identiques ou différents, représentent un entier compris inclusivement entre 0 et 6 avec m+n, m'+n', identiques ou différents, représentent un entier compris inclusivement entre 1 et 10 ;
➢ $An^-$ représentant un contre-ion anionique ; et
➢ Y représente : i) un atome d'hydrogène ; ii) un métal alcalin ; iii) un métal alcalino-terreux ; iv) un groupe ammonium : $N^+R^\alpha R^\beta R^\gamma R^\delta$ ou un groupe phosphonium : $P^+R^\alpha R^\beta R^\gamma R^\delta$ avec $R^\alpha$, $R^\beta$, $R^\gamma$ et $R^\delta$, identiques ou différents, représentant un atome d'hydrogène ou un groupe $(C_1-C_4)$alkyle ; ou v) un groupe protecteur de fonction thiol

choisi parmi les radicaux suivants :

■ $(C_1-C_4)$alkylcarbonyle ;
■ $(C_1-C_4)$alkylthiocarbonyle ;
■ $(C_1-C_4)$alcoxycarbonyle ;
■ $(C_1-C_4)$alcoxythiocarbonyle ;
■ $(C_1-C_4)$alkylthio-thiocarbonyle ;
■ (di)$(C_1-C_4)$(alkyl)aminocarbonyle ;
■ (di)$(C_1-C_4)$(alkyl)aminothiocarbonyle;
■ arylcarbonyle ;
■ aryloxycarbonyle ;
■ aryl$(C_1-C_4)$alkcoxycarbonyle ;
■ (di)$(C_1-C_4)$(alkyl)aminocarbonyle ;
■ $(C_1-C_4)$(alkyl)arylaminocarbonyle ;
■ carboxy ;
■ $SO_3^-$; $M^+$ avec $M^+$ représentant un métal alcalin ou alors $An^-$ de la formule (II) et $M^+$ sont absents ;
■ aryle éventuellement substitué ;
■ hétéroaryle éventuellement substitué ;
■ hétérocycloalkyle éventuellement substitué, éventuellement cationique,
■ le groupe suivant :

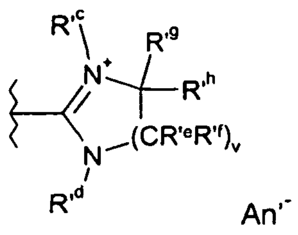

dans lequel R'$^c$, R'$^d$, R'$^e$, R'$^f$, R'$^g$ et R'$^h$, identiques ou différents représentent un atome d'hydrogène ou un groupe ($C_1$-$C_4$) alkyle, ou alors deux groupe R'$^g$ avec R'$^h$, et/ou R'$^e$ avec R'$^f$ forment un groupe oxo ou thioxo, ou alors R'$^g$ avec R'$^e$ forment ensemble un cycloalkyle ; et v représente un entier compris inclusivement entre 1 et 3 ; préférentiellement R'$^c$ à R'$^h$ représentent un atome d'hydrogène ; et An'$^-$ représente un contre-ion anionique;

- ■ isothiouronium ;
- ■ -C(NR'$^c$R'$^d$)=N$^+$R'$^e$R'$^f$; An'$^-$ avec R'$^c$, R'$^d$, R'$^e$ et R'$^f$, identiques ou différents représentent un atome d'hydrogène ou un groupe ($C_1$-$C_4$)alkyle et An$^-$ est tel que défini précédemment ;
- ■ isothiourée ;
- ■ -C(NR'$^e$R'$^d$)=NR'$^e$; An'$^-$ avec R'$^c$, R'$^d$, R'$^e$ et An$^-$ sont tels que définis précédemment ;
- ■ (di)aryl($C_1$-$C_4$)alkyle éventuellement substitué ;
- ■ (di)hétéroaryl($C_1$-$C_4$)akyle éventuellement substitué;
- ■ -CR$^1$R$^2$R$^3$ avec R$^1$, R$^2$ et R$^3$ identiques ou différents, représentant un atome d'halogène ou un groupe choisi parmi :

  i) ($C_1$-$C_4$)alkyle ;
  ii) ($C_1$-$C_4$)alcoxy ;
  iii) aryle éventuellement substitué ;
  iv) hétéroaryle éventuellement substitué ;
  v) P(Z$^1$)R'$^1$R'$^2$R'$^3$ avec R'$^1$, et R'$^2$ identiques ou différents représentent un groupe hydroxy, ($C_1$-$C_4$) alcoxy ou alkyle, R'$^3$ représente un groupe hydroxy ou ($C_1$-$C_4$)alcoxy, et Z$^1$ représente un atome d'oxygène ou de soufre ;

- ■ cyclique stériquement encombré ; et
- ■ alcoxyalkyle éventuellement substitué;

étant entendu que lorsque le composé de formule (I) ou (II) contient d'autres parties cationiques, il se trouve associé à un ou plusieurs contre-ions anioniques permettant d'atteindre l'électroneutralité de la formule **(I)** ou **(II).**

**2.** Colorant fluorescent de formule **(II)** selon la revendication précédente dans lequel Y représente un atome d'hydrogène, ou un métal alcalin.

**3.** Colorant fluorescent de formule **(II)** selon la revendication 1 dans lequel Y représente un groupe protecteur.

**4.** Colorant fluorescent de formule **(II)** selon l'une quelconque des revendications précédentes dans lequel Y représente un métal alcalin ou un groupe protecteur choisi parmi:

- ➢ ($C_1$-$C_4$)alkylcarbonyle ;
- ➢ arylcarbonyle ;
- ➢ ($C_1$-$C_4$)alkoxycarbonyle ;
- ➢ aryloxycarbonyle ;
- ➢ aryl($C_1$-$C_4$)alkoxycarbonyle ;
- ➢ (di) ($C_1$-$C_4$) (alkyl)aminocarbonyle ;
- ➢ ($C_1$-$C_4$)(alkyl)arylaminocarbonyle ;
- ➢ aryle éventuellement substitué ;
- ➢ hétéroaryle monocyclique cationique à 5 ou 6 chaînons éventuellement substitué par un ou plusieurs, identiques ou différents, groupes ($C_1$-$C_4$)alkyle ;
- ➢ hétéroaryle bicyclique cationique à 8 à 11 chaînons éventuellement substitué par un ou plusieurs, identiques

ou différents, groupes $(C_1-C_4)$alkyle ;

➢ hétérocycle cationique de formule suivante :

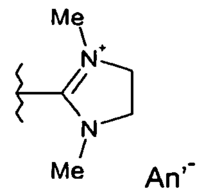

➢ isothiouronium ;

➢ $-C(NH_2)=N^+H_2$; An'⁻;

➢ isothiourée ;

➢ $-C(NH_2)=NH$ ; et

➢ $SO_3^-$ ; $M^+$ avec $M^+$ représentant un métal alcalin ou alors $An^-$ de la formule **(II)** et $M^+$ sont absents.

5. Colorant fluorescent disulfure selon la revendication 1 de formule **(I)** présentant un axe de symétrie C2 entre les deux atomes de soufre du radical central disulfure.

6. Colorant fluorescent selon l'une quelconque des revendications précédentes appartenant une des deux formules **(Ia)** ou **(IIa)** :

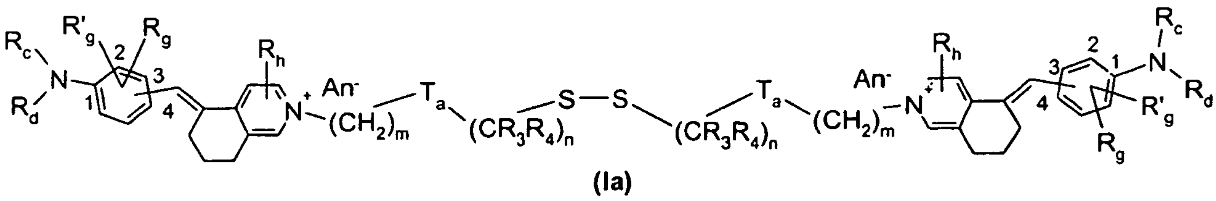

**(Ia)**

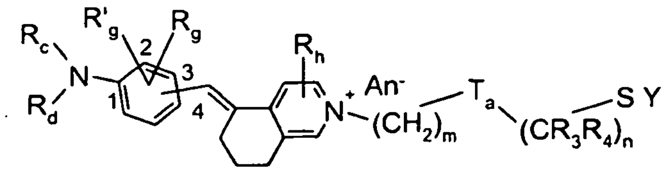

**(IIa)**

avec pour les formules **(Ia)**, **(IIa)** :

➢ $R_3$ et $R_4$, identiques ou différents, représentent :

- un atome d'hydrogène,
- un groupe $(C_1-C_4)$alkyle,
- $-C(O)O^-M^+$ avec $M^+$ représentant un métal alcalin ou $M^+$ et $An^-$ sont absents,
- Carboxy ;

➢ $R_g$, $R'_g$, et $R_h$, identiques ou différents, représentent un atome d'hydrogène, d'halogène, un groupe (di) $(C_1-C_4)$(alkyl)amino, hydroxy, acylamino, $(C_1-C_4)$alcoxy, $(C_1-C_4)$alkylcarbonylamino ou un radical $(C_1-C_6)$ alkyle ;

➢ deux groupes $R_c$ et $R'_g$ et/ou $R_d$ et $R_g$, forment ensemble un hétérocycle saturé ou un hétéroaryle, éventuellement substitué par un ou plusieurs groupe $(C_1-C_6)$alkyle ;

➢ $R_c$ et $R_d$, identiques ou différents, représentent un atome d'hydrogène, un groupe $(C_1-C_6)$alkyle éventuellement substitué, aryl$(C_1-C_4)$alkyle, un groupe $(C_1-C_6)$alkyle éventuellement substitué ;

> ou alors les deux radicaux $R_c$ et $R_d$ portés par le même atome d'azote forment ensemble un groupe hétérocyclique ou hétéroaryle ;
> $T_a$, représente une liaison covalente σ, ou un groupe choisi parmi : -N(R)-, -C(O)-N(R)-, -N(R)-C(O)- ;
> m et n, identiques ou différents, représentent un entier compris inclusivement entre 1 et 6 avec la somme m+n représente un entier compris inclusivement entre 2 et 6 ;
> $An^-$ représentant un contre-ion anionique ; et
> Y, est tel que défini dans une quelconque des revendications 1 à 5 ;

étant entendu que lorsque les composés de formules **(Ia)** ou **(IIa)** contiennent d'autres parties cationiques, ils se trouvent associés à un ou plusieurs contre-ions anioniques permettant d'atteindre l'électroneutralité de la formules **(Ia)** ou **(IIa).**

**7.** Colorant fluorescent selon une quelconque des revendications 1, 5 et 6 de formule suivante :

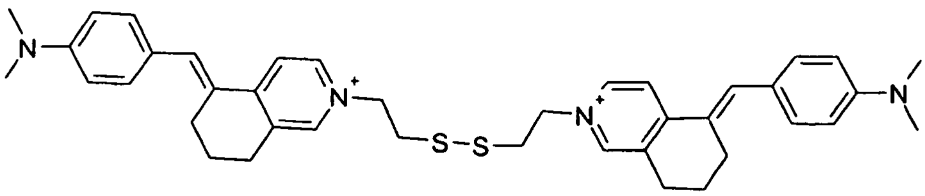

2 $An^-$

**1**

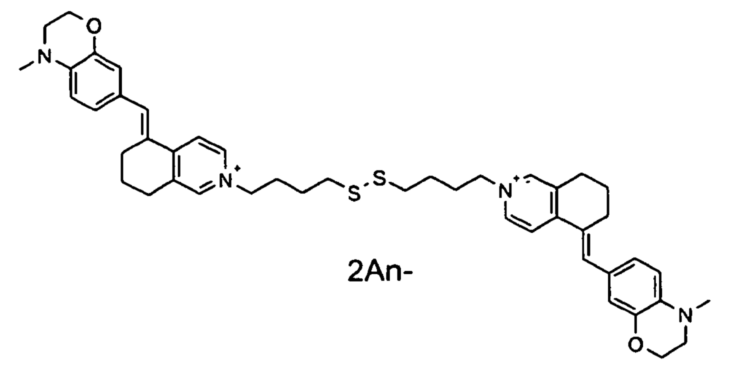

**2**

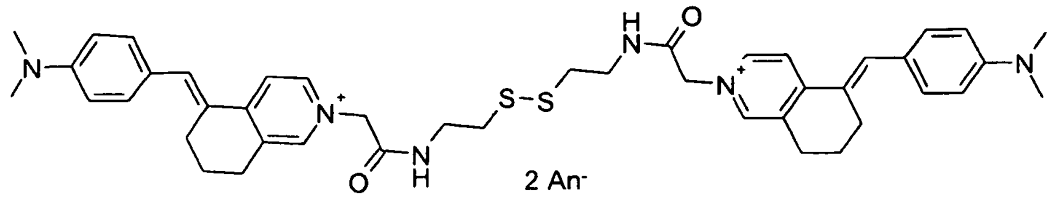

**3**

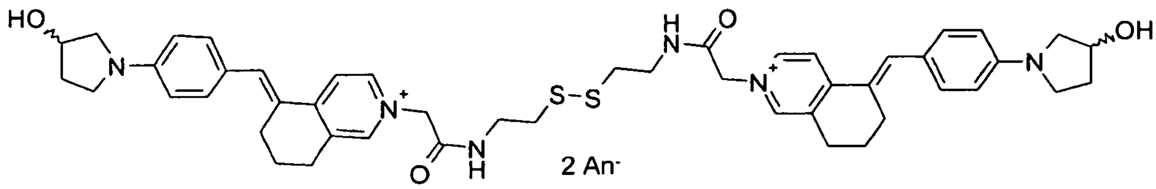

**4**

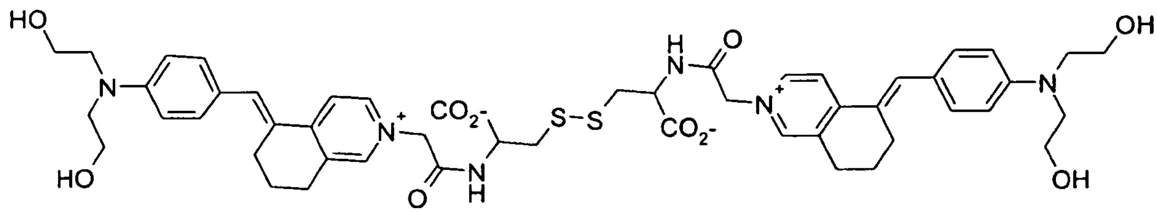

**5**

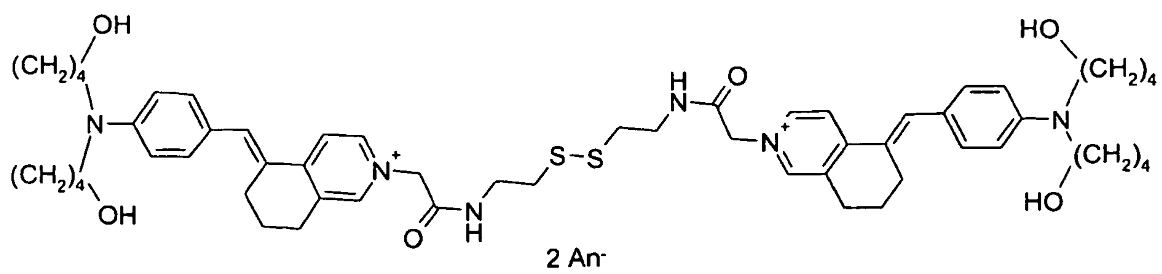

**6**

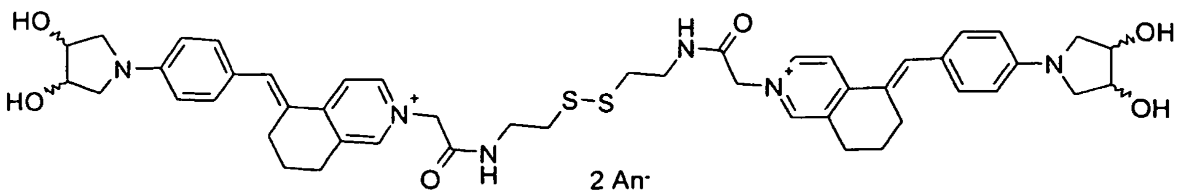

**7**

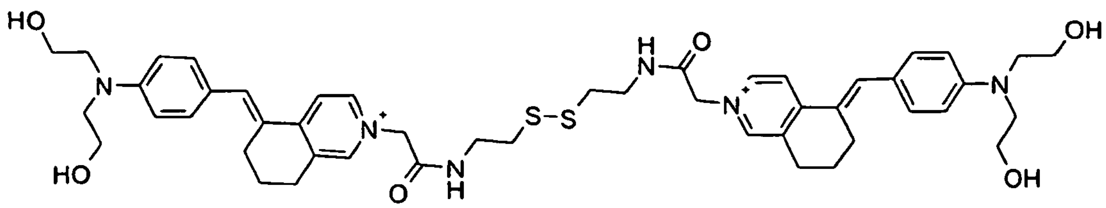

$2\ An^-$

**8**

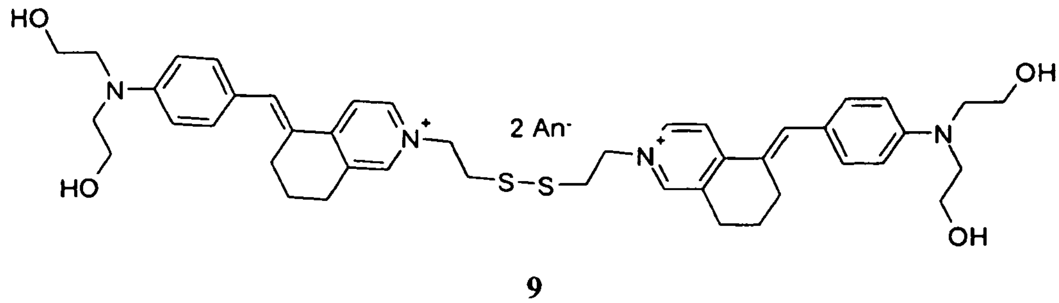

$2\ An^-$

**9**

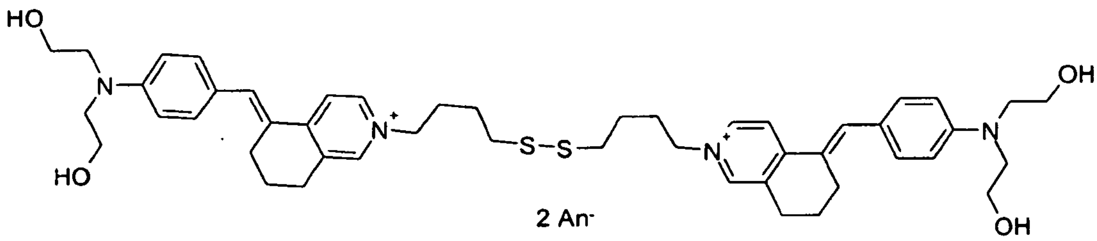

$2\ An^-$

**10**

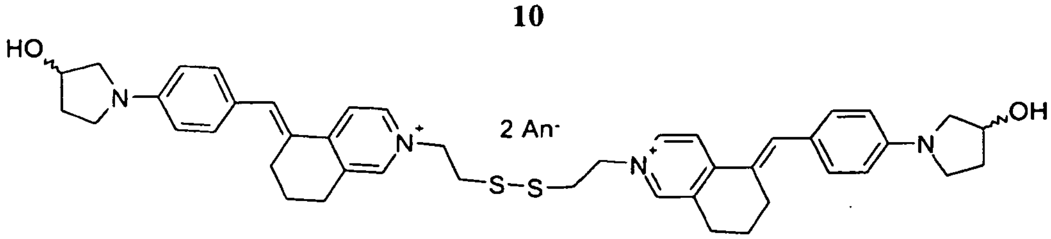

$2\ An^-$

**11**

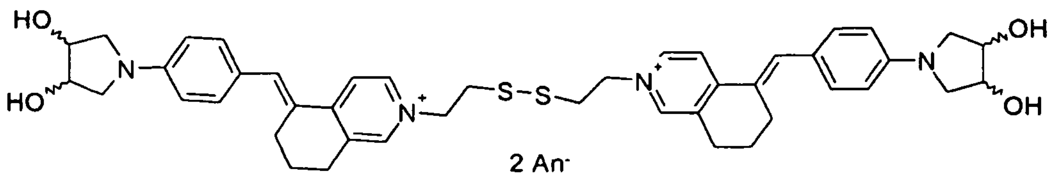

$2\ An^-$

**12**

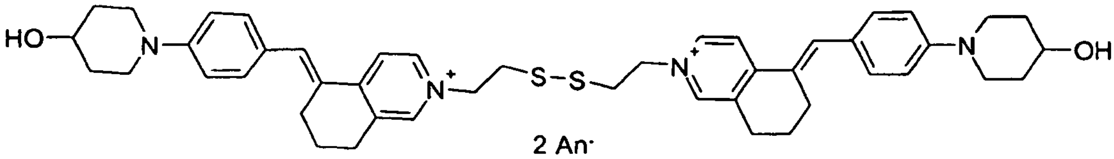

$2\ An^-$

**13**

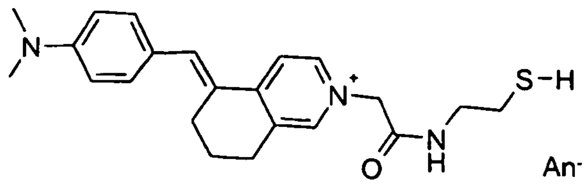

14

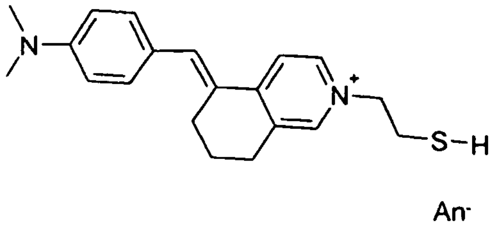

15

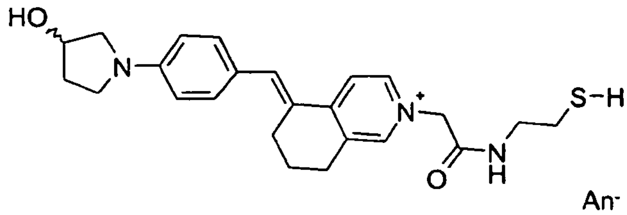

16

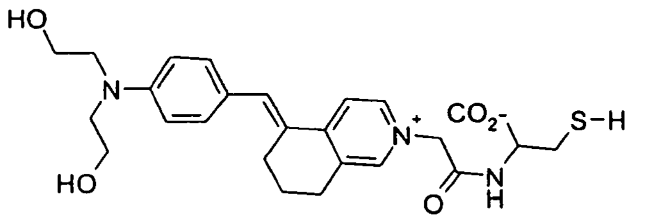

17

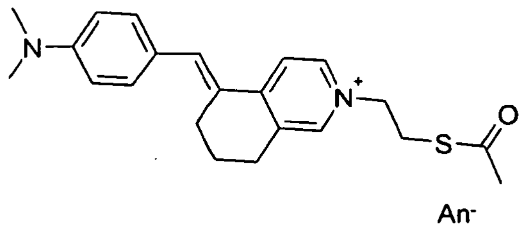

18

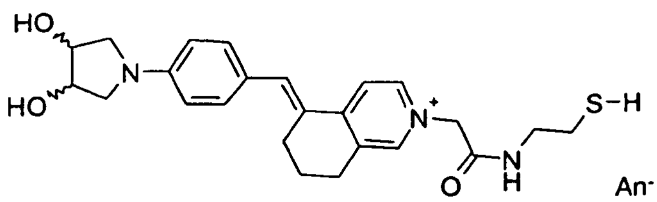

19

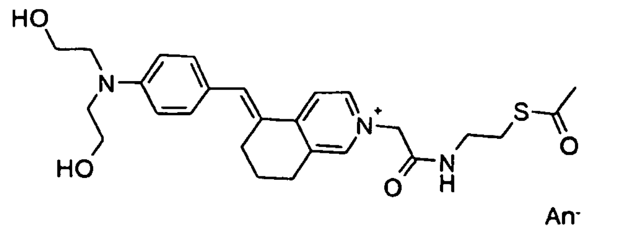

**20**

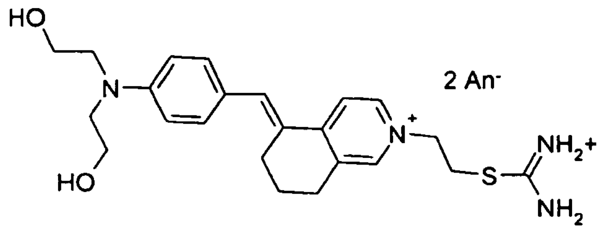

**21**

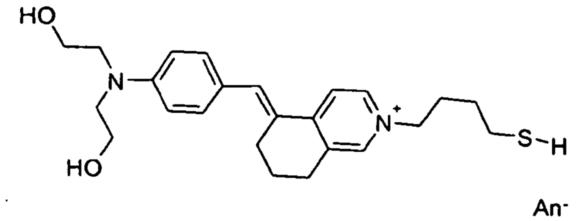

**22**

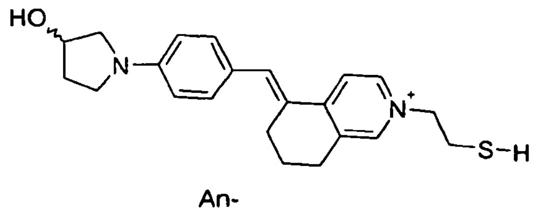

**23**

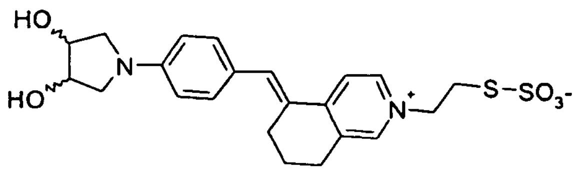

**24**

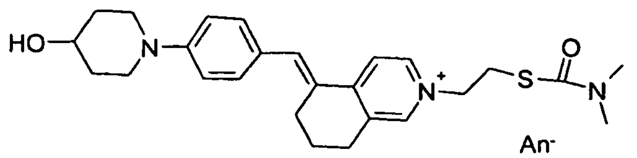

**25**

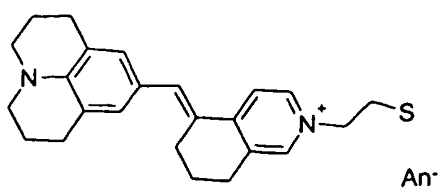

**26**

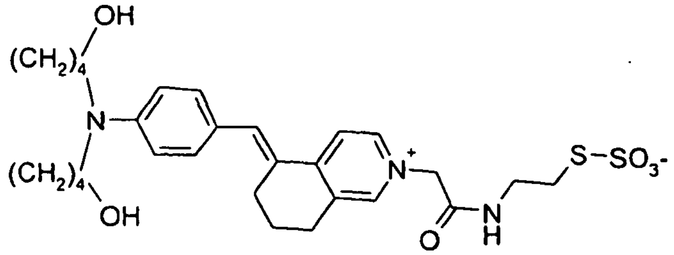

**27**

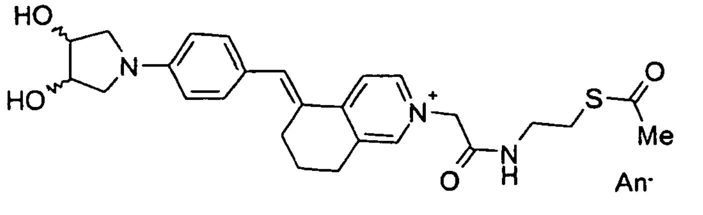

**28**

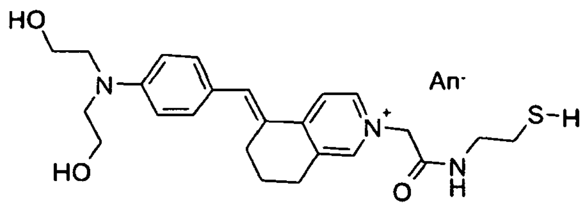

**29**

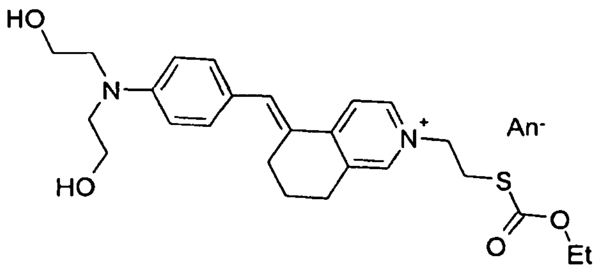

**30**

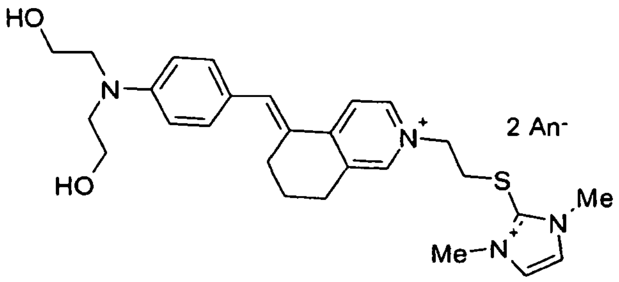

**31**

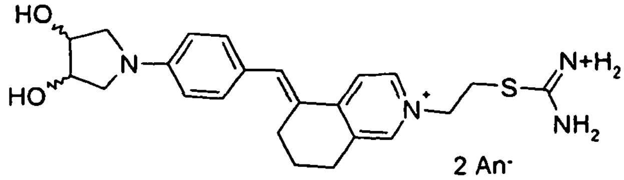

**32**

avec $An^-$, identiques ou différents, représentant un contre-ion anionique.

**8.** Composition tinctoriale comprenant dans un milieu cosmétique approprié, un colorant fluorescent de formule **(I)** ou **(II)** tel que défini dans une quelconque des revendications 1 à 7.

**9.** Composition tinctoriale selon la revendication précédente contenant en outre au moins un agent réducteur.

**10.** Procédé de coloration de matières kératiniques, dans lequel on applique sur les matières, une composition tinctoriale approprié comprenant au moins un colorant fluorescent de formule (I) ou (II) tel que défini dans une quelconque des revendications 1 à 7, éventuellement en présence d'un agent réducteur.

**11.** Procédé de coloration de matières kératiniques selon la revendication précédente **caractérisé en ce que** les matières kératiniques sont des fibres kératiniques foncées possédant une hauteur de ton inférieure ou égale à 6.

**12.** Dispositif à plusieurs compartiments dans lequel un premier compartiment contient une composition tinctoriale comprenant un colorant fluorescent de formule **(I)** ou (II) tel que défini aux revendications 1 à 7 et un deuxième compartiment contient un agent réducteur.

**13.** Utilisation des colorants fluorescents de formule **(I)** ou **(II)** tels que définis aux revendications 1 à 7 pour la teinture de fibres kératiniques humaines notamment foncées particulièrement celles possédant une hauteur de ton inférieure à 6.

**14.** Utilisation selon la revendication précédente pour l'éclaircissement des fibres kératiniques foncées notamment celles possédant une hauteur de ton inférieure à 6.

## Claims

**1.** Fluorescent dye of formula **(I)** or **(II):**

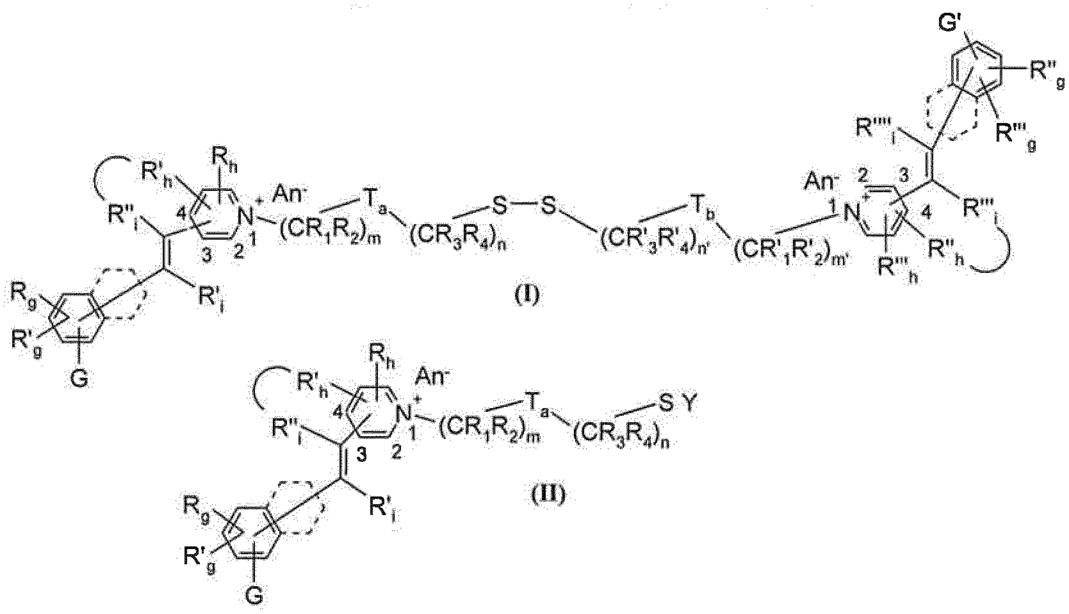

(I)

(II)

organic or mineral acid salts, optical isomers and geometric isomers thereof, and the solvates such as hydrates:

in which formulae **(I)** and **(II):**

➢

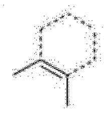 ;

represents an aryl, heterocyclic or heteroaryl; group fused ton the phenyl ring; or else is absent from the phenyl ring;

➢ G and G', which may be identical or different, represent an $-NR_cR_d$ group or a $(C_1-C_6)$alkoxy group, which is optionally substituted;

➢ $R_c$ and $R_d$, which may be identical or different, represent a hydrogen atom, an optionally substituted $(C_1-C_6)$ alkyl group, an aryl $(C_1-C_4)$ alkyl group, a $(C_1-C_6)$ alkoxy$(C_1-C_6)$alkyl group or a $(C_1-C_6)$alkyl group which is optionally substituted;

➢ or else the two $R_c$ and $R_d$ radicals borne by the same nitrogen atom together form a heterocyclic or heteroaryl group;

➢ $R_g$, $R'_g$, $R''_g$, $R'''_g$, $R_h$ and $R'''_h$, which may be identical or different, represent a hydrogen or halogen atom, an amino, (di) $(C_1-C_4)$alkylamino, cyano, carboxyl, hydroxyl, trifluoromethyl, acylamino, $C_1-C_4$ alkoxy, $C_2-C_4$ (poly)hydroxyalkoxy, $(C_1-C_4)$alkylcarbonyloxy $(C_1-C_4)$ alkoxycarbonyl, $(C_1-C_4)$alkylcarbonylamino, acylamino, carbamoyl or $(C_1-C_4)$alkylsulphonylamino group or an aminosulphonyl radical, or a $(C_1-C_{16})$ alkyl radical optionally substituted with a group chosen from $(C_1-C_{12})$alkoxy, hydroxyl, cyano, carboxyl, amino and (di) $(C_1-C_4)$alkylamino, or else the two alkyl radicals borne by the nitrogen atom of the amino group form a heterocycle comprising from 5 to 7 members and optionally comprising another heteroatom identical to or different from that of the nitrogen atom; in particular, $R_g$, $R'_g$, $R''_g$, $R'''_g$, $R_h$ and $R'''_h$ represent a hydrogen atom; or else two groups $R_g$ and $R'_g$, $R''_g$ and $R'''_g$, borne by two adjacent carbon atoms, together form a benzo or indeno ring, or a fused heterocycloalkyl or fused heteroaryl group; the benzo, indeno, heterocycloalkyl or heteroaryl ring being optionally substituted with a halogen atom, a $(C_1-C_4)$ alkyl, amino, $(C_1-C_4)$ alkylamino, $(C_1-C_4)$ dialkylamino, cyano, carboxyl, hydroxyl or trifluoromethyl group, an acylamino, $C_1-C_4$ alkoxy, $C_2-C_4$ (poly)hydroxyalkoxy, alkylcarbonyloxy, alkoxycarbonyl or alkylcarbonylamino radical, an acylamino, carbamoyl or alkylsulphonylamino radical, an aminosulphonyl radical, or a $(C_1-C_{16})$ alkyl radical optionally substituted with: a group chosen from $(C_1-C_{12})$alkoxy, hydroxyl, cyano, carboxyl, amino, $(C_1-C_4)$alkylamino and $(C_1-C_4)$ dialkylamino, or else the two alkyl radicals borne by the nitrogen atom of the amino group form a heterocycle comprising from 5 to 7 members and optionally comprising another heteroatom identical to or different from that of the nitrogen atom;

➢ or else, when G and/or G' represent $-NR_cR_d$, two groups $R_c$ and $R'_g$, $R_d$ and $R_g$ and/or $R_c$ and $R''_g$, $R_d$ and $R'''_g$ together form a saturated heteroaryl or heterocycle, optionally substituted with one or more $(C_1-C_6)$ alkyl groups;

➢ $R'_i$ and $R'''_i$, which may be identical or different, represent a hydrogen atom or a $(C_1-C_4)$alkyl group;

➢ $R'_h$ with $R''_i$ and $R''_h$ with $R'''_i$ form, together with the carbon atoms which bear them, an optionally substituted $C_5-C_7$ cycloalkyl group fused to the pyridinium group, it being understood that the $R'_h$ or $R''_h$ radical and the styryl group bearing the $R''_i$ or $R'''_i$ radical are positioned on adjacent carbon atoms of the pyridinium groups;

➢ $R_1$, $R_2$ , $R_3$ , $R_4$, $R'_1$, $R'_2$, $R'_3$ and $R'_4$, which may be identical or different, represent:

- a hydrogen atom,
- a $(C_1-C_4)$ alkyl group,
- $(C_1-C_{12})$alkoxy,
- hydroxyl,
- cyano,
- $-C(O)O^-M^+$ with $M^+$ representing an alkali metal or $M^+$ and $An^-$ are absent,
- carboxyl,
- (di) $(C_1-C_4)$ (alkyl) amino, it being possible for said alkyl radicals to form, with the nitrogen atom which bears them, a heterocycle comprising from 5 to 7 members, optionally comprising another heteroatom which may or may not be different from nitrogen;

➢ $T_a$, $T_b$, which may be identical or different, represent:

i) either a σ covalent bond,
ii) or one or more radicals or combinations thereof, chosen from $-SO_2-$, -O-, -S-, -N(R)-, $-N^+ (R) (R^\circ) -$

and -C (O)-, with R and R°, which may be identical or different, representing a hydrogen atom, a $C_1$-$C_4$ alkyl radical, a $C_1$-$C_4$ hydroxyalkyl radical or an aryl($C_1$-$C_4$)alkyl;

(iii) or a preferably monocyclic, cationic or noncationic, heterocycloalkyl or heteroaryl radical;

➢ m, m', n and n', which may be identical or different, represent an integer between 0 and 6 inclusive, with m + n and m' + n', which may be identical or different, representing an integer between 1 and 10 inclusive;

➢ $An^-$ representing an anionic counterion; and

➢ Y represents: i) a hydrogen atom; ii) an alkali metal; iii) an alkaline earth metal; iv) an ammonium group: $N'R^{\alpha}R^{\beta}R^{\gamma}R^{\delta}$ or a phosphonium group: $P^+R^{\alpha}R^{\beta}R^{\gamma}R^{\delta}$ with $R^{\alpha}$, $R^{\beta}$, $R^{\gamma}$ and $R^{\delta}$, which may be identical or different, representing a hydrogen atom or a ($C_1$-$C_4$) alkyl group; or v) a thiol-function-protecting group chosen from the following radicals:

- ($C_1$-$C_4$) alkylcarbonyl;
- ($C_1$-$C_4$) alkylthiocarbonyl;
- ($C_1$-$C_4$) alkoxycarbonyl ;
- ($C_1$-$C_4$) alkoxythiocarbonyl ;
- ($C_1$-$C_4$) alkylthiothiocarbonyl;
- (di) ($C_1$-$C_4$) (alkyl) aminocarbonyl;
- (di)($C_1$-$C_4$) (alkyl) aminothiocarbonyl;
- arylcarbonyl;
- aryloxycarbonyl;
- aryl ($C_1$-$C_4$)alkoxycarbonyl¡
- (di) ($C_1$-$C_4$) (alkyl) aminocarbonyl;
- ($C_1$-$C_4$) (alkyl) arylaminocarbonyl;
- carboxyl;
- $SO_3^-$; $M^+$ with $M^+$ representing an alkali metal or else $An^-$ of formula (II) and $M^+$ are absent;
- optionally substituted aryl;
- optionally substituted heteroaryl;
- optionally cationic, optionally substituted heterocycloalkyl,
- the following group:

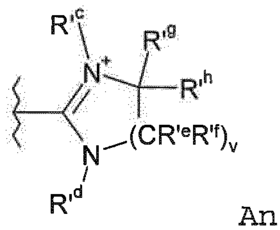

$An'^-$

in which $R'^c$, $R'^d$, $R'^e$, $R'^f$, $R'^g$ and $R'^h$, which may be identical or different, represent a hydrogen atom or a ($C_1$-$C_4$) alkyl group, or else two groups $R'^g$ with $R'^h$, and/or $R'^e$ with $R'^f$ form an oxo or thioxo group, or else $R'^g$ with $R'^e$ together form a cycloalkyl; and v represents an integer between 1 and 3 inclusive; preferably $R'^c$ to $R'^h$ represent a hydrogen atom; and $An'^-$ represents an anionic counterion;

■ isothiouronium;

■ $-C(NR'^cR'^d)=N^+R^eR'^f$; $An'^-$ with $R'^c$, $R'^d$, $R'^e$ and $R'^f$, which may be identical or different, representing a hydrogen atom or a ($C_1$-$C_4$)alkyl group and $An^-$ being as defined above;

■ isothiourea;

■ $-C(NR'^cR'^d)=NR'^e$; An' with $R'^c$, $R'^d$, $R'^e$ and $An^-$ as defined above;

■ optionally substituted (di)aryl($C_1$-$C_4$)alkyl;

■ optionally substituted (di)heteroaryl($C_1$-$C_4$) alkyl ;

■ $-CR^1R^2R^3$ with $R^1$, $R^2$ and $R^3$, which may be identical or different, representing a halogen atom or a group chosen from:

i) ($C_1$-$C_4$) alkyl;
ii) ($C_1$-$C_4$) alkoxy;
iii) optionally substituted aryl;

iv) optionally substituted heteroaryl;

v) P ($Z^1$) R' $^1$R' $^2$R'$^3$ with R'$^1$ and R'$^2$, which may be identical or different, representing a hydroxyl, ($C_1$-$C_4$)alkoxy or alkyl group, R'$^3$ representing a hydroxyl or ($C_1$-$C_4$)alkoxy group, and $Z^1$ representing an oxygen or sulphur atom;

■ a sterically hindered cyclic group; and

■ optionally substituted alkoxyalkyl. it being understood that, when the compound of formula (I) or (II) contains other cationic parts, it is associated with one or more anionic counterions allowing formula (I) or (II) to achieve electroneutrality.

**2.** Fluorescent dye of formula (II) according to the preceding claim, in which Y represents a hydrogen atom or an alkali metal.

**3.** Fluorescent dye of formula (II) according to Claim 1, in which Y represents a protecting group.

**4.** Fluorescent dye of formula (II) according to any one of the preceding claims, in which Y represents an alkali metal or a protecting group chosen from:

➢ ($C_1$-$C_4$)alkylcarbonyl;
➢ arylcarbonyl;
➢ ($C_1$-$C_4$) alkoxycarbonyl ;
➢ aryloxycarbonyl;
➢ aryl ($C_1$-$C_4$) alkoxycarbonyl ;
➢ (di) ($C_1$-$C_4$) (alkyl) aminocarbonyl;
➢ ($C_1$-$C_4$) (alkyl) arylaminocarbonyl;
➢ optionally substituted aryl;
➢ 5- or 6-membered cationic monocyclic heteroaryl optionally substituted with one or more ($C_1$-$C_4$) alkyl groups which may be identical or different;
➢ 8- to 11-membered cationic bicyclic heteroaryl optionally substituted with one or more ($C_1$-$C_4$)alkyl groups which may be identical or different;
➢ cationic heterocycle of the following formula:

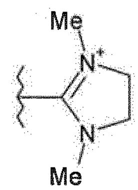

$An'^-$

➢ isothiouronium:
➢ -C ($NH_2$) =$N^+H_2$; $An'^-$;
➢ isothiourea;
➢ -C($NH_2$)=NH; and
➢ $SO_3^-$; $M^+$ with $M^+$ representing an alkali metal or else $An^-$ of formula (II) and $M^+$ are absent.

**5.** Disulphide fluorescent dye according to Claim 1, of formula (I), having a C2 axis of symmetry between the two sulphur atoms of the central disulphide radical.

**6.** Fluorescent dye according to any one of the preceding claims, belonging to one of the two formulae (Ia) and (IIa);

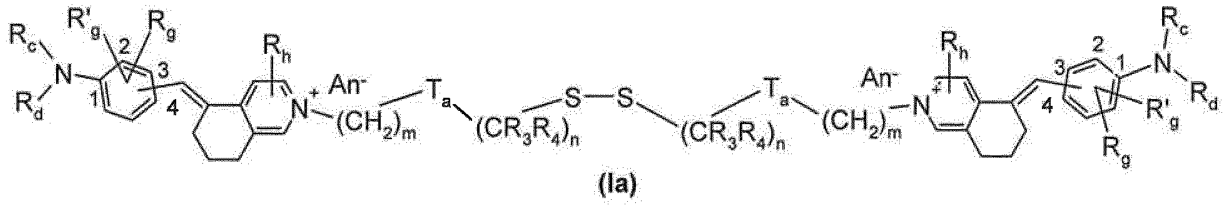

(Ia)

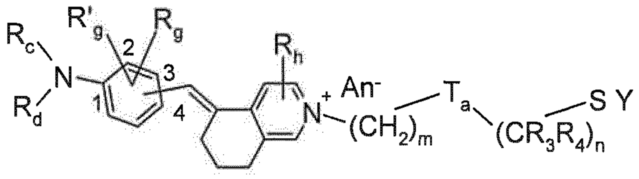

(IIa)

with, for formulae **(Ia)** and **(IIa) :**

- ➢ $R_3$ and $R_4$, which may be identical or different, represent:
  - a hydrogen atom,
  - a $(C_1\text{-}C_4)$alkyl group,
  - $-C(O)O\text{-}M^+$ with $M^+$ representing an alkali metal, or $M^+$ and $An^-$ are absent,
  - carboxyl;
- ➢ $R_g$, $R'_g$ and $R_h$, which may be identical or different, represent a hydrogen or a halogen atom, a (di) $(C_1\text{-}C_4)$ (alkyl) amino, hydroxyl, acylamino, $(C_1\text{-}C_4)$alkoxy or $(C_1\text{-}C_4)$alkylcarbonylamino group or a $(C_1\text{-}C_6)$alkyl radical;
- ➢ two groups $R_c$ and $R'_g$ and/or $R_d$ and $R_g$ together form a saturated heterocycle or a heteroaryl, optionally substituted with one or more $(C_1\text{-}C_6)$ alkyl groups;
- ➢ $R_c$ and $R_d$, which may be identical or different, represent a hydrogen atom, an optionally substituted $(C_1\text{-}C_6)$ alkyl group, aryl $(C_1\text{-}C_4)$alkyl, or an optionally substituted $(C_1\text{-}C_6)$ alkyl group;
- ➢ or else the two radicals $R_c$ and $R_d$ borne by the same nitrogen atom together form a heterocyclic or heteroaryl group;
- ➢ $T_a$ represents a σ covalent bond or a group chosen from: -N(R)-, -C(O)-N(R)- and N(R)-C(O)-;
- ➢ m and n, which may be identical or different, represent an integer between 1 and 6 inclusive, with the sum m + n representing an integer between 2 and 6 inclusive;
- ➢ $An^-$ represents an anionic counterion; and
- ➢ Y is as defined in any one of claims 1 to 5;

it being understood that, when the compounds of formula **(Ia)** or **(IIa)** contain other cationic parts, they are associated with one or more anionic counterions allowing formula **(Ia)** or **(IIa)** to achieve electroneutrality.

**7.** Fluorescent dye according to any one of claims 1, 5 and 6, of the following formula:

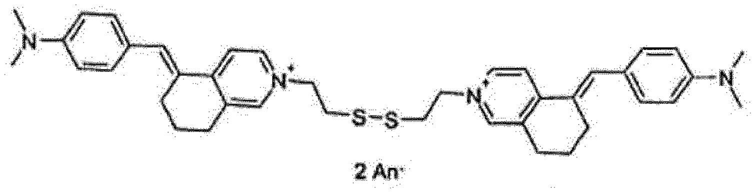

2 An⁻

1

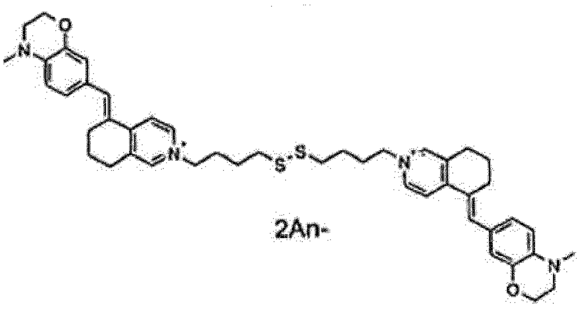

2An-

2

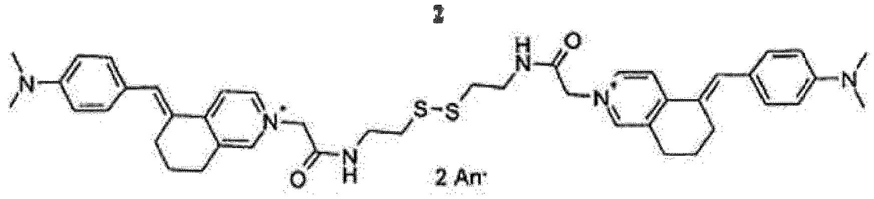

2 An-

3

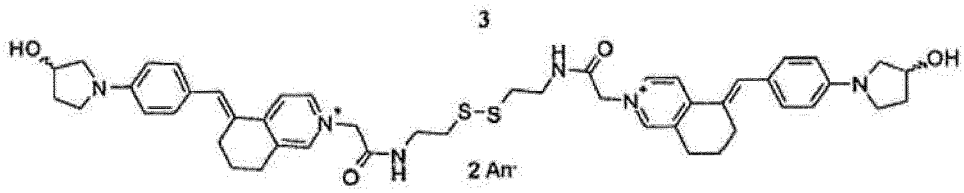

2 An-

4

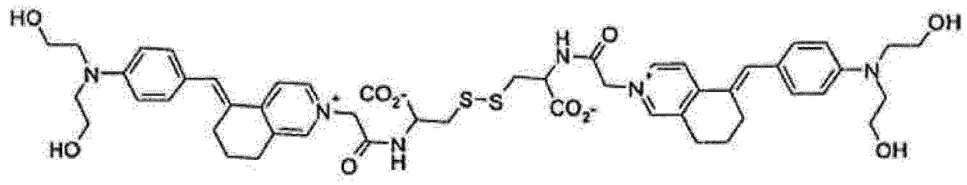

5

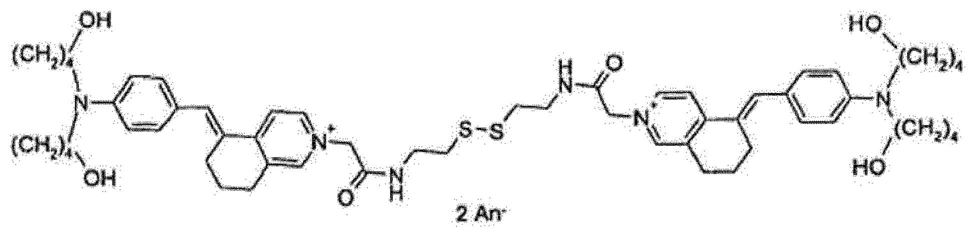

2 An-

6

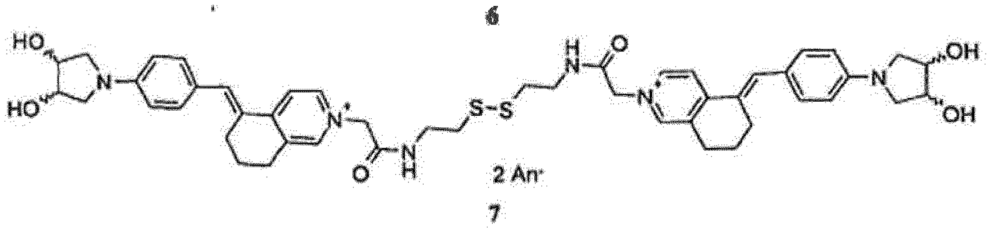

2 An-

7

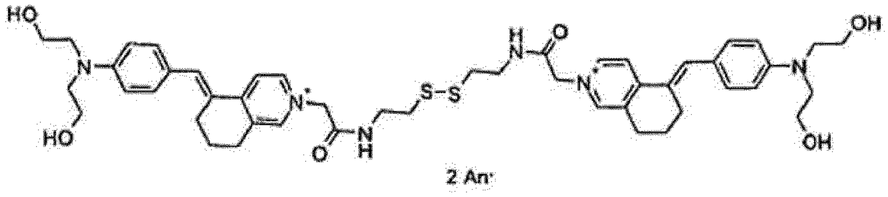

2 $An^-$

**8**

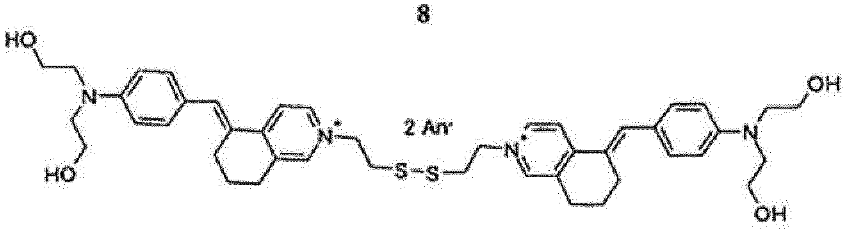

2 $An^-$

**9**

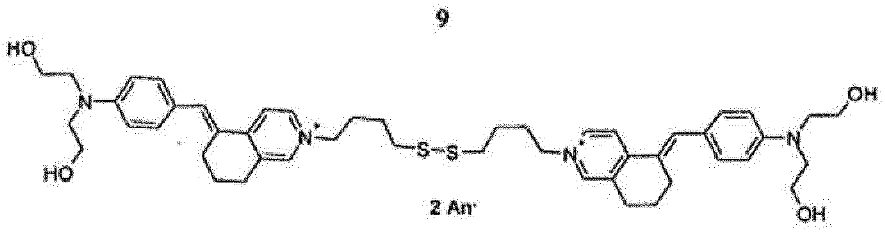

2 $An^-$

**10**

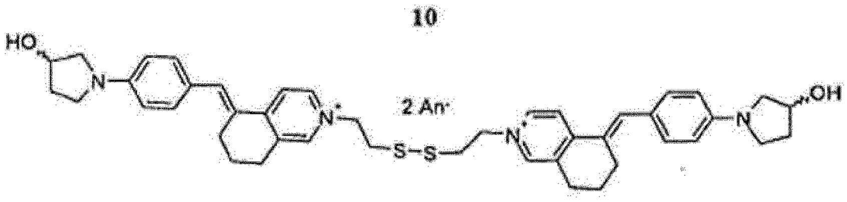

2 $An^-$

**11**

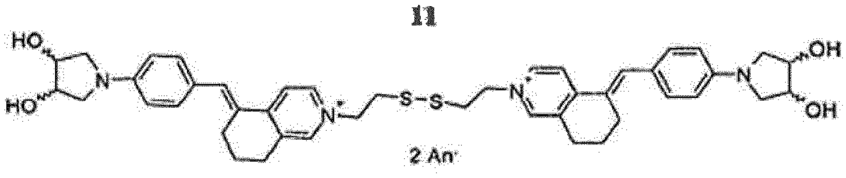

2 $An^-$

**12**

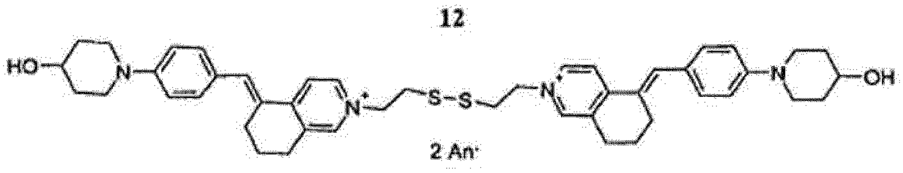

2 $An^-$

**13**

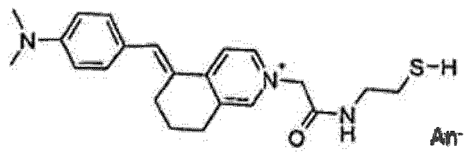

14

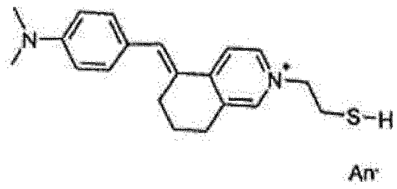

15

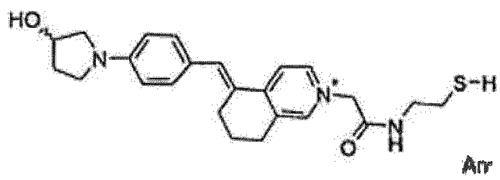

16

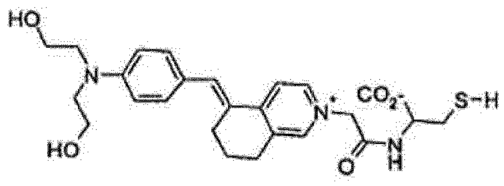

17

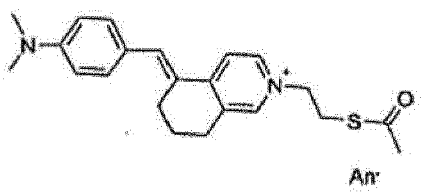

18

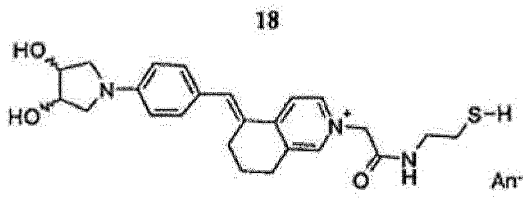

19

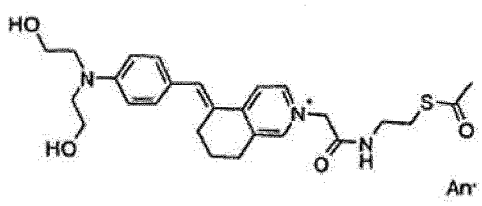

20

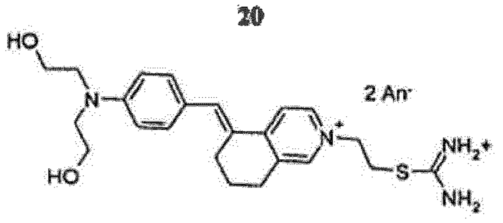

21

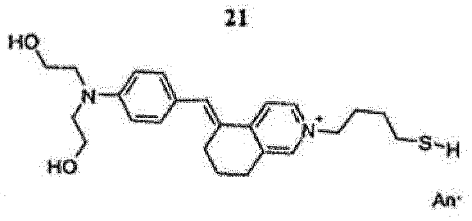

22

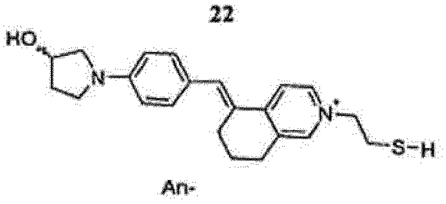

23

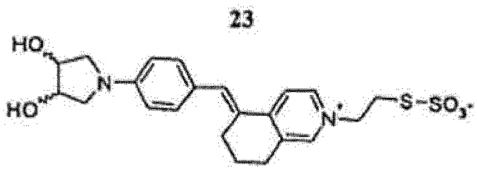

24

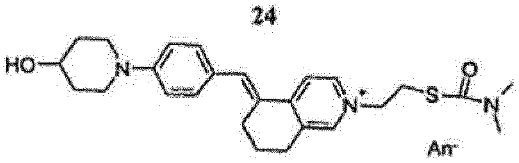

25

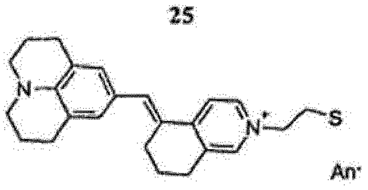

26

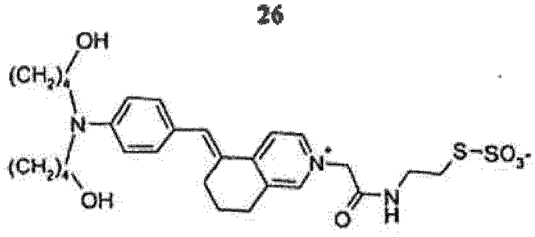

27

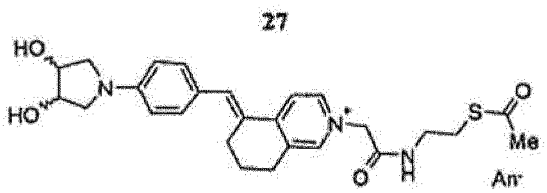

28

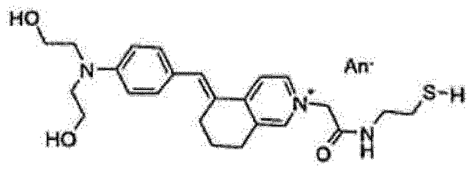

29

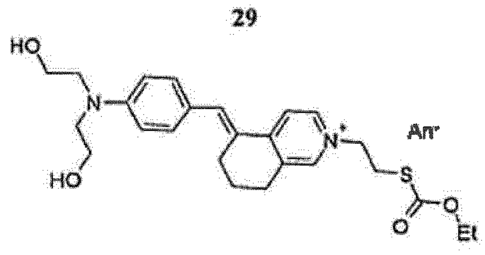

30

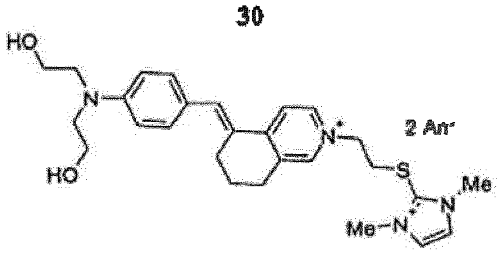

31

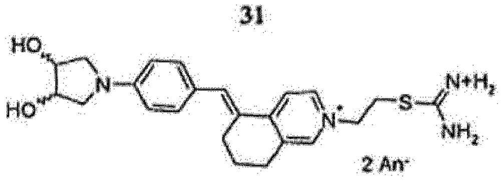

32

with An⁻, which may be identical or different, representing an anionic counterion.

8. Dye composition comprising, in a suitable cosmetic medium, a fluorescent dye of formula **(I)** or **(II)** as defined in any one of Claim 1 to 7.

9. Dye composition according to the preceding claim, also containing at least one reducing agent.

10. Process for dyeing keratin materials, in which a suitable dye composition comprising at least one fluorescent dye of formula (I) or (II) as defined in any one of Claims 1 to 7, optionally in the presence of a reducing agent, is applied to the materials.

11. Dyeing process according to the preceding claim, **characterized in that** the keratin materials are dark keratin fibers

having a tone height of less than or equal to 6.

**12.** Multicompartment device in which a first compartment contains a dye composition comprising a fluorescent dye of formula **(I)** or **(II)** as defined in Claims 1 to 7, and a second compartment contains a reducing agent.

**13.** Use of the fluorescent dyes of formula **(I)** or **(II)** as defined in Claims 1 to 7, for dyeing human keratin fibers, in particular dark human keratin fibers and in particular those having a tone height of less than 6.

**14.** Use according to the preceding claim, for lightening dark keratin fibers, in particular those having a tone height of less than 6.

**Patentansprüche**

**1.** Fluoreszenzfarbstoff der Formel (I) oder (II):

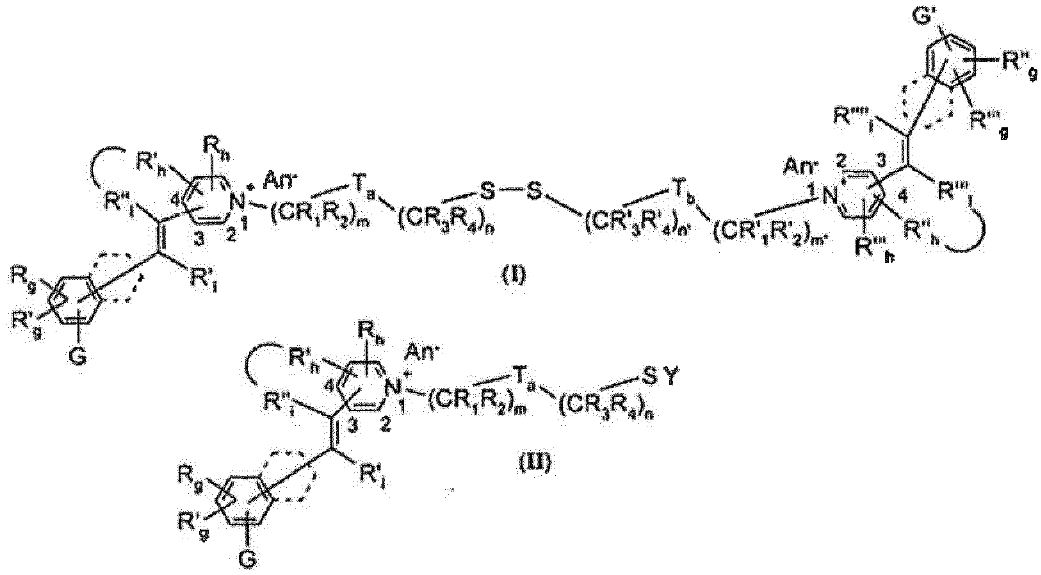

(I)

(II)

Salze davon mit einer organischen oder anorganischen Säure, optische Isomere davon, geometrische Isomere davon und Solvate wie Hydrate;
wobei in den Formeln (I) und (II):

- ➢ 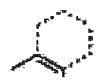

  für eine an den Phenylring ankondensierte Aryl-, Heterocyclyl- oder Heteroarylgruppe steht oder auch am Phenylring fehlt;
- ➢ G und G' gleich oder verschieden sind und für eine -$NR_cR_d$-Gruppe oder ($C_1$-$C_6$) -Alkoxygruppe, die gegebenenfalls substituiert ist, stehen;
- ➢ $R_c$ und $R_d$ gleich oder verschieden sind und für ein Wasserstoffatom oder eine gegebenenfalls substituierte ($C_1$-$C_6$) -Alkylgruppe, Aryl($C_1$-$C_6$)-alkylgruppe, ($C_1$-$C_6$)-Alkoxy($C_1$-$C_6$)alkylgruppe oder gegebenenfalls substituierte ($C_1$-$C_6$)-Alkylgruppe stehen;
- ➢ oder auch die beiden Reste $R_c$ und $R_d$, die an dasselbe Stickstoffatom gebunden sind, zusammen eine Heterocyclyl- oder Heteroarylgruppe bilden;
- ➢ $R_g$, $R'_g$, $R''_g$, $R'''_g$, $R_h$ und $R'''_h$ gleich oder verschieden sind und für ein Wasserstoff- oder Halogenatom, eine Amino-, (Di) ($C_1$-$C_4$)alkylamino, Cyano-, Carboxyl-, Hydroxyl-, Trifluormethyl, Acylamino-, $C_1$-$C_4$-Alkoxy-,

$C_2$-$C_4$-(Poly)hydroxyalkoxy-, ($C_1$-$C_4$)Alkylcarbonyloxy-, ($C_1$-$C_4$)Alkoxycarbonyl-, ($C_1$-$C_4$)Alkylcarbonylamino-, Acylamino-, Carbamoyl- oder ($C_1$-$C_4$)-Alkylsulfonylaminogruppe, einen Aminosulfonylrest oder einen ($C_1$-$C_{16}$) Alkylrest, der gegebenenfalls durch eine unter ($C_1$-$C_{12}$)Alkoxy, Hydroxyl, Cyano, Carboxyl, Amino und (Di) ($C_1$-$C_4$)alkylamino ausgewählte Gruppe substituiert ist, stehen oder die beiden Alkylreste, die an das Stickstoffatom der Aminogruppe gebunden sind, einen 5- bis 7-gliedrigen Heterocyclus bilden, der gegebenenfalls ein weiteres mit dem Stickstoffatom identisches oder davon verschiedenes Heteroatom enthält; $R_g$, $R'_g$, $R''_g$, $R'''_g$, $R_h$ und $R'''_h$ insbesondere für ein Wasserstoffatom stehen;

oder auch zwei Gruppen $R_g$ und $R'_g$, $R''_g$ und $R'''_g$, die an zwei benachbarte Kohlenstoffatome gebunden sind, zusammen einen Benzo- oder Indenoring oder eine ankondensierte Heterocycloalkyl- oder Heteroarylgruppe bilden; wobei der Benzo-, Indeno-, Heterocycloalkyl- oder Heteroarylring gegebenenfalls durch ein Halogenatom, eine ($C_1$-$C_4$)Alkyl-, Amino-, ($C_1$-$C_4$)-Alkylamino-, ($C_1$-$C_4$)Dialkylamino-, Cyano-, Carboxyl-, Hydroxyl- oder Trifluormethylgruppe, einen Acylamino-, $C_1$-$C_4$-Alkoxy-, $C_2$-$C_4$-(Poly)-hydroxyalkoxy-, Alkylcarbonyloxy-, Alkoxycarbonyl-, oder Alkylcarbonylaminorest, einen Acylamino-, Carbamoyl- oder Alkylsulfonylaminorest, einen Aminosulfonylrest oder einen ($C_1$-$C_{16}$)Alkylrest, der gegebenenfalls durch eine unter ($C_1$-$C_{12}$)Alkoxy, Hydroxyl, Cyano, Carboxyl, Amino, ($C_1$-$C_4$)Alkylamino und ($C_1$-$C_4$)Dialkylamino ausgewählte Gruppe substituiert ist, oder auch die beiden Alkylreste, die an das Stickstoffatom der Aminogruppe gebunden sind, einen 5-bis 7-gliedrigen Heterocyclus bilden, der gegebenenfalls ein weiteres mit dem Stickstoffatom identisches oder davon verschiedenes Heteroatom enthält;

➢ oder auch dann, wenn G und/oder G' für $-NR_cR_d$ stehen, zwei Gruppen $R_c$ und $R'_g$, $R_d$ und $R_g$ und/oder $R_c$ und $R''_g$, $R_d$ und $R'''_g$ zusammen ein Heteroaryl oder gesättigtes Heterocyclyl, das gegebenenfalls durch eine oder mehrere ($C_1$-$C_6$)Alkylgruppen substituiert ist, bilden;

➢ $R'_i$ und $R''''_i$ gleich oder verschieden sind und für ein Wasserstoffatom oder eine ($C_1$-$C_4$) - Alkylgruppe stehen;

➢ $R'_h$ mit $R''_i$ und $R''_h$ mit $R'''_i$ zusammen mit den Kohlenstoffatomen, an die sie gebunden sind, eine an die Pyridiniumgruppe ankondensierte gegebenenfalls substituierte $C_5$-$C_7$-Cycloalkylgruppe bilden, mit der Maßgabe, dass der Rest $R'_h$ oder $R''_h$ und die Styrylgruppe, an die der Rest $R''_i$ oder $R'''_i$ gebunden ist, an benachbarten Kohlenstoffatomen der Pyridiniumgruppen stehen;

➢ $R_1$, $R_2$, $R_3$, $R_4$, $R'_1$, $R'_2$, $R'_3$ und $R'_4$ gleich oder verschieden sind und für

- ein Wasserstoffatom,
- eine ($C_1$-$C_4$)Alkylgruppe,
- ($C_1$-$C_{12}$)Alkoxy,
- Hydroxyl,
- Cyano,
- $-C(O)O^-M^+$, wobei $M^+$ für ein Alkalimetall steht oder $M^+$ und $An^-$ fehlen,
- Carboxyl,
- (Di) ($C_1$-$C_4$) (alkyl) amino, wobei die Alkylreste mit dem Stickstoffatom, an das sie gebunden sind, einen 5- bis 7-gliedrigen Heterocyclus bilden können, der gegebenenfalls ein weiteres mit Stickstoff identisches oder davon verschiedenes Heteroatom enthält,

stehen;

➢ $T_a$ und $T_b$ gleich oder verschieden sind und für:

i) eine kovalente $\sigma$-Bindung;
ii) einen oder mehrere Reste oder Kombinationen davon, ausgewählt unter $-SO_2$-, -O-, -S-, -N (R) -, $-N^+(R)(R°)$- und -C(O) -, wobei R und R° gleich oder verschieden sind und für ein Wasserstoffatom oder einen $C_1$-$C_4$-Alkyl-, $C_1$-$C_4$-Hydroxyalkyl-oder Aryl($C_1$-$C_4$)alkylrest stehen;
iii) oder einen kationischen oder nichtkationischen Heterocycloalkyl- oder Heteroarylrest, der vorzugsweise monocyclisch ist,

stehen;

➢ m, m', n und n' gleich oder verschieden sind und für eine ganze Zahl zwischen 0 und 6 einschließlich stehen, wobei m+n und m'+n' gleich oder verschieden sind und für eine ganze Zahl zwischen 1 und 10 einschließlich stehen;

➢ $An^-$ für ein anionisches Gegenion steht und

➢ Y für Folgendes steht: i) ein Wasserstoffatom; ii) ein Alkalimetall; iii) ein Erdalkalimetall; iv) eine Ammoniumgruppe: $N^+R^\alpha R^\beta R^\gamma R^\delta$ oder eine Phosphoniumgruppe: $P+R^\alpha R^\beta R^\gamma R^\delta$, wobei $R^\alpha$, $R^\beta$, $R^\gamma$ und $R^\delta$ gleich oder verschieden sind und für ein Wasserstoffatom oder eine ($C_1$-$C_4$)Alkylgruppe stehen; oder v) eine Schutzgruppe für die Thiolfunktion, die unter den folgenden Resten ausgewählt ist:

- $(C_1-C_4)$ Alkylcarbonyl;
- $(C_1-C_4)$ Alkylthiocarbonyl;
- $(C_1-C_4)$ Alkoxycarbonyl ;
- $(C_1-C_4)$ Alkoxythiocarbonyl;
- $(C_1-C_4)$ Alkylthiothiocarbonyl ;
- (Di) $(C_1-C_4)$ (alkyl)aminocarbonyl;
- (Di) $(C_1-C_4)$ (alkyl)aminothiocarbonyl;
- Arylcarbonyl;
- Aryloxycarbonyl;
- Aryl $(C_1-C_4)$ alkoxycarbonyl;
- (Di) $(C_1-C_4)$ (alkyl)aminocarbonyl;
- $(C_1-C_4)$ (Alkyl) arylaminocarbonyl ;
- Carboxyl;
- $SO_3^-$; $M^+$, wobei $M^+$ für ein Alkalimetall steht oder auch $An^-$ der Formel (II) und $M^+$ fehlen;
- gegebenenfalls substituiertem Aryl;
- gegebenenfalls substituiertem Heteroaryl;
- gegebenenfalls kationischem, gegebenenfalls substituiertem Heterocycloalkyl;
- der folgenden Gruppe:

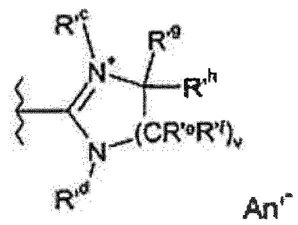

worin $R'^c$, $R'^d$, $R'^e$, $R'^f$, $R'^g$ und $R'^h$ gleich oder verschieden sind und für ein Wasserstoffatom oder eine $(C_1-C_4)$Alkylgruppe stehen oder auch zwei Gruppen $R'^g$ mit $R'^h$ und/oder $R'^e$ mit $R'^f$ eine Oxo- oder Thioxogruppe bilden oder $R'^g$ mit $R'^e$ zusammen ein Cycloalkyl bilden und v für eine ganze Zahl zwischen 1 und 3 einschließlich steht; $R'^c$ bis $R'^h$ vorzugsweise für ein Wasserstoffatom stehen; und $An'^-$ für ein anionisches Gegenion steht;

- Isothiouronium;
- $-C(NR'^cR,^d)=N^+R'^eR'^f$; $An'^-$, wobei $R'^c$, $R'^d$, $R'^e$ und $R'^f$ gleich oder verschieden sind und für ein Wasserstoffatom oder eine $(C_1-C_4)$Alkylgruppe stehen und $An^-$ die oben angegebene Bedeutung besitzt;
- Isothioharnstoff;
- $-C(NR'^cR'^d)=NR'^e$; An'- wobei $R'^c$, $R'^d$ und $R'^e$ und $An^-$ die oben angegebene Bedeutung besitzen;
- gegebenenfalls substituiertem (Di) aryl $(C_1-C_4)$ - alkyl;
- gegebenenfalls substituiertem $(D_i)$heteroaryl$(C_1-C_4)$alkyl ;
- $-CR^1R^2R^3$, wobei $R^1$, $R^2$ und $R^3$ gleich oder verschieden sind und für ein Halogenatom oder eine unter:

  i) $(C_1-C_4)$Alkyl;
  ii) $(C_1-C_4)$ Alkoxy;
  iii) gegebenenfalls substituiertem Aryl;
  iv) gegebenenfalls substituiertem Heteroaryl;
  v) $P(Z^1)R'^1R'^2R'^3$, wobei $R'^1$ und $R'^2$ gleich oder verschieden sind und für eine Hydroxyl-, $(C_1-C_4)$ Alkoxy- oder Alkylgruppe stehen, $R'^3$ für eine Hydroxyloder $(C_1-C_4)$Alkoxygruppe steht und $Z^1$ für ein Sauerstoff- oder Schwefelatom steht;

  ausgewählte Gruppe stehen;
- einer sterisch gehinderten cyclischen Gruppe und
- gegebenenfalls substituiertem Alkoxyalkyl;

mit der Maßgabe, dass die Verbindung der Formel (I) oder (II) dann, wenn sie andere kationische Teile enthält, mit einem oder mehreren anionischen Gegenionen assoziiert ist, so dass die Elektroneutralität der Formel (I) oder (II) gewährleistet ist.

**2.** Fluoreszenzfarbstoff der Formel (II) nach dem vorhergehenden Anspruch, worin Y für ein Wasserstoffatom oder ein Alkalimetall steht.

**3.** Fluoreszenzfarbstoff der Formel (II) nach Anspruch 1, worin Y für eine Schutzgruppe steht.

**4.** Fluoreszenzfarbstoff der Formel (II) nach einem der vorhergehenden Ansprüche, worin Y für ein Alkalimetall oder eine unter:

- ➢ ($C_1$-C4) Alkylcarbonyl ;
- ➢ Arylcarbonyl;
- ➢ ($C_1$-$C_4$)Alkoxycarbonyl;
- ➢ Aryloxycarbonyl;
- ➢ Aryl ($C_1$-$C_4$) alkoxycarbonyl;
- ➢ (Di) ($C_1$-$C_4$) (alkyl)aminocarbonyl;
- ➢ ($C_1$-$C_4$) (Alkyl)arylaminocarbonyl;
- ➢ gegebenenfalls substituiertem Aryl;
- ➢ 5- oder 6-gliedrigem kationischem monocyclischem Heteroaryl, das gegebenenfalls durch eine oder mehrere gleiche oder verschiedene ($C_1$-$C_4$)-Alkylgruppen substituiert ist;
- ➢ 8- bis 11-gliedrigem kationischem bicyclischem Heteroaryl, das gegebenenfalls durch eine oder mehrere gleiche oder verschiedene ($C_1$-$C_4$)-Alkylgruppen substituiert ist;
- ➢ dem kationischen Heterocyclus der nachstehenden Formel:

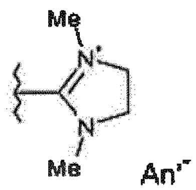

- ➢ Isothiouronium;
- ➢ -C($NH_2$)=$N^+H_2$; An'-;
- ➢ Isothioharnstoff;
- ➢ -C($NH_2$)=NH und
- ➢ $SO_3^-$; $M^+$, wobei $M^+$ für ein Alkalimetall steht oder auch An der Formel (II) und $M^+$ fehlen;

ausgewählte Schutzgruppe steht.

**5.** Disulfid-Fluoreszenzfarbstoff nach Anspruch 1 der Formel (I) mit einer C2-Symmetrieachse zwischen den beiden Schwefelatomen des zentralen Disulfidrests.

**6.** Fluoreszenzfarbstoff nach einem der vorhergehenden Ansprüche, der zu einer der Formeln (Ia) oder (IIa) gehört:

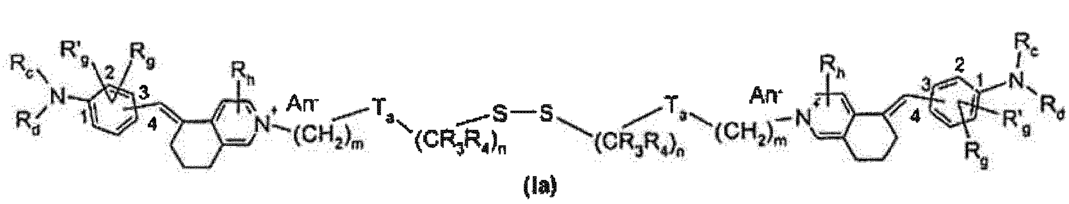

**(Ia)**

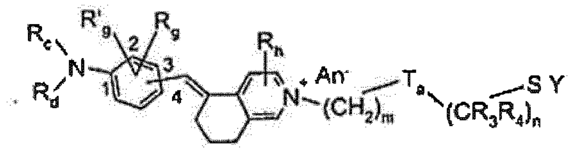

(IIa)

wobei für die Formeln (Ia) und (IIa):

➢ $R_3$ und $R_4$ gleich oder verschieden sind und für:

- ein Wasserstoffatom,
- eine $(C_1\text{-}C_4)$Alkylgruppe,
- $-C(O)O^-M^+$, wobei $M^+$ für ein Alkalimetall steht oder $M^+$ und $An^-$ fehlen,
- Carboxyl

stehen;

➢ $R_g$, $R'_g$ und $R_h$ gleich oder verschieden sind und für ein Wasserstoff- oder Halogenatom, eine (Di) $(C_1\text{-}C_4)$ (alkyl) amino-, Hydroxyl-, Acylamino-, $C_1\text{-}C_4$-Alkoxy- oder $(C_1\text{-}C_4)$Alkylcarbonylamino-gruppe, oder einen $(C_1\text{-}C_6)$Alkylrest stehen;

➢ zwei Gruppen $R_c$ und $R'_g$ und/oder $R_d$ und $R_g$ zusammen ein gesättigtes Heterocyclyl oder ein Heteroaryl, das gegebenenfalls durch eine oder mehrere $(C_1\text{-}C_6)$Alkylgruppen substituiert ist, bilden;

➢ $R_c$ und $R_d$ gleich oder verschieden sind und für ein Wasserstoffatom oder eine gegebenenfalls substituierte $(C_1\text{-}C_6)$ -Alkylgruppe, Aryl$(C_1\text{-}C_4)$-alkylgruppe oder gegebenenfalls substituierte $(C_1\text{-}C_6)$ -Alkylgruppe stehen;

➢ oder auch die beiden Reste $R_c$ und $R_d$, die an dasselbe Stickstoffatom gebunden sind, zusammen eine Heterocyclyl- oder Heteroarylgruppe bilden;

➢ $T_a$ für eine kovalente σ-Bindung oder eine unter -N(R)-, -C (O) -N (R) - und -N(R) -C(O) - ausgewählte Gruppe steht;

➢ m und n gleich oder verschieden sind und für eine ganze Zahl zwischen 1 und 6 einschließlich stehen und die Summe m+n für eine ganze Zahl zwischen 2 und 6 einschließlich steht;

➢ $An^-$ für ein anionisches Gegenion steht und

➢ Y die in einem der Ansprüche 1 bis 5 angegebene Bedeutung besitzt;

mit der Maßgabe, dass die Verbindungen der Formel (Ia) oder (IIa) dann, wenn sie andere kationische Teile enthalten, mit einem oder mehreren anionischen Gegenionen assoziiert sind, so dass die Elektroneutralität der Formeln (Ia) oder (IIa) gewährleistet ist.

**7.** Fluoreszenzfarbstoff nach einem der Ansprüche 1, 5 und 6 der folgenden Formel:

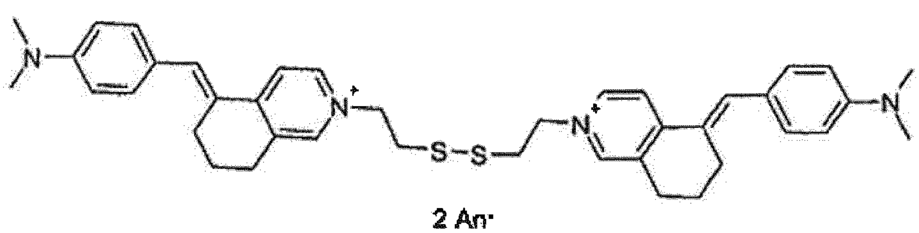

2 An⁻

1

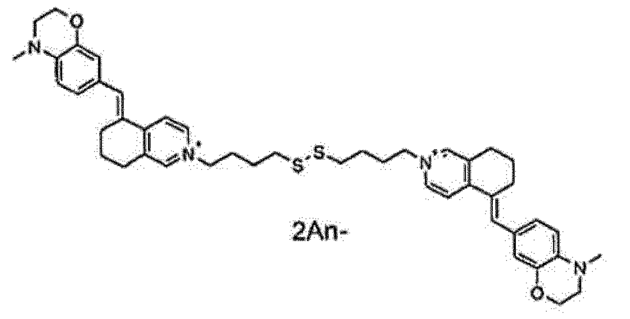

2

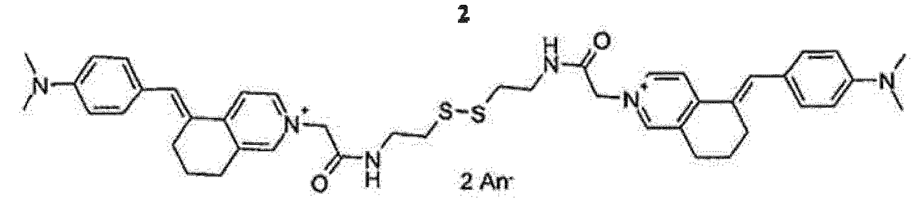

3

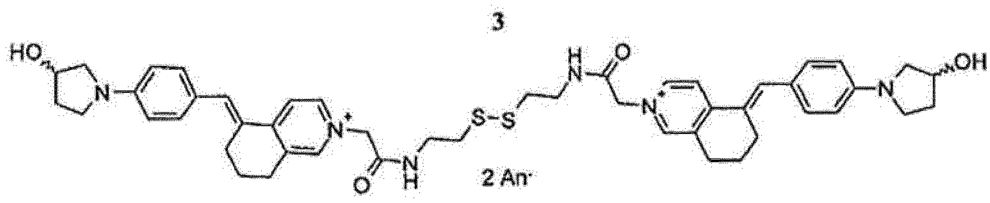

4

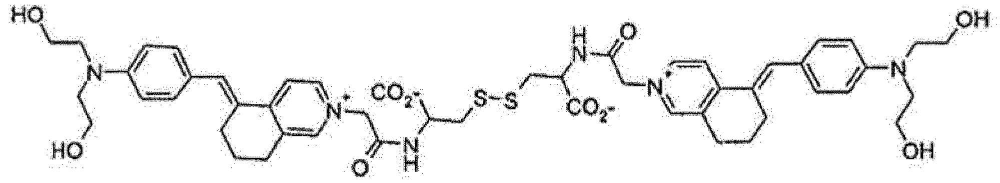

5

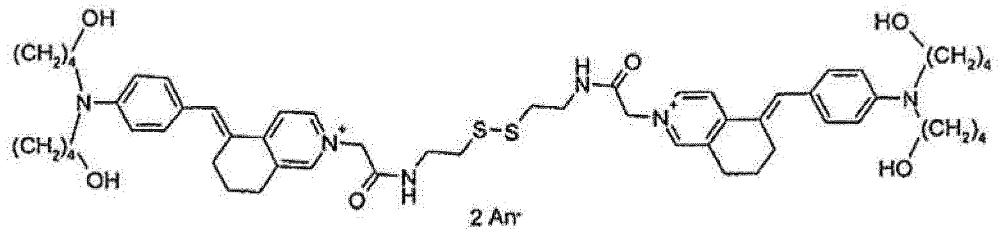

6

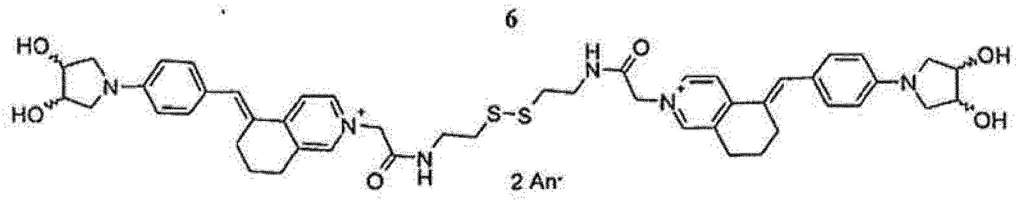

7

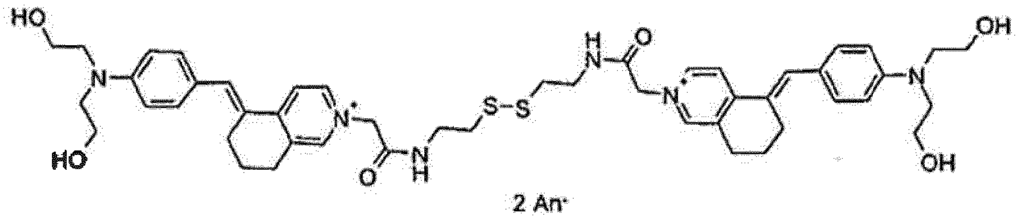

2 An$^-$

**8**

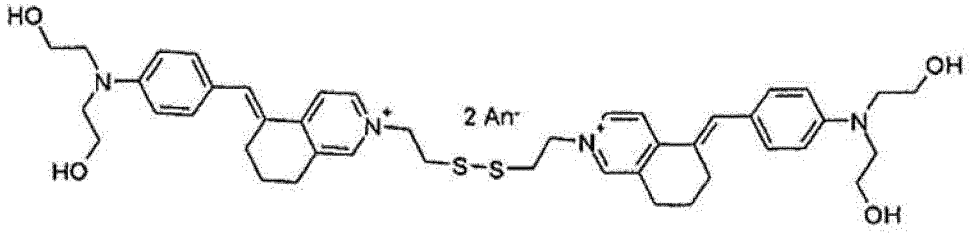

2 An$^-$

**9**

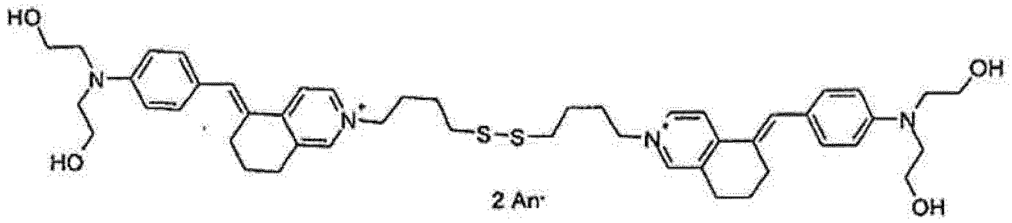

2 An$^-$

**10**

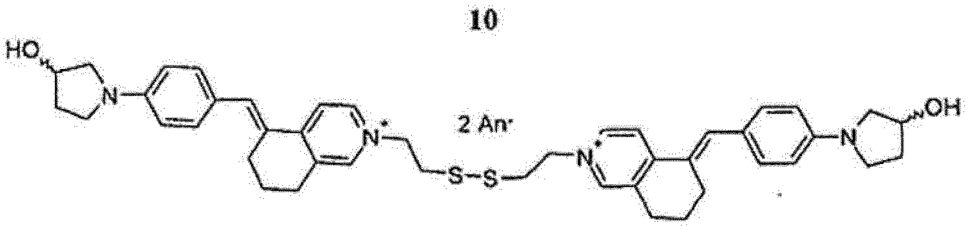

2 An$^-$

**11**

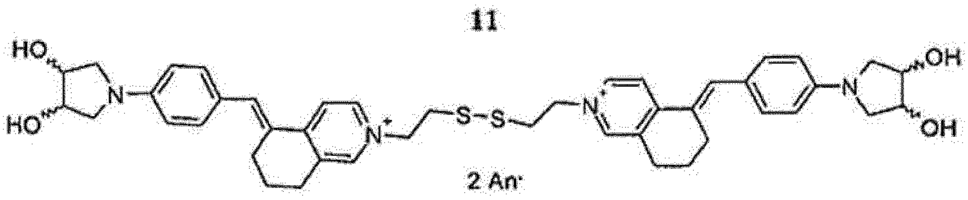

2 An$^-$

**12**

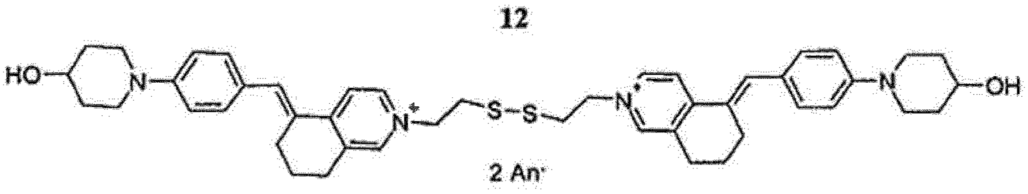

2 An$^-$

**13**

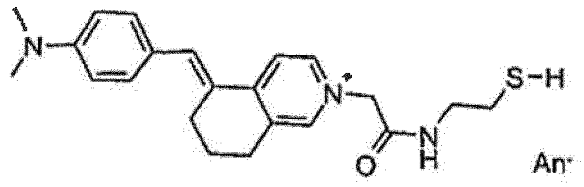

14

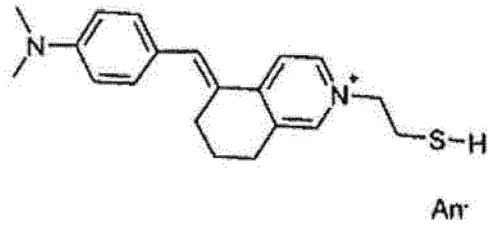

15

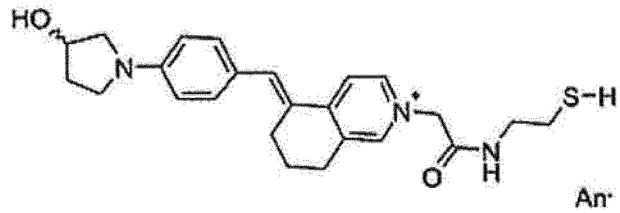

16

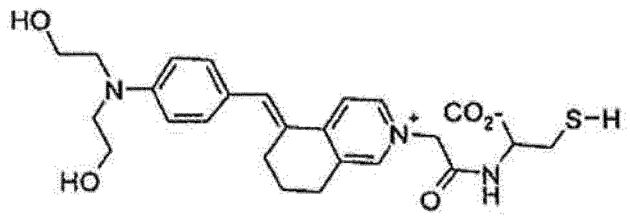

17

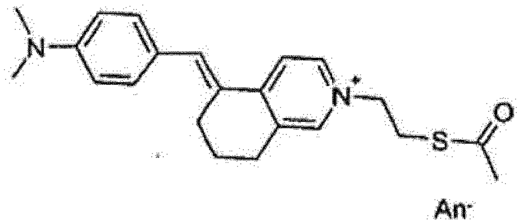

18

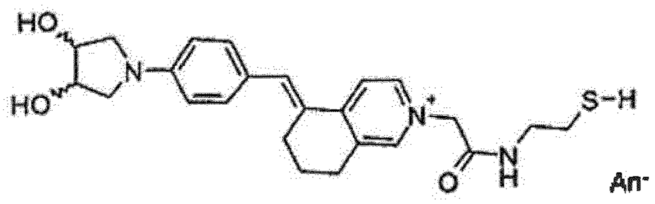

19

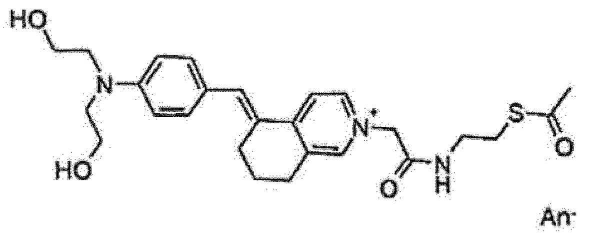

20

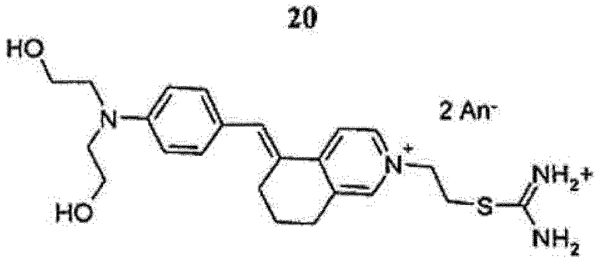

21

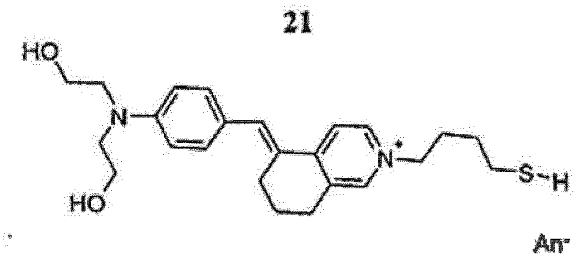

22

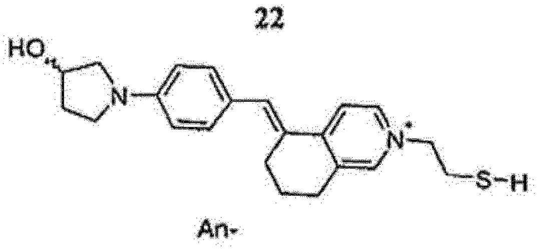

23

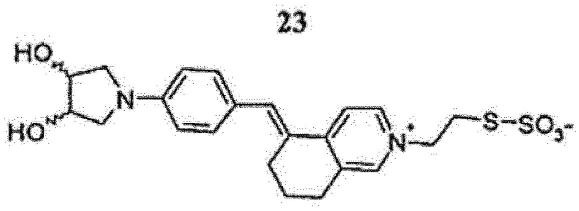

24

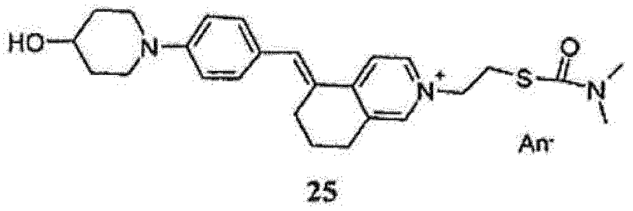

25

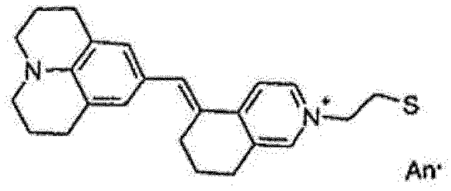

**26**

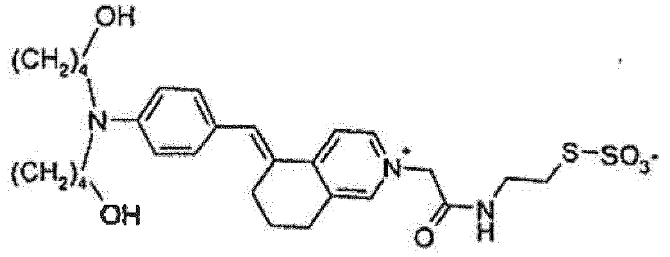

**27**

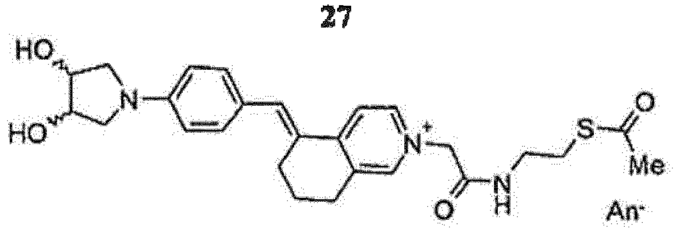

**28**

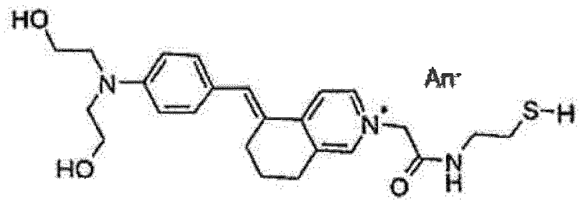

**29**

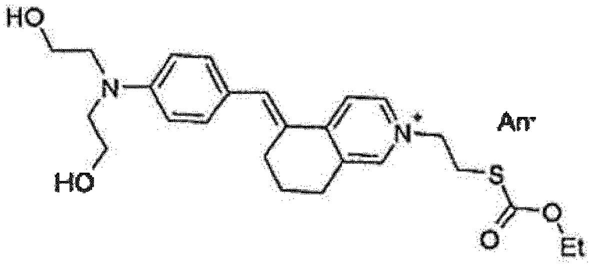

**30**

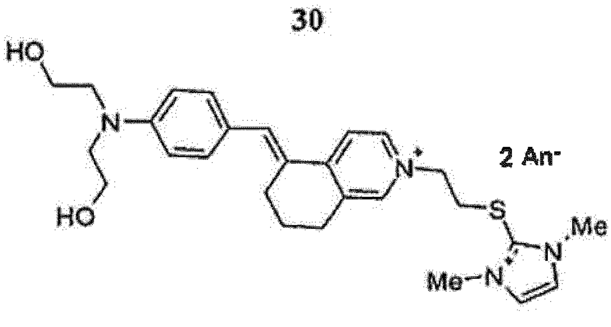

**31**

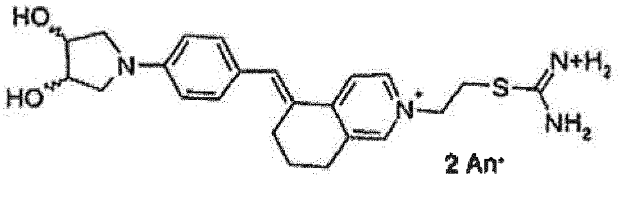

**32**

wobei die Gruppen $An^-$ gleich oder verschieden sind und für ein anionisches Gegenion stehen.

8. Färbezusammensetzung, die in einem geeigneten kosmetischen Medium einen Fluoreszenzfarbstoff der Formel (I) oder (II) gemäß einem der Ansprüche 1 bis 7 umfasst.

9. Färbezusammensetzung nach dem vorhergehenden Anspruch, die außerdem mindestens ein Reduktionsmittel umfasst.

10. Verfahren zum Färben von Keratinmaterialien, bei dem man auf die Materialien eine geeignete Färbezusammensetzung, die mindestens einen Fluoreszenzfarbstoff der Formel (I) oder (II) gemäß einem der Ansprüche 1 bis 7 umfasst, aufbringt, gegebenenfalls in Gegenwart eines Reduktionsmittels.

11. Verfahren zum Färben von Keratinmaterialien nach dem vorhergehenden Anspruch, **dadurch gekennzeichnet, dass** es sich bei den Keratinmaterialien um dunkle Keratinfasern mit einer Tonhöhe kleiner gleich 6 handelt.

12. Vorrichtung mit mehreren Kompartimenten, in der ein erstes Kompartiment eine Färbezusammensetzung, die einen Fluoreszenzfarbstoff der Formel (I) oder (II) gemäß einem der Ansprüche 1 bis 7 umfasst, enthält und ein zweites Kompartiment ein Reduktionsmittel enthält.

13. Verwendung der Fluoreszenzfarbstoffe der Formel (I) oder (II) gemäß einem der Ansprüche 1 bis 7 zum Färben von menschlichen Keratinfasern, insbesondere dunklen Keratinfasern, speziell solchen mit einer Tonhöhe kleiner 6.

14. Verwendung nach dem vorhergehenden Anspruch zur Aufhellung von dunklen Keratinfasern, insbesondere solchen mit einer Tonhöhe kleiner 6.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 03028685 A
- WO 2004091473 A
- WO 2005004822 A
- WO 2005097051 A
- EP 1647580 A
- WO 2006134043 A
- WO 2006136617 A
- FR 2586913


### Littérature non-brevet citée dans la description


- **T. W. GREENE.** Protective Groups in Organic Synthesis. John Willey & Sons, 1981, 193-217
- **P. KOCIENSKI.** Protecting Groups. Thieme, 2005
- Thiocyanates and Isothiocyanates, organic. Ullmann's Encyclopedia. Wiley-VCH, 2005
- Reactions, Mechanisms and Structures. **J. MARCH.** Advanced Organic Chemistry. John Willey & Sons, 1992
- **T. W. GREENE.** *Protective Groups in Organic Synthesis*
- Advanced Organic Chemistry. **J. MARCH.** Reactions, Mechanisms and Structures. John Willey & Sons, 1992
- **T. W. GREENE.** Protective Groups in Organic Synthesis. John Willey & Sons, 1981